(19) Europäisches Patentamt
European Patent Office
Office européen des brevets

(11) EP 4 782 534 A1

(12) **EUROPEAN PATENT APPLICATION**
published in accordance with Art. 153(4) EPC

(43) Date of publication:
**29.07.2026 Bulletin 2026/31**

(21) Application number: **24867436.8**

(22) Date of filing: **18.09.2024**

(51) International Patent Classification (IPC):
***C12N 5/0783*** *(2010.01)* ***A61K 35/17*** *(2025.01)*
***A61P 35/00*** *(2006.01)*

(52) Cooperative Patent Classification (CPC):
**A61K 35/17; A61P 35/00; C12N 5/06**

(86) International application number:
**PCT/CN2024/119388**

(87) International publication number:
**WO 2025/061028 (27.03.2025 Gazette 2025/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.09.2023 CN 202311204655**

(71) Applicant: **Linxcell Biotechnologies**
**Hefei, Anhui 230601 (CN)**

(72) Inventor: **ZHOU, Peng**
**Beijing 100176 (CN)**

(74) Representative: **Weidner Stern Jeschke Patentanwälte Partnerschaft mbB**
**Arnstädter Straße 50**
**99096 Erfurt (DE)**

Remarks:
The complete document including Reference Table(s) and the Sequence Listing(s) can be downloaded from the EPO website

# (54) METHOD FOR MODIFYING CELLS AND USE THEREOF

(57) Provided is a method for preparing a modified cell. The method comprises: 1) contacting a monosaccharide derivative containing a first functional group with an immune cell to obtain an immune cell on which surface the monosaccharide derivative containing the first functional group is expressed; 2) contacting an antigen binding protein with a compound containing a second functional group to obtain the antigen binding protein containing the second functional group; and/or, 3) contacting the immune cell on which surface the monosaccharide derivative containing the first functional group is expressed with the antigen binding protein containing the second functional group, such that the first functional group and the second functional group are connected and reacted to obtain the cell modified with the antigen binding protein on the surface. Also provided are a cell prepared according to the method, and the use thereof in treating diseases.

This international publication:
- contains the international search report (Article 21(3)).
- contains the sequence listing part of the description (Rule 5.2(a)).

EP 4 782 534 A1

## Description

### TECHNICAL FIELD

The present application relates to the field of biomedicine, specifically to a method for modifying cells via metabolic glycan labeling and use thereof.

### BACKGROUND

The incidence and mortality of cancer are increasing year by year. With continuous exploration and practice of the body's immunity, research on immunotherapy has become increasingly intense. Immunocyte therapy, which involves activating and enhancing the immune system to kill tumors, has become the fourth pillar of cancer treatment. Adoptive immunocyte therapies, such as Chimeric Antigen Receptor T (CAR-T) cells, have achieved significant success against hematological tumors, but they suffer from high production costs, long preparation times, and complex processes.

Antibody-Cell Conjugates (ACC) can conjugate antibodies to cell surfaces to enhance the targeting of immune cells, thereby specifically killing tumor cells. However, cell therapies, particularly allogeneic cell therapies, often lead to graft-versus-host disease (GVHD). Furthermore, current methods for conjugating immune cells with antibodies are characterized by long preparation cycles and inconsistent product quality, failing to meet the needs of tumor patients with rapid disease progression. Additional challenges include off-target effects within tumor tissues and tumor immune evasion.

Therefore, developing a novel, convenient, and low-cost immune cell-antibody labeling technology that selectively kills tumor cells without damaging normal cells would greatly advance the field of immunocyte therapies.

### SUMMARY OF THE INVENTION

The present application provides a method for preparing modified cells. Through metabolic labeling with non-natural sugar analogs, tumor antigen-targeting molecules are labeled onto the surface of immune cells via a conjugation reaction, thereby enhancing the killing activity of immune cells against tumor cells. The method of the present application allows for quick and efficient modification of tumor antigen-targeting molecules (e.g., antibodies) onto the surface of immune cells (e.g., $\gamma\delta$ T cells or NK cells). The method features high labeling efficiency, short cycle time, and low economic cost. The modified cells prepared according to the method of the present application exhibit at least one of the following beneficial effects: (1) uniform and stable quality, (2) enhanced enrichment within tumors, (3) low off-target effects and low toxicity/side effects, (4) strong killing capability against target cells, and (4) significant inhibition of tumor cell growth. The methods, cells, pharmaceutical compositions, etc., provided in the present application have substantial clinical application value.

In one aspect, the present application provides a method for preparing modified cells, comprising step S: contacting an immune cell expressing on its surface a monosaccharide derivative containing a first functional group with an antigen-binding protein containing a second functional group, such that the first functional group and the second functional group undergo a conjugation reaction to obtain the modified cells; wherein the monosaccharide derivative comprises any one or more structures selected from Formula I to Formula X:

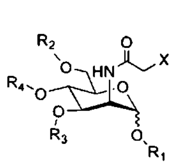
(Formula I)

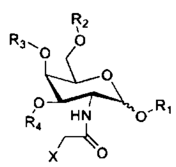
(Formula II)

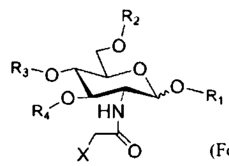
(Formula III)

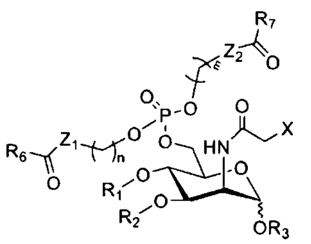
(Formula IV)

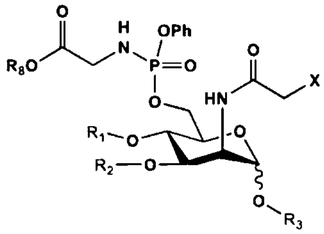
(Forumula V)

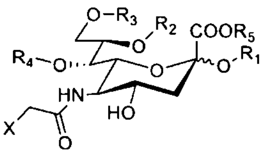
(Formula VI)

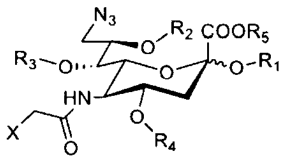
(Formula VII)

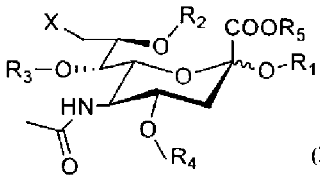
(Formula VIII)

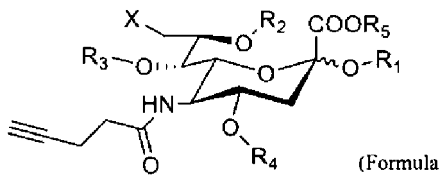
(Formula IX)

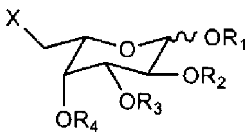
(Formula X)

wherein, X is the first functional group, $R_1$, $R_2$, $R_3$, $R_4$, $R_5$, and $R_6$ are each independently selected from protecting groups that can be hydrolyzed or enzymatically cleaved in cells, with the purpose of facilitating entry of the non-natural sugar into cells and exposing the hydroxyl groups on the sugar after deprotection. Therefore, any group fulfilling this function falls within the scope of the present invention.

In some embodiments, $R_1$, $R_2$, $R_3$, $R_4$, $R_5$, and $R_6$ are each independently selected from the group consisting of hydrogen, halogen, alkyl, cycloalkyl, heterocycloalkyl, alkenyl, alkynyl, aryl, polyethylene glycol, alkylthio, alkylcarbonyl, alkoxycarbonyl, and acyl, wherein said group is optionally substituted with one or more substituents selected from halogen, hydroxyl, amino, nitro, mercapto, and $C_{1-6}$ alkyl. In some further embodiments, $R_1$, $R_2$, $R_3$, $R_4$, $R_5$, and $R_6$ are each independently selected from the group consisting of hydrogen, halogen, $C_{1-6}$ alkyl, $C_{1-6}$ alkylthio, C1-6 alkylcarbonyl, $C_{2-6}$ alkenyl, $C_{2-6}$ alkynyl, $C_{2-6}$ alkoxy, $C_{2-6}$ alkoxycarbonyl, and acyl, wherein said group is optionally substituted with one or more substituents selected from halogen, hydroxyl, amino, nitro, mercapto, and $C_{1-6}$ alkyl.

In some embodiments, $R_1$, $R_2$, $R_3$, $R_4$ in Formula I and Formula II can be the same, for example, selected from acyl and alkoxycarbonyl. In other embodiments, $R_1$, $R_2$, $R_3$, $R_4$ in Formula I and Formula II can be different, for example, each independently selected from acyl and alkoxycarbonyl.

In some embodiments, the monosaccharide derivative comprises any one or more structures selected from the group consisting of

[Image placeholder for structure II-b]

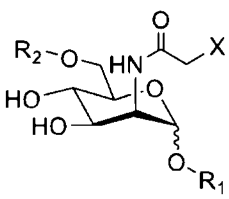
(Formula I-a)

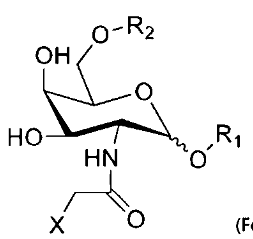
(Formula II-a)

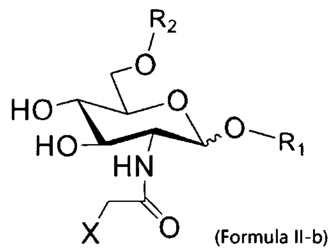
(Formula II-b)

wherein, X is the first functional group, $R_1$ and $R_2$ are each independently selected from the group consisting of hydrogen, halogen, alkyl, cycloalkyl, heterocycloalkyl, alkenyl, alkynyl, aryl, polyethylene glycol, alkylthio, alkylcarbonyl, alkoxycarbonyl, and acyl, wherein said group is optionally substituted with one or more substituents selected from halogen, hydroxyl, amino, nitro, mercapto, and $C_{1-6}$ alkyl. In some further embodiments, $R_1$ and $R_2$ are each independently selected from the group consisting of hydrogen, halogen, $C_{1-6}$ alkyl, $C_{1-6}$ alkylthio, $C_{1-6}$ alkylcarbonyl, $C_{2-6}$ alkenyl, $C_{2-6}$ alkynyl, $C_{2-6}$ alkoxy, $C_{2-6}$ alkoxycarbonyl, and acyl, wherein said group is optionally substituted with one or more substituents selected from halogen, hydroxyl, amino, nitro, mercapto, and $C_{1-6}$ alkyl. In some still further embodiments, $R_1$ and $R_2$ are the same, for example, selected from acetyl, propionyl, and butyryl. In some still further embodiments, $R_1$ and $R_2$ are different, for example, each independently selected from acetyl, propionyl, and butyryl.

In some embodiments, the monosaccharide derivative comprises any one or more structures selected from the group consisting of

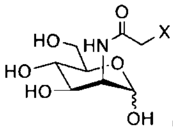 (formula I-a-1) 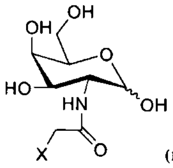 (formula II-a-1) 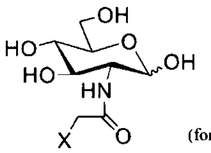 (formula II-b-)

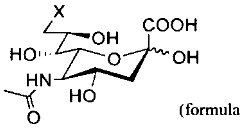 (formula III-1) 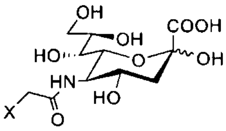 (formula IV-1) 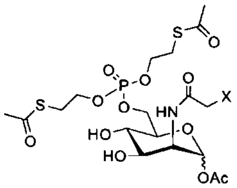 (formula V-1)

wherein X is the first functional group.

In some embodiments, the method further comprises step S1: contacting a monosaccharide derivative containing a first functional group with an immune cell to obtain the immune cell expressing on its surface the monosaccharide derivative containing the first functional group.

In some embodiments, the method further comprises step S2: contacting an antigen-binding protein with a compound containing a second functional group to obtain the antigen-binding protein containing the second functional group.

In some embodiments, the first functional group and/or the second functional group is a group having bioorthogonal reactivity.

In some embodiments, the bioorthogonal reaction comprises at least one selected from the group consisting of click chemistry, photo-crosslinking, photosensitive, and glycosylation.

In some embodiments, the first functional group comprises one or more selected from the group consisting of azido, alkynyl, trans-cyclooctene, triazine, tetrazine, acyl hydrazone, cyclopropene, isonitrile, and cyclooctyne.

In some embodiments, the second functional group comprises one or more selected from the group consisting of azido, alkynyl, trans-cyclooctene, triazine, tetrazine, acyl hydrazone, cyclopropene, isonitrile, and cyclooctyne.

In some embodiments, $R_1$, $R_2$, $R_3$, and $R_4$ are each independently selected from the group consisting of hydrogen, halogen, substituted or unsubstituted $C_{1-6}$ alkyl, $C_{1-6}$ alkylthio, $C_{1-6}$ alkylcarbonyl, $C_{2-6}$ alkenyl, $C_{2-6}$ alkynyl, $C_{2-6}$ alkoxy, $C_{2-6}$ alkoxycarbonyl, and acyl, wherein said group is optionally substituted with a substituent selected from halogen, hydroxyl, amino, nitro, mercapto, and $C_{1-6}$ alkyl; $R_5$ is selected from the group consisting of hydrogen, halogen, and substituted or unsubstituted $C_{1-6}$ alkyl, wherein said alkyl is optionally substituted with a substituent selected from halogen, hydroxyl, amino, nitro, and mercapto; $R_6$ and $R_7$ are each independently selected from the group consisting of halogen and substituted or unsubstituted $C_{1-10}$ alkyl, wherein said alkyl is optionally substituted with a substituent selected from halogen, hydroxyl, amino, nitro, and mercapto; **$R_8$ is selected from the group consisting of halogen, substituted or unsubstituted $C_{1-10}$ alkyl, phenyl, and substituted or unsubstituted $C_{1-10}$ alkylene-phenyl,** wherein said alkyl is optionally substituted with a substituent selected from halogen, hydroxyl, amino, nitro, and mercapto; $Z_1$ and $Z_2$ are each independently selected from O and S; n and m are each independently selected from the integers 1, 2, 3, 4, 5, and 6.

In some embodiments, $R_1$ is selected from the group consisting of hydrogen and -C(=O)-$C_{1-6}$ alkyl. For example, $R_1$ is hydrogen or -C(=O)-$CH_3$. In some embodiments, $R_2$ is selected from the group consisting of hydrogen and -C(=O)-C1-6 alkyl. For example, $R_2$ is hydrogen or - C(=O)-$CH_3$. In some embodiments, $R_3$ is selected from the group consisting of hydrogen and - C(=O)-$C_{1-6}$ alkyl. For example, $R_3$ is hydrogen or -C(=O)-$CH_3$. In some embodiments, $R_4$ is selected from the group consisting of hydrogen and -C(=O)-$C_{1-6}$ alkyl. For example, $R_4$ is hydrogen or -C(=O)-$CH_3$. In some embodiments, $R_5$ is selected from the group consisting of hydrogen and substituted or unsubstituted $C_{1-6}$ alkyl. In some embodiments, $R_5$ is hydrogen or substituted or unsubstituted $C_{1-3}$ alkyl. For example, $R_4$ is hydrogen or -$CH_3$. In some embodiments, $R_6$ is selected from the group consisting of hydrogen and substituted or unsubstituted $C_{1-10}$ alkyl.

In some embodiments, $R_7$ is selected from the group consisting of hydrogen and substituted or unsubstituted $C_{1-10}$ alkyl. In some embodiments, $R_8$ is selected from the group consisting of methyl, ethyl, and -$CH_2$-phenyl.

In some embodiments, m is 1, 2, or 3.

In some embodiments, n is 1, 2, or 3.

In some embodiments, in Formula I, $R_1$ is selected from hydrogen and -C(=O)-$C_{1-6}$ alkyl, $R_2$ is selected from hydrogen and -C(=O)-$C_{1-6}$ alkyl, $R_3$ is selected from hydrogen and -C(=O)-$C_{1-6}$ alkyl, $R_4$ is selected from hydrogen and -C(=O)-$C_{1-6}$ alkyl, and $R_1$, $R_2$, $R_3$, and $R_4$ are not all hydrogen simultaneously.

In some embodiments, in Formula II, $R_1$ is selected from hydrogen and -C(=O)-$C_{1-6}$ alkyl, $R_2$ is selected from hydrogen and -C(=O)-$C_{1-6}$ alkyl, $R_3$ is selected from hydrogen and -C(=O)-$C_{1-6}$ alkyl, $R_4$ is selected from hydrogen and -C(=O)-$C_{1-6}$ alkyl, and $R_1$, $R_2$, $R_3$, and $R_4$ are not all hydrogen simultaneously.

In some embodiments, in Formula III, $R_1$ is selected from hydrogen and -C(=O)-$C_{1-6}$ alkyl, $R_2$ is selected from hydrogen and -C(=O)-$C_{1-6}$ alkyl, $R_3$ is selected from hydrogen and -C(=O)-$C_{1-6}$ alkyl, $R_4$ is selected from hydrogen and -C(=O)-$C_{1-6}$ alkyl, and $R_1$, $R_2$, $R^3$, and $R_4$ are not all hydrogen simultaneously.

In some embodiments, in Formula IV, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_6$ is selected from $C_{1-10}$ alkyl, $R_7$ is selected from $C_{1-10}$ alkyl, $Z_1$ is selected from O and S, $Z_2$ is selected from O and S, n is 1, 2, or 3, m is 1, 2, or 3, and $R_1$, $R_2$, and $R_3$ are not all hydrogen simultaneously.

In some embodiments, in Formula V, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_8$ is selected from $C_{1-3}$ alkyl, phenyl, $C_{1-3}$ alkylene-phenyl, and $R_1$, $R_2$, and $R_3$ are not all hydrogen simultaneously.

In some embodiments, in Formula VI, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_4$ is selected from hydrogen and -C(=O)-$C_{1-6}$ alkyl (preferably -C(=O)-$CH_3$), $R_5$ is selected from hydrogen and $C_{1-6}$ alkyl (preferably -$CH_3$), and $R_1$, $R_2$, $R_3$, and $R_4$ are not all hydrogen simultaneously, or $R_1$, $R_2$, $R_3$, $R_4$, $R_5$ are not all hydrogen simultaneously. In a specific embodiment, at most four of $R_1$, $R_2$, $R_3$, $R_4$, $R_5$ are hydrogen.

In some embodiments, in Formula VII, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_4$ is selected from hydrogen and -C(=O)-$C_{1-6}$ alkyl (preferably -C(=O)-$CH_3$), $R_5$ is selected from hydrogen and $C_{1-6}$ alkyl (preferably -$CH_3$), and $R_1$, $R_2$, $R_3$, and $R_4$ are not all hydrogen simultaneously, or $R_1$, $R_2$, $R_3$, $R_4$, $R_5$ are not all hydrogen simultaneously. In a specific embodiment, at most four of $R_1$, $R_2$, $R_3$, $R_4$, $R_5$ are hydrogen.

In some embodiments, in Formula VIII, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_4$ is selected from hydrogen and -C(=O)-$C_{1-6}$ alkyl (preferably -C(=O)-$CH_3$), $R_5$ is selected from hydrogen and $C_{1-6}$ alkyl (preferably -$CH_3$), and $R_1$, $R_2$, $R_3$, and $R_4$ are not all hydrogen simultaneously, or $R_1$, $R_2$, $R_3$, $R_4$, $R_5$ are not all hydrogen simultaneously. In a specific embodiment, at most four of $R_1$, $R_2$, $R_3$, $R_4$, $R_5$ are hydrogen.

In some embodiments, in Formula IX, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_4$ is selected from hydrogen and -C(=O)-$C_{1-6}$ alkyl (preferably -C(=O)-$CH_3$), and $R_1$, $R_2$, $R_3$, and $R_4$ are not all hydrogen simultaneously, or $R_1$, $R_2$, $R_3$, $R_4$, $R_5$ are not all hydrogen simultaneously. In a specific embodiment, at most four of $R_1$, $R_2$, $R_3$, $R_4$, $R_5$ are hydrogen.

In some embodiments, in Formula X, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), and $R_4$ is selected from hydrogen and -C(=O)-$C_{1-6}$ alkyl (preferably -C(=O)-$CH_3$). In a specific embodiment, $R_1$, $R_2$, $R_3$, and $R_4$ are all hydrogen. In a specific embodiment, $R_1$, $R_2$, $R_3$, and $R_4$ are all -C(=O)-$C_{1-6}$ alkyl (preferably -C(=O)-$CH_3$).

In some embodiments, the monosaccharide derivative containing the first functional group is selected from any one in Table A below:

Table A

| No. | Formula | Structure |
|---|---|---|
| 1 | ManNAz | 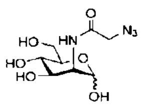 |
| 2 | 1,6-Ac2ManNAz | 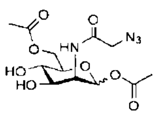 |

(continued)

| No. | Formula | Structure |
|---|---|---|
| 3 | 1,6-Pr2ManNAz | 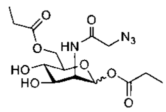 |
| 4 | 1,6-Bu2ManNAz | 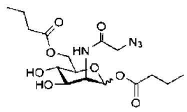 |
| 5 | Ac4ManNAz | 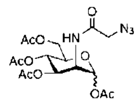 |
| 6 | GlcNAz | 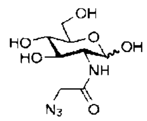 |
| 7 | 1,6-Ac2GalNAz | 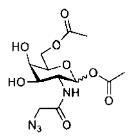 |
| 8 | 1,6-Pr2GalNAz | 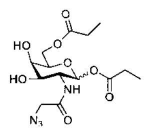 |
| 9 | 1,6-Bu2GalNAz | 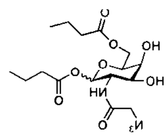 |
| 10 | GalNAz | 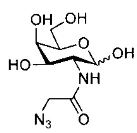 |
| 11 | 1,6-Ac2GlcNAz | 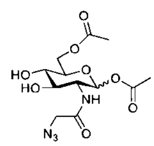 |
| 12 | 1,6-Pr2GlcNAz | 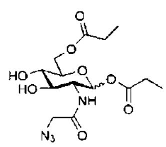 |
| 13 | 1,6-Bu2GlcNAz | 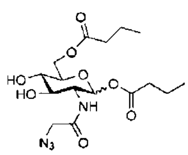 |

(continued)

| No. | Formula | Structure |
|---|---|---|
| 14 | Ac4GlcNAz | 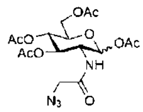 |
| 15 | Ac4GalNAz | 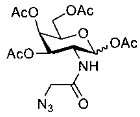 |
| 16 | 1-O-Ac-ManNAz-6-bis(Et-SATE)-P | 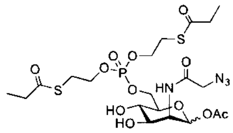 |
| 17 | 1-O-Ac-ManNAz-6-bis(Bu-SATE)-P | 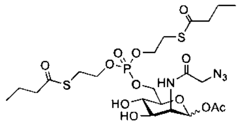 |
| 18 | 1-O-Ac-ManNAz-6-bis(tBu-SATE)-P | 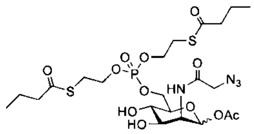 |
| 19 | 1-O-Ac-ManNAz-6-bis(Hep-SATE)-P | 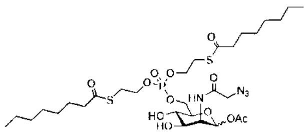 |
| 20 | 1-O-Ac-6-bis(Me-SATE)-P-ManNAz | 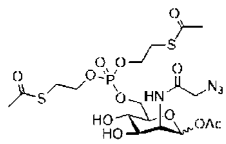 |
| 21 | 1-O-Ac-6-bis-POM-P-ManNAz | 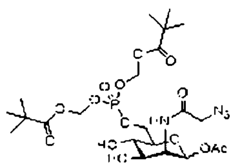 |
| 22 | 1-O-Pr-6-bis-POM-P-ManNAz | 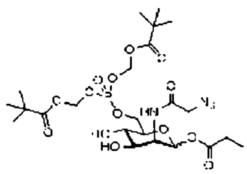 |
| 23 | Ac3-6-bis(Me-SATE)-P-ManNAz | 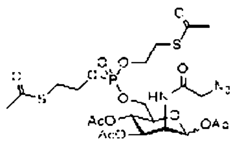 |

(continued)

| No. | Formula | Structure |
|---|---|---|
| 24 | 1-O-Pr-6-(Gly-OMe-ProTide)-P-ManNAz | 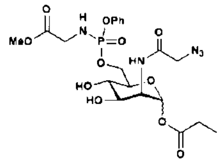 |
| 25 | 1-O-Pr-6-(Leu-OEt-ProTide)-P-ManNAz | 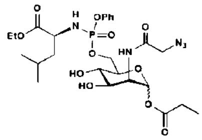 |
| 26 | 1-O-Pr-6-(Ala-OMe-ProTide)-P-ManNAz | 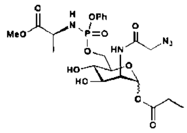 |
| 27 | 1-O-Pr-6-(Ala-OEt-ProTide)-P-ManNAz | 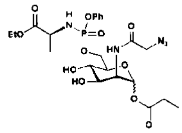 |
| 28 | 1-O-Pr-6-(Ala-OBn-ProTide)-P-ManNAz | 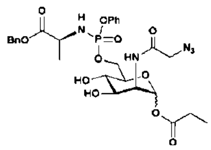 |
| 29 | SiaNAz | 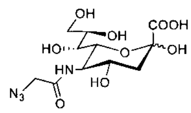 |
| 30 | 9AzSiaNAz | 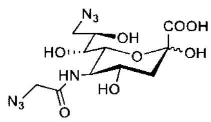 |
| 31 | 9AzSia | 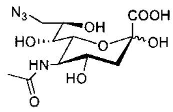 |
| 32 | 9AzSiaNAl | 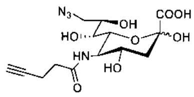 |
| 33 | SiaNAz1Met | 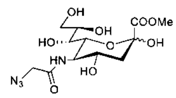 |
| 34 | 9AzSiaNAz1Met | 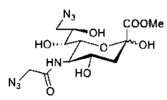 |
| 35 | 9AzSia1Met | 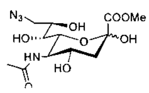 |

(continued)

| No. | Formula | Structure |
|---|---|---|
| 36 | 9AzSiaNAl1Met | 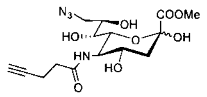 |
| 37 | Ac5SiaNAz1Met | 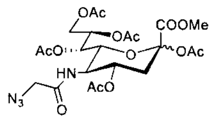 |
| 38 | Ac49AzSiaNAz1Met | 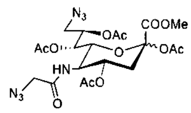 |
| 39 | Ac49AzSia1Met | 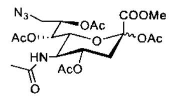 |
| 40 | Ac49AzSiaNAl1Met | 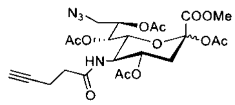 |
| 41 | 6AzFucose | 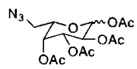 |
| 42 | Ac36AzFucose | 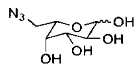 |

In some embodiments, the first functional group is azido, and the second functional group is cyclooctyne. In some embodiments, the first functional group is azido, and the second functional group is selected from the group consisting of OCT, ALO, MOFO, DBCO, DIFO, DIMAC, and BCN. In some embodiments, the first functional group is azido, and the second functional group is

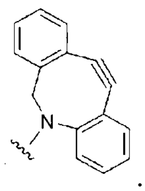

In some embodiments, the monosaccharide derivative is selected from any one of the group consisting of

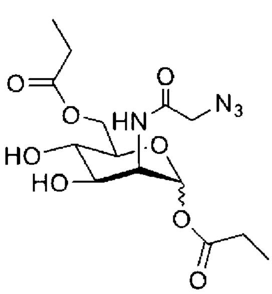

(compound A)

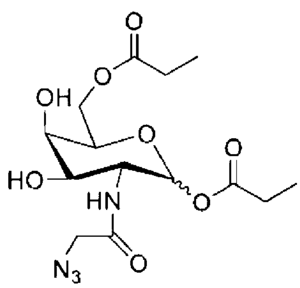

(compound B)

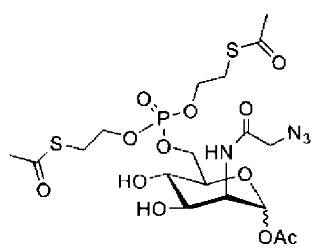

(compound C)

In some embodiments, the compound containing the second functional group further comprises a group capable of being covalently linked to a thiol group, preferably, said group capable of being covalently linked to a thiol group is a

succinimidyl ester group, a maleimide group, or a maleic anhydride group.

In some embodiments, step S2 comprises contacting the antigen-binding protein with a compound having a structure shown in Formula L1, Formula L2, or Formula L3, to obtain the antigen-binding protein containing the second functional group:

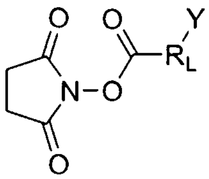

(Formula L1)

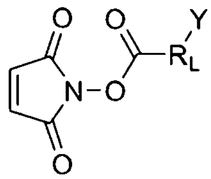

(Formula L2)

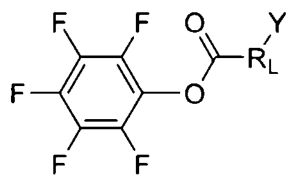

(Formula L3)

wherein,

Y represents the second functional group, $R_L$ is selected from the group consisting of polyethylene glycol, amide group, and alkyl ether group, preferably, $R_L$ is selected from $C_{1-20}$ alkylene or $C_{1-20}$ alkylene ether group.

In some embodiments, the compound of Formula L1 is DBCO-PEG4-NHS:

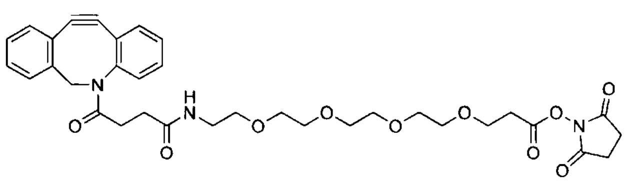

DBCO-PEG$_4$-NHS

In some embodiments, the compound of Formula L2 is DBC0-PEG4-MAL:

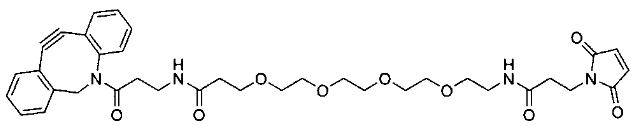

DBC0-PEG4-MAL

In some embodiments, the immune cells comprise NK cells, DC cells, NKT cells, monocytes, macrophages, T cells, and/or B cells. In some embodiments, the immune cells are NK cells. In some embodiments, the immune cells are T cells, particularly γδ T cells. In some embodiments, the γδ T cells comprise Vγ9Vδ2 and/or Vδ1 T cells.

In some embodiments, the antigen-binding protein comprises an antibody or antigen-binding fragment thereof.

In some embodiments, the antibody comprises a monoclonal antibody, chimeric antibody, humanized antibody, or fully human antibody.

In some embodiments, the antigen-binding protein comprises a Fab, Fab', F(ab)2, Fv fragment, F(ab')2, scFv, di-scFv, VHH, and/or dAb.

In some embodiments, the antigen-binding protein is capable of specifically binding a tumor antigen. In some embodiments, the tumor antigen comprises a tumor-associated antigen and/or a tumor-specific antigen. In some embodiments, the tumor antigen is selected from the group consisting of CD20 and/or claudin18.2.

In some embodiments, the antigen-binding protein is an anti-CD20 antibody. For example, the anti-CD20 antibody is rituximab. For example, the anti-CD20 antibody comprises HCDR1-3 of rituximab. For example, the anti-CD20 antibody comprises LCDR1-3 of rituximab. For example, the anti-CD20 antibody comprises the light chain variable region of rituximab. For example, the anti-CD20 antibody comprises the heavy chain variable region of rituximab.

In some embodiments, the antigen-binding protein is an anti-claudin18.2 antibody. For example, the anti-claudin18.2 antibody is the hu7v3 antibody. For example, the anti-claudin18.2 antibody comprises HCDR1-3 of the hu7v3 antibody. For example, the anti-claudin18.2 antibody comprises the heavy chain variable region of the hu7v3 antibody.

In some embodiments, the immune cells are derived from a human sample. In some embodiments, the immune cells are derived from human peripheral blood.

In some embodiments, in step S of the method, the contact time between the immune cell expressing on its

surface the monosaccharide derivative containing the first functional group and the antigen-binding protein containing the second functional group is about 10 min to 60 min, for example, about 20 min to 40 min, or about 30 min. In some embodiments, in step S of the method, the concentration of the second functional group contained in the antigen-binding protein is about 10-30 μM, for example about 20 μM, and the density of the immune cell expressing on its surface the monosaccharide derivative containing the first functional group does not exceed $1\times10^7$ cells/mL.

In some embodiments, in step S1 of the method, the contact time between the monosaccharide derivative containing the first functional group and the immune cell is about 2 h to 48 h, for example, about 16 h to 32 h, for example, about 24 h.

In some embodiments, in step S2 of the method, the contact time between the antigen-binding protein and the compound containing the structure shown in Formula L1 and/or Formula L2 is about 30 min to 6 h, for example, about 1 h to 2 h, for example, about 1 h.

In another aspect, the present application provides a modified cell prepared according to the method described above. In some embodiments, the modified cell is an antibody-modified γδ T cell.

In another aspect, the present application provides a modified cell, wherein the modified cell is linked on its surface to an antigen-binding protein via a monosaccharide derivative containing an azido group, wherein the structure of the monosaccharide derivative containing the azido group is selected from one or more of the group consisting of:

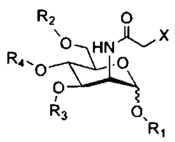
(Formula I)

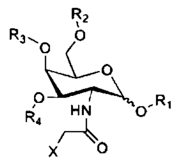
(Formula II)

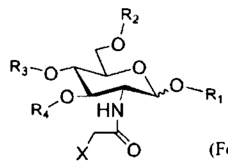
(Formula III)

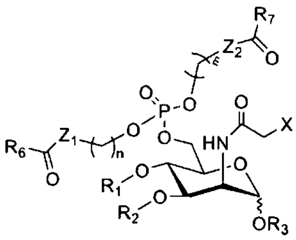
(Formula IV)

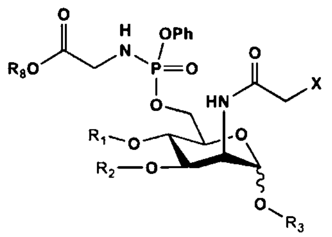
(Forumula V)

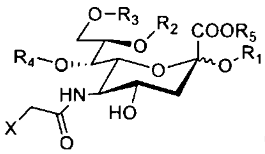
(Formula VI)

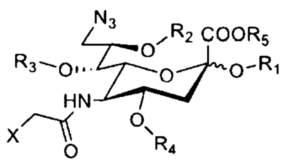
(Formula VII)

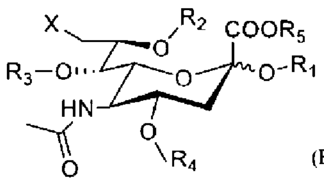
(Formula VIII)

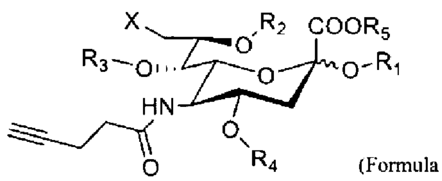
(Formula IX)

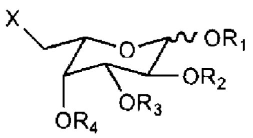
(Formula X)

wherein, X is an azido group ($-N_3$),

$R_1$, $R_2$, $R_3$, and $R_4$ are each independently selected from the group consisting of hydrogen, halogen, substituted or unsubstituted $C_{1-6}$ alkyl, $C_{1-6}$ alkylthio, $C_{1-6}$ alkylcarbonyl, $C_{2-6}$ alkenyl, $C_{2-6}$ alkynyl, $C_{2-6}$ alkoxy, $C_{2-6}$ alkoxycarbonyl, and acyl, wherein said group is optionally substituted with a substituent selected from halogen, hydroxyl, amino, nitro, mercapto, and $C_{1-6}$ alkyl;

$R_5$ is selected from the group consisting of hydrogen, halogen, and substituted or unsubstituted $C_{1-6}$ alkyl, wherein

said alkyl is optionally substituted with a substituent selected from halogen, hydroxyl, amino, nitro, and mercapto;
$R_6$ and $R_7$ are each independently selected from the group consisting of halogen and substituted or unsubstituted $C_{1-10}$ alkyl, wherein said alkyl is optionally substituted with a substituent selected from halogen, hydroxyl, amino, nitro, and mercapto;
$R_8$ is selected from the group consisting of halogen, substituted or unsubstituted $C_{1-10}$ alkyl, phenyl, and substituted or unsubstituted $C_{1-10}$ alkylene-phenyl, wherein said alkyl is optionally substituted with a substituent selected from halogen, hydroxyl, amino, nitro, and mercapto;
$Z_1$ and $Z^2$ are each independently selected from O and S;
n and m are each independently selected from the integers 1, 2, 3, 4, 5, and 6.

In some embodiments, $R_1$ is selected from the group consisting of hydrogen and -C(=O)-$C_{1-6}$ alkyl. For example, $R_1$ is hydrogen or -C(=O)-$CH_3$.

In some embodiments, $R_2$ is selected from the group consisting of hydrogen and -C(=O)-$C_{1-6}$ alkyl. For example, $R_2$ is hydrogen or -C(=O)-$CH_3$.

In some embodiments, $R_3$ is selected from the group consisting of hydrogen and -C(=O)-$C_{1-6}$ alkyl. For example, $R_3$ is hydrogen or -C(=O)-$CH_3$.

In some embodiments, $R_4$ is selected from the group consisting of hydrogen and -C(=O)-$C_{1-6}$ alkyl. For example, $R_4$ is hydrogen or -C(=O)-$CH_3$.

In some embodiments, $R_5$ is selected from the group consisting of hydrogen and substituted or unsubstituted $C_{1-6}$ alkyl. In some embodiments, $R_5$ is hydrogen or substituted or unsubstituted $C_{1-3}$ alkyl. For example, $R_4$ is hydrogen or -$CH_3$.

In some embodiments, $R_6$ is selected from the group consisting of hydrogen and substituted or unsubstituted $C_{1-10}$ alkyl.

In some embodiments, $R_7$ is selected from the group consisting of hydrogen and substituted or unsubstituted $C_{1-10}$ alkyl.

In some embodiments, $R_8$ is selected from the group consisting of methyl, ethyl, and -$CH_2$-phenyl.

In some embodiments, m is 1, 2, or 3.

In some embodiments, n is 1, 2, or 3.

In some embodiments, in Formula I, $R_1$ is selected from hydrogen and -C(=O)-$C_{1-6}$ alkyl, $R_2$ is selected from hydrogen and -C(=O)-$C_{1-6}$ alkyl, $R_3$ is selected from hydrogen and -C(=O)-$C_{1-6}$ alkyl, $R_4$ is selected from hydrogen and -C(=O)-$C_{1-6}$ alkyl, and $R_1$, $R_2$, $R_3$, and $R_4$ are not all hydrogen simultaneously.

In some embodiments, in Formula II, $R_1$ is selected from hydrogen and -C(=O)-$C_{1-6}$ alkyl, $R_2$ is selected from hydrogen and -C(=O)-$C_{1-6}$ alkyl, $R_3$ is selected from hydrogen and -C(=O)-$C_{1-6}$ alkyl, $R_4$ is selected from hydrogen and -C(=O)-$C_{1-6}$ alkyl, and $R_1$, $R_2$, $R_3$, and $R_4$ are not all hydrogen simultaneously.

In some embodiments, in Formula III, $R_1$ is selected from hydrogen and -C(=O)-$C_{1-6}$ alkyl, $R_2$ is selected from hydrogen and -C(=O)-$C_{1-6}$ alkyl, $R_3$ is selected from hydrogen and -C(=O)-$C_{1-6}$ alkyl, $R_4$ is selected from hydrogen and -C(=O)-$C_{1-6}$ alkyl, and $R_1$, $R_2$, $R_3$, and $R_4$ are not all hydrogen simultaneously.

In some embodiments, in Formula IV, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_6$ is selected from $C_{1-10}$ alkyl, $R_7$ is selected from $C_{1-10}$ alkyl, $Z_1$ is selected from O and S, $Z_2$ is selected from O and S, n is 1, 2, or 3, m is 1, 2, or 3, and $R_1$, $R_2$, and $R_3$ are not all hydrogen simultaneously.

In some embodiments, in Formula V, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_8$ is selected from $C_{1-3}$ alkyl, phenyl, $C_{1-3}$ alkylene-phenyl, and $R_1$, $R_2$, and $R_3$ are not all hydrogen simultaneously.

In some embodiments, in Formula VI, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_4$ is selected from hydrogen and -C(=O)-$C_{1-6}$ alkyl (preferably -C(=O)-$CH_3$), $R_5$ is selected from hydrogen and $C_{1-6}$ alkyl (preferably -$CH_3$), and $R_1$, $R_2$, $R_3$, and $R_4$ are not all hydrogen simultaneously, or $R_1$, $R_2$, $R_3$, $R_4$, $R_5$ are not all hydrogen simultaneously. In a specific embodiment, at most four of $R_1$, $R_2$, $R_3$, $R_4$, $R_5$ are hydrogen.

In some embodiments, in Formula VII, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_4$ is selected from hydrogen and -C(=O)-$C_{1-6}$ alkyl (preferably -C(=O)-$CH_3$), $R_5$ is selected from hydrogen and $C_{1-6}$ alkyl (preferably -$CH_3$), and $R_1$, $R_2$, $R_3$, and $R_4$ are not all hydrogen simultaneously, or $R_1$, $R_2$, $R_3$, $R_4$, $R_5$ are not all hydrogen simultaneously. In a specific embodiment, at most four of $R_1$, $R_2$, $R_3$, $R_4$, $R_5$ are hydrogen.

In some embodiments, in Formula VIII, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_4$ is selected from hydrogen and -C(=O)-$C_{1-6}$ alkyl (preferably -C(=O)-$CH_3$), $R_5$ is selected from hydrogen and $C_{1-6}$ alkyl (preferably -$CH_3$), and $R_1$, $R_2$, $R_3$, and $R_4$ are not all hydrogen simultaneously, or $R_1$, $R_2$, $R_3$, $R_4$, $R_5$ are not all hydrogen simultaneously. In a specific embodiment, at most four of $R_1$, $R_2$, $R_3$, $R_4$, $R_5$ are hydrogen.

In some embodiments, in Formula IX, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_4$ is selected from hydrogen and -C(=O)-$C_{1-6}$ alkyl (preferably -C(=O)-$CH_3$), $R_5$ is selected from hydrogen and $C_{1-6}$ alkyl (preferably -$CH_3$), and $R_1$, $R_2$, $R_3$, and $R_4$ are not all hydrogen simultaneously, or $R_1$, $R_2$, $R_3$, $R_4$, $R_5$ are not all hydrogen simultaneously. In a specific embodiment, at most four of $R_1$, $R_2$, $R_3$, $R_4$, $R_5$ are hydrogen.

In some embodiments, in Formula X, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), and $R_4$ is selected from hydrogen and -C(=O)-$C_{1-6}$ alkyl (preferably -C(=O)-$CH_3$). In a specific embodiment, $R_1$, $R_2$, $R_3$, and $R_4$ are all hydrogen. In a specific embodiment, $R_1$, $R_2$, $R_3$, and $R_4$ are all -C(=O)-$C_{1-6}$ alkyl (preferably -C(=O)-$CH_3$).

In some embodiments, the monosaccharide derivative containing the first functional group is selected from any one in Table A.

In some embodiments, the immune cells comprise NK cells, DC cells, NKT cells, monocytes, macrophages, T cells, and/or B cells. In some embodiments, the immune cells NK cells. In some embodiments, the immune cells are T cells, particularly γδ T cells. In some embodiments, the γδ T cells comprise Vγ9Vδ2 and/or Vδ1 T cells.

In some embodiments, the antigen-binding protein comprises an antibody or antigen-binding fragment thereof.

In some embodiments, the antibody comprises a monoclonal antibody, chimeric antibody, humanized antibody, or fully human antibody.

In some embodiments, the antigen-binding protein comprises a Fab, Fab', F(ab)2, Fv fragment, F(ab')2, scFv, di-scFv, VHH, and/or dAb.

In some embodiments, the antigen-binding protein is capable of specifically binding to a tumor antigen. In some embodiments, the tumor antigen comprises a tumor-associated antigen and/or a tumor-specific antigen.

In some embodiments, the antigen-binding protein is capable of specifically binding a tumor antigen selected from the group consisting of CEA (carcinoembryonic antigen), CA-125 (cancer antigen 125), CA-19-9 (cancer antigen 19-9), CA-15-3 (cancer antigen 15-3), PSA (prostate-specific antigen), HER2 (human epidermal growth factor receptor 2), EGFR (epidermal growth factor receptor), PD-L1 (programmed death-ligand 1), CTLA-4 (cytotoxic T-lymphocyte-associated protein 4), CD56 (neural cell adhesion molecule), MUC1 (mucin 1), Glypican-3, WT1 (Wilms tumor protein 1), NY-ESO-1 (New York esophageal squamous cell carcinoma 1), 5T4 (tumor-associated antigen 5T4), α-Fetoprotein, Gastrin-releasing peptide, Carcinoembryonic antigen, HER2, EGFR, PD-L1, CTLA-4, CD20 (B lymphocyte surface antigen), CD30 (activated lymphocyte surface antigen), CD56, CD38 (monoclonal antibody target on T cell surface), CD19 (monoclonal antibody target on B lymphocyte surface), CD33 (monoclonal antibody target on myeloid cell surface), CD70 (T cell co-stimulatory molecule), CD47 (immunosuppressive molecule), CD117 (c-Kit, stem cell factor receptor), CD44 (cell surface adhesion molecule), CD123 (IL-3 receptor alpha chain), CD52 (monoclonal antibody target on T cell surface), CD34 (monoclonal antibody target on hematopoietic stem cell surface), CD138 (B cell maturation antigen), CD79b (B cell surface antigen), and BCMA (B cell maturation antigen).

In some embodiments, the tumor antigen is selected from the group consisting of CD20 and/or claudin18.2.

In some embodiments, the antigen-binding protein comprises an antibody heavy chain variable region HCDR3, wherein the HCDR3 comprises the amino acid sequence shown in SEQ ID NO: 3, or wherein the HCDR3 comprises an amino acid sequence having at least 80%, at least 85%, at least 90%, at least 95%, or at least 98% sequence identity to the amino acid sequence shown in SEQ ID NO: 3. For example, the CDRs are numbered according to the Kabat scheme.

In some embodiments, the antigen-binding protein comprises an antibody heavy chain variable region HCDR2, wherein the HCDR2 comprises the amino acid sequence shown in SEQ ID NO: 2, or wherein the HCDR2 comprises an amino acid sequence having at least 80%, at least 85%, at least 90%, at least 95%, or at least 98% sequence identity to the amino acid sequence shown in SEQ ID NO: 3. For example, the CDRs are numbered according to the Kabat scheme.

In some embodiments, the antigen-binding protein comprises an antibody heavy chain variable region HCDR1, wherein the HCDR1 comprises the amino acid sequence shown in SEQ ID NO: 1, or wherein the HCDR1 comprises an amino acid sequence having at least 80%, at least 85%, at least 90%, at least 95%, or at least 98% sequence identity to the amino acid sequence shown in SEQ ID NO: 1. For example, the CDRs are numbered according to the Kabat scheme.

In some embodiments, the antigen-binding protein comprises an antibody heavy chain variable region, wherein the antibody heavy chain variable region comprises the amino acid sequence shown in SEQ ID NO: 4, or wherein the antibody heavy chain variable region comprises an amino acid sequence having at least 80%, at least 85%, at least 90%, at

least 95%, or at least 98% sequence identity to the amino acid sequence shown in SEQ ID NO: 4.

In some embodiments, the antigen-binding protein comprises an antibody light chain variable region LCDR3, wherein the LCDR3 comprises the amino acid sequence shown in SEQ ID NO: 7, or wherein the LCDR3 comprises an amino acid sequence having at least 80%, at least 85%, at least 90%, at least 95%, or at least 98% sequence identity to the amino acid sequence shown in SEQ ID NO: 7. For example, the CDRs are numbered according to the Kabat scheme.

In some embodiments, the antigen-binding protein comprises an antibody light chain variable region LCDR2, wherein the LCDR2 comprises the amino acid sequence shown in SEQ ID NO: 6, or wherein the LCDR2 comprises an amino acid sequence having at least 80%, at least 85%, at least 90%, at least 95%, or at least 98% sequence identity to the amino acid sequence shown in SEQ ID NO: 6. For example, the CDRs are numbered according to the Kabat scheme.

In some embodiments, the antigen-binding protein comprises an antibody light chain variable region LCDR1, wherein the LCDR1 comprises the amino acid sequence shown in SEQ ID NO: 5, or wherein the LCDR1 comprises an amino acid sequence having at least 80%, at least 85%, at least 90%, at least 95%, or at least 98% sequence identity to the amino acid sequence shown in SEQ ID NO: 5. For example, the CDRs are numbered according to the Kabat scheme.

In some embodiments, the antigen-binding protein comprises an antibody light chain variable region, wherein the antibody light chain variable region comprises the amino acid sequence shown in SEQ ID NO: 8, or wherein the antibody light chain variable region comprises an amino acid sequence having at least 80%, at least 85%, at least 90%, at least 95%, or at least 98% sequence identity to the amino acid sequence shown in SEQ ID NO: 8.

In some embodiments, the antigen-binding protein comprises rituximab.

In some embodiments, the antigen-binding protein comprises an antibody heavy chain variable region HCDR3, wherein the HCDR3 comprises the amino acid sequence shown in SEQ ID NO: 11, or wherein the HCDR3 comprises an amino acid sequence having at least 80%, at least 85%, at least 90%, at least 95%, or at least 98% sequence identity to the amino acid sequence shown in SEQ ID NO: 11. For example, the CDRs are numbered according to the IMGT scheme.

In some embodiments, the antigen-binding protein comprises an antibody heavy chain variable region HCDR2, wherein the HCDR2 comprises the amino acid sequence shown in SEQ ID NO: 10, or wherein the HCDR2 comprises an amino acid sequence having at least 80%, at least 85%, at least 90%, at least 95%, or at least 98% sequence identity to the amino acid sequence shown in SEQ ID NO: 10. For example, the CDRs are numbered according to the IMGT scheme.

In some embodiments, the antigen-binding protein comprises an antibody heavy chain variable region HCDR1, wherein the HCDR1 comprises the amino acid sequence shown in SEQ ID NO: 9, or wherein the HCDR1 comprises an amino acid sequence having at least 80%, at least 85%, at least 90%, at least 95%, or at least 98% sequence identity to the amino acid sequence shown in SEQ ID NO: 9. For example, the CDRs are numbered according to the IMGT scheme.

In some embodiments, the antigen-binding protein comprises an antibody heavy chain variable region, wherein the antibody heavy chain variable region comprises the amino acid sequence shown in SEQ ID NO: 12, or wherein the antibody heavy chain variable region comprises an amino acid sequence having at least 80%, at least 85%, at least 90%, at least 95%, or at least 98% sequence identity to the amino acid sequence shown in SEQ ID NO: 12.

In some embodiments, the antigen-binding protein is a VHH.

In some embodiments, the antigen-binding protein comprises an antibody heavy chain, wherein the antibody heavy chain comprises the amino acid sequence shown in SEQ ID NO: 13, or wherein the antibody heavy chain comprises an amino acid sequence having at least 80%, at least 85%, at least 90%, at least 95%, or at least 98% sequence identity to the amino acid sequence shown in SEQ ID NO: 13.

In some embodiments, the antigen-binding protein comprises an Fc. For example, the Fc is from IgG1.

In some embodiments, the antigen-binding protein is a fusion protein of VHH and Fc.

In some embodiments, the antigen-binding protein comprises the Hu7v3 antibody.

In some embodiments, the cell has azido groups expressed on its surface via the metabolic glycan pathway by uptake of the monosaccharide derivative.

In some embodiments, the cell is linked to the antigen-binding protein bearing the second functional group via the azido group of the monosaccharide derivative, preferably linked via a bond formed by a conjugation reaction between the azido group and the second functional group.

In some embodiments, the second functional group comprises

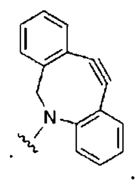

In some embodiments, the structure of is as shown in Formula L1, Formula L2, or Formula L3:

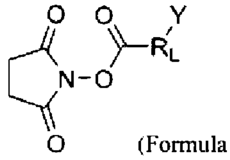 (Formula L1) 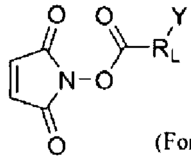 (Formula L2) 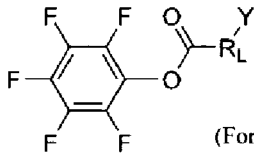 (Formula L3)

wherein, Y is the second functional group, $R_L$ is selected from the group consisting of polyethylene glycol, amide group, and alkyl ether group, preferably, $R_L$ is selected from $C_{1-20}$ alkylene or $C_{1-20}$ alkylene ether group.

In some embodiments, the structure of the second functional group is selected from the group consisting of

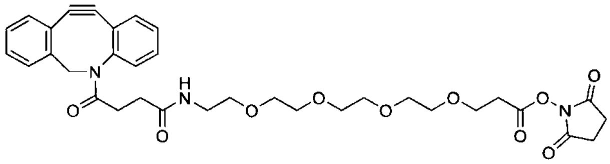

DBCO-PEG4-NHS

and

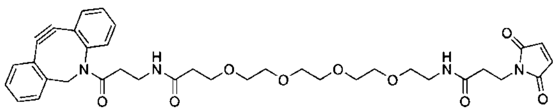

DBCO-PEG4-MAL>

In another aspect, the present application provides a pharmaceutical composition comprising the modified cell described above, and optionally a pharmaceutically acceptable adjuvant.

In another aspect, the present application provides the use of the modified cell in the manufacture of a medicament for treating and/or alleviating tumors.

In another aspect, the present application provides a method for treating and/or alleviating tumors, comprising administering the modified cell to a subject in need thereof.

In some embodiments, the tumor comprises a solid tumor. In some embodiments, the tumor comprises a hematologic tumor.

In some embodiments, the tumor includes lung cancer, breast cancer, colorectal cancer, prostate cancer, gastric cancer, liver cancer, pancreatic cancer, ovarian cancer, cervical cancer, thyroid cancer, melanoma, lymphoma, leukemia, brain tumor, bone cancer, kidney cancer, bladder cancer, esophageal cancer, nasopharyngeal carcinoma, melanoma, skin cancer, laryngeal cancer, oral cancer, tongue cancer, gallbladder cancer, cholangiocarcinoma, skin cancer, testicular cancer, uterine cancer, endometrial cancer, renal pelvis cancer, renal cell carcinoma, bladder cancer, glioma, neuroblastoma, multiple myeloma, Hodgkin's lymphoma, non-Hodgkin's lymphoma, myeloid leukemia, myelodysplastic syndrome, soft tissue sarcoma, osteosarcoma, liposarcoma, neurofibrosarcoma, angiosarcoma, gastrointestinal stromal tumor, thymoma, thymic carcinoma, pituitary tumor, retinoblastoma, and/or glioblastoma.

In some embodiments, the tumor comprises a tumor with high CD20 expression. In some embodiments, the tumor comprises lymphoma. In some embodiments, the tumor comprises B-cell lymphoma.

In some embodiments, the tumor comprises a tumor with high claudin18.2 expression. In some embodiments, the tumor comprises gastric cancer, colon cancer, esophageal cancer, and/or pancreatic cancer.

Other aspects and advantages of the present application will be readily apparent to those skilled in the art from the detailed description below. The following detailed description merely shows and describes exemplary embodiments of the present application. As those skilled in the art will recognize, the content of the present application enables modifications to the disclosed specific embodiments without departing from the spirit and scope of the invention involved. Accordingly, the descriptions in the drawings and specification of the present application are exemplary only and not restrictive.

## BRIEF DESCRIPTION OF THE DRAWINGS

The specific features of the invention involved in the present application are shown in the appended claims. The

characteristics and advantages of the invention involved in the present application can be better understood by reference to the exemplary embodiments and drawings described in detail below. The drawings are briefly described as follows:

Figure 1 shows a schematic diagram of the cell preparation process of the present application.
Figures 2 to 7 show exemplary structures of non-natural sugars.
Figure 8 shows a flow cytometry statistical chart of γδ T cells incubated with 42 sugars for 48 h.
Figure 9 shows a flow cytometry statistical chart of γδ T cells incubated with 42 sugars followed by labeling with rituximab.
Figure 10 shows a flow cytometry statistical chart of γδ T cells incubated with 42 sugars followed by labeling with Hu7v3.
Figure 11 shows a killing assay statistical chart of γδ T cells incubated with 42 sugars followed by labeling with rituximab.
Figure 12 shows a killing assay statistical chart of γδ T cells incubated with 42 sugars followed by labeling with Hu7v3.
Figure 13 shows a flow cytometry statistical chart of T cells incubated with 42 sugars for 48 h.
Figure 14 shows a flow cytometry statistical chart of T cells incubated with 42 sugars followed by labeling with rituximab.
Figure 15 shows a flow cytometry statistical chart of T cells incubated with 42 sugars followed by labeling with Hu7v3.
Figure 16 shows a killing assay statistical chart of T cells incubated with 42 sugars followed by labeling with rituximab.
Figure 17 shows a killing assay statistical chart of T cells incubated with 42 sugars followed by labeling with Hu7v3.
Figure 18 shows a flow cytometry statistical chart of NK cells incubated with 42 sugars for 48 h.
Figure 19 shows a flow cytometry statistical chart of NK cells incubated with 42 sugars followed by labeling with rituximab.
Figure 20 shows a flow cytometry statistical chart of NK cells incubated with 42 sugars followed by labeling with Hu7v3.
Figure 21 shows a killing assay statistical chart of NK cells incubated with 42 sugars followed by labeling with rituximab.
Figure 22 shows a killing assay statistical chart of NK cells incubated with 42 sugars followed by labeling with Hu7v3.
Figure 23 shows bioluminescence imaging of mice after administration of γδ T cells linked with CD20 antibody via sugars No. 20, 22, and 25; PBS is the solvent control group, V2 is the control group without antibody labeling.
Figure 24 shows mouse survival rate after administration of γδ T cells linked with CD20 antibody via sugars No. 20, 22, and 25; PBS is the control group.
Figure 25 shows bioluminescence imaging of mice after administration of γδ T cells linked with CD20 antibody via sugars No. 3, 29, 23, 5, and 15; PBS is the solvent control group, V2 is the control group without antibody labeling.
Figure 26 shows mouse survival rate after administration of γδ T cells linked with CD20 antibody via sugars No. 3, 29, 23, 5, and 15; PBS is the control group.
Figure 27 shows bioluminescence imaging of mice after administration of γδ T cells linked with CD20 antibody via sugars No. 12, 8, 14, 41, and 42; PBS is the solvent control group, V2 is the control group without antibody labeling.
Figure 28 shows mouse survival rate after administration of γδ T cells linked with CD20 antibody via sugars No. 12, 8, 14, 41, and 42; PBS is the control group.
Figures 29 to 31 show mouse survival rates after administration of γδ T cells linked with Claudin18.2 antibody via sugars No. 29, 3, 22, 12, 5, 15, 20, 23, 25, 8, 41, 42, and 14; PBS is the solvent control group, γδ T represents the cell control group without antibody connection.

## DETAILED DESCRIPTION OF THE EMBODIMENTS

The following specific examples illustrate the implementation of the invention of the present application. Those skilled in the art can easily understand other advantages and effects of the present application from the content disclosed in this specification.

## DEFINITION OF TERMS

In the present application, the term "bioorthogonal reaction" generally refers to a set of reactions that can occur in biological environments with minimal impact on biomolecules or minimal interference with biochemical processes. Bioorthogonal reactions typically have one or more of the following characteristics: (1) can proceed under physiological temperature and pH, (2) selectively provide high yields of products, (3) are unaffected by water or endogenous nucleophiles, electrophiles, reducing agents, or oxidants in complex biological environments, (4) can form stable reaction products, (5) are rapid, and (6) can involve functional groups not naturally present in biological systems. Bioorthogonal reactions applicable to the method of the present application may include, such as azide-alkyne cycloaddition (SPAAC), reaction of nitrones with cyclooctynes, reaction of aldehydes or ketones to form oximes or hydrazones, Diels-Alder

reaction of tetrazines with cyclic alkenes or cyclic alkynes, norbornene cycloaddition, oxanorbornadiene cycloaddition, tetrazine coupling, and Staudinger ligation. The first or second functional group of the present application may be a group capable of participating in the above bioorthogonal reactions or a derivative thereof. The group obtained after the orthogonal reaction of the first or second functional group may be referred to as a "linker obtained through a bioorthogonal reaction", for example, an exemplary linker can be the structure after reaction of an azido group and DBCO:

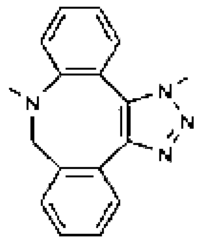

In the present application, the term "antigen-binding protein" generally refers to a protein comprising a portion that binds an antigen, and optionally a scaffold or framework portion that allows the antigen-binding portion to adopt a conformation that promotes binding of the antigen-binding protein to the antigen. Examples of antigen-binding proteins include, but are not limited to, antibodies, antigen-binding fragments (Fab, Fab', F(ab)2, Fv fragment, F(ab')2, VHH, scFv, di-scFv, and/or dAb), immunoconjugates, multispecific antibodies (e.g., bispecific antibodies), antibody fragments, antibody derivatives, antibody analogs or fusion proteins, etc., as long as they exhibit the desired antigen-binding activity.

In the present application, the term "antibody" generally refers to an immunoglobulin that can specifically bind a corresponding antigen. The antibody may be secreted by immune cells (e.g., effector B cells). The antibody may be a monoclonal antibody (including a full-length monoclonal antibody comprising two light chains and two heavy chains), polyclonal antibody, multispecific antibody (e.g., bispecific antibody), humanized antibody, fully human antibody, chimeric antibody, and/or camelized single domain antibody. "Antibody" generally may refer to a protein comprising at least two heavy chains (HC) and two light chains (LC) linked to each other by disulfide bonds, or antigen-binding fragments thereof. Each heavy chain comprises a heavy chain variable region (VH) and a heavy chain constant region. In certain naturally occurring IgG, IgD, and IgA antibodies, the heavy chain constant region comprises three domains: CH1, CH2, and CH3. In certain naturally occurring antibodies, each light chain comprises a light chain variable region (VL) and a light chain constant region. The light chain constant region comprises one domain, CL. The VH and VL regions can be further subdivided into hypervariable regions called complementarity determining regions (CDRs), interspersed with more conserved regions called framework regions (FRs). Each VH and VL comprises three CDRs and four framework regions (FRs), arranged from the amino terminus to the carboxyl terminus in the following order: FR1, CDR1, FR2, CDR2, FR3, CDR3, and FR4. The natural variable domains of heavy and light chains each comprise four FR regions (H-FR1, H-FR2, H-FR3, H-FR4; L-FR1, L-FR2, L-FR3, L-FR4), mostly adopting a β-sheet configuration, linked via three CDRs to form loop connection, and in some cases forming part of the β-sheet structure. The CDRs in each chain are brought into close proximity by the FR regions and, together with the CDRs from the other chain, form the antigen-binding site of the antibody.

In the present application, the term "γδ T cell" generally refers to a T cell whose T cell receptor (TCR) is composed of a γ chain and a δ chain. The TCR γ chain includes, but is not limited to, Vδ1, Vδ2, Vδ3, and Vδ5. The TCR δ chain includes, but is not limited to, Vγ2, Vγ3, Vγ4, Vγ5, Vγ8, and Vγ9. The term "γδ T cell" includes different γδ T cell types formed by the combination of different TCR δ chains and TCR γ chains. For example, γδ T cells expressing a TCR containing the δ chain variable region 1 (Vδ1) can be called Vδ1 T cells. Vδ1 T cells can be further classified into Vγ2Vδ1 T, Vγ3Vδ1 T, Vγ4Vδ1 T, Vγ5Vδ1 T, Vγ8Vδ1 T, and Vγ9Vδ1 T cells according to the type of γ chain variable region. For example, γδ T cells expressing a TCR containing the γ chain variable region 9 (Vγ9) and the δ chain variable region 2 (Vδ2) can be called Vγ9Vδ2 T cells. γδ T cells can be obtained from peripheral blood. The γδ T cell may be a mammalian γδ T cell, such as rodent γδ T cells, primate γδ T cells. In a specific embodiment, the γδ T cell is a human γδ T cell. In a specific embodiment, the γδ T cell is a mouse γδ T cell. In a specific embodiment, the γδ T cell is a monkey γδ T cell. The term also includes γδ T cells obtained from in vivo, as well as γδ T cells cultured and passaged in vitro or ex vivo. The γδ T cell may be an engineered γδ T cell, for example, a γδ T cell transfected with exogenous genes, a γδ T cell with endogenous gene knockout, or for example, CAR γδ T cells, TCR γδ T cells.

In the present application, the term "monosaccharide derivative" generally refers to a compound derived from a monosaccharide. In the present application, a monosaccharide derivative may refer to a compound where certain groups are substituted compared to a naturally occurring hydrolyzed monosaccharide, for example, a compound obtained after acylation. Monosaccharide derivatives can be hydrolyzed to yield natural sugars, such as glucose, galactose, mannose, fucose, or sialic acid. In particular, the monosaccharide derivative of the present application may be a product obtained by substitution of hydrogen atoms bonded to carbon atoms of a natural monosaccharide, and/or substitution of hydrogen atoms on hydroxyl groups. For example, the monosaccharide derivative of the present application may be an acetylated

product of a natural monosaccharide. The monosaccharide derivative of the present application may be hydrolyzed to yield N-acetylgalactosamine, N-acetylmannosamine, or sialic acid.

In the present application, the term "alkyl" generally includes saturated aliphatic groups, including straight-chain alkyl (e.g., methyl, ethyl, propyl, butyl, pentyl, hexyl, heptyl, octyl, nonyl, decyl, etc.), branched alkyl (e.g., isopropyl, tert-butyl, isobutyl, etc.). The term alkyl may further include alkyl where one or more carbon atoms in the backbone are replaced by oxygen, nitrogen, sulfur, or phosphorus atoms. The alkyl described in the present application may contain 1-20, 1-12, 1-10, 1-8, or 1-6 carbon atoms.

In the present application, the term "alkenyl" generally refers to any cyclic or acyclic, branched or unbranched unsaturated carbon chain moiety having one or more double bonds. In the present application, the term "alkynyl" generally refers to any cyclic or acyclic, branched or unbranched unsaturated carbon chain moiety having one or more triple bonds. The alkenyl or alkynyl described in the present application may contain 2-20, 2-12, 2-10, 2-8, or 2-6 carbon atoms.

In the present application, the term "alkynyl" generally refers to unsaturated straight-chain or branched alkynyl, such as ethynyl, 1-propynyl, propargyl, butynyl, etc. The alkynyl may be substituted or unsubstituted. The alkynyl described in the present application may contain 2-20, 2-12, 2-10, 2-8, or 2-6 carbon atoms.

In the present application, as known to those skilled in the art, terms such as "alkyl", "alkenyl", "cycloalkyl", etc., may be preceded by a designation indicating the number of atoms present in the group under specific circumstances, for example, $C_{1-6}$ alkyl, etc. The subscript number following "C" indicates the number of carbon atoms present in the group. For example, $C_3$ alkyl refers to an alkyl group having three carbon atoms (e.g., n-propyl, isopropyl). For example, the term "$C_{1-6}$ alkyl" includes alkyl groups containing 1 to 6 carbon atoms.

In the present application, the term "aryl" generally refers to a 3 to 12-membered substituted or unsubstituted monocyclic aromatic group, where all atoms of the ring may be carbon (i.e., carbocyclic aryl), or one or more atoms may be heteroatoms (i.e., heteroaryl).

The term "heteroatom" is well-known in the art and refers to an atom of any element other than carbon or hydrogen. Exemplary heteroatoms include boron, nitrogen, oxygen, phosphorus, sulfur, and selenium.

In the present application, the compounds of the present application include their tautomers, meso compounds, racemates, enantiomers, and/or diastereomers. In the present application, the term "diastereomer" generally refers to stereoisomers that have two or more chiral centers and whose molecules are not mirror images of each other. Diastereomers may have different physical properties, such as melting point, boiling point, spectral properties, and reactivity. In the present application, the term "meso compound" generally refers to a molecule containing atoms of asymmetry but having symmetry factors such that the overall rotation within the molecule is zero. The term "racemate" or "racemic mixture" refers to a composition comprising equimolar amounts of two enantiomeric substances.

In the present application, certain atoms of the compounds of the present application may exist in more than one isotopic form. For example, hydrogen may exist as protium ($^1H$), deuterium ($^2H$), and tritium ($^3H$); carbon may exist naturally in three different isotopes ($^{12}C$, $^{13}C$, and $^{14}C$). Examples of isotopes that can be incorporated into the compounds of the present application also include, but are not limited to, $^{15}N$, $^{18}O$, $^{17}O$, $^{18}F$, $^{32}P$, $^{33}P$, $^{129}I$, $^{131}I$, $^{123}I$, $^{124}I$, $^{125}I$, or similar isotopes. Unless otherwise indicated, the structures described in the present application also include compounds that differ only in the presence or absence of one or more isotopically enriched atoms. For example, compounds consistent with the structures of the present application except that hydrogen atoms are replaced by deuterium or tritium, or carbon atoms are replaced by $^{13}C$ or $^{14}C$, are within the scope of the present application.

In the present application, the term "immune cell" generally refers to a cell that can participate in or be related to an immune response. Immune cells may include all leukocytes and non-leukocyte antigen-presenting cells. The term includes individual cells, cell lines, or cell cultures. The cell includes not only a specific cell type but also progeny of these cells.

In the present application, the term "pharmaceutically acceptable adjuvant" generally includes pharmaceutically acceptable carriers, excipients, or stabilizers that are non-toxic to cells or mammals exposed to them at the dosages and concentrations employed. Typically, a physiologically acceptable carrier is a pH-buffered aqueous solution.

As used herein, the term "treatment" refers to administering internally or externally a therapeutic agent, for example, comprising any modified cell of the present application or a pharmaceutical composition comprising the modified cell, to a patient who has one or more disease symptoms, wherein the therapeutic agent is known to have a therapeutic effect on these symptoms. Typically, the patient is administered an amount of the therapeutic agent (a therapeutically effective amount) effective to alleviate one or more disease symptoms. Desired effects of treatment include slowing the rate of disease progression, improving or alleviating the disease state, and regression or improved prognosis. For example, an individual is successfully "treated" if one or more symptoms associated with cancer are alleviated or eliminated, including but not limited to, reducing (or destroying) cancer cell proliferation, reducing symptoms resulting from the disease, improving the quality of life of individuals suffering from the disease, reducing the dose of other drugs required to treat the disease, delaying disease progression, and/or prolonging the survival of the individual.

In the present application, the term "administration" generally refers to delivering a substance to a subject in need thereof by any route known in the art. Pharmaceutical carriers and formulations or compositions are also well known in the

art. Routes of administration may include intravenous, intramuscular, intradermal, subcutaneous, transdermal, mucosal, intratumoral, and/or mucosal.

In the present application, a disease "associated with abnormal expression" of a certain target generally means that the occurrence and/or progression of the disease is associated with the expression level of that target. For example, "high expression" of a target means that the expression level of a target in cells from a disease area, such as within a specific tissue or organ of a patient, is increased relative to the expression level in normal cells from the tissue or organ, i.e., it is highly expressed. Or for example, "low expression" of a target means that the expression level of a target in cells from a disease area, such as within a specific tissue or organ of a patient, is decreased relative to the expression level in normal cells from the tissue or organ, i.e., it is lowly expressed. Or for example, cells from a disease area, such as within a specific tissue or organ of a patient, express a target, i.e., they are positive. Or for example, cells from a disease area, such as within a specific tissue or organ of a patient, do not express a target, i.e., they are negative. For example, the characteristics of target expression can be determined by standard assays known in the art.

In the present application, the term "comprising" generally means including, encompassing, containing, or incorporating. In some contexts, it may also mean "being", "consisting of".

In the present application, the term "about" generally means varying within a range of 0.5% to 10% above or below the specified value, for example, within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

## DETAILED DESCRIPTION OF THE INVENTION

### Method for Modifying Cells

In one aspect, the present application provides a method for modifying cells, the method comprising one or more of the following steps:

1. Contacting a monosaccharide derivative containing a first functional group with an immune cell to obtain an immune cell expressing on its surface the monosaccharide derivative containing the first functional group;

2. Contacting an antigen-binding protein with a compound containing a second functional group to obtain the antigen-binding protein containing the second functional group; and/or,

3. Contacting the immune cell expressing on its surface the monosaccharide derivative containing the first functional group with the antigen-binding protein containing the second functional group, such that the first functional group and the second functional group undergo a conjugation reaction to obtain a cell modified on its surface with the antigen-binding protein.

### Step S1

In the present application, the method may comprise step S1: contacting a monosaccharide derivative containing a first functional group with an immune cell to obtain an immune cell expressing on its surface the monosaccharide derivative containing the first functional group. The monosaccharide derivative may be derived from mannose, galactose, fucose, xylose, glucose, mannosamine, galactosamine, glucosamine, and/or sialic acid. The first functional group may be linked to the monosaccharide molecule via a suitable spacer, for example, an amide bond or alkylamino bond. The first functional group is linked to the monosaccharide molecule at the C1, C2, C3, C4, or C5 position, and optionally may be further acylated on one or more remaining C atoms. In one embodiment, the monosaccharide molecule may contain hydrolyzable substituents on one or more of the C1, C2, C3, C4, or C5 positions not containing the first functional group; hydrolyzable substituents help with the hydrophobicity of the polymer and, once inside the cell, can be hydrolyzed and converted to hydroxyl groups. An example of a hydrolyzable substituent is

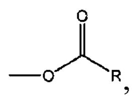

where R is alkyl, for example, R is methyl.

For example, the monosaccharide derivative may be selected from the group consisting of

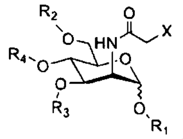
(Forumula I)

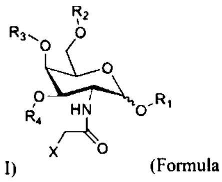
(Formula II)

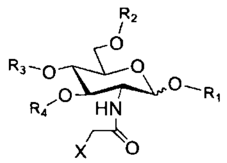
(Formula III)

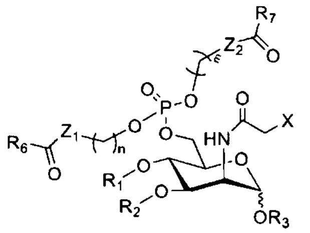
(Formula IV)

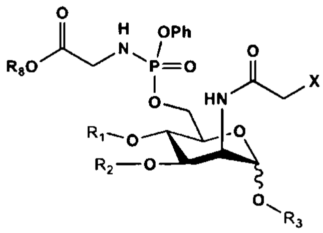
(Formula V)

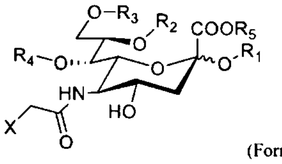
(Formula VI)

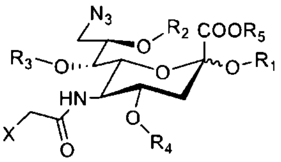
(Formula VII)

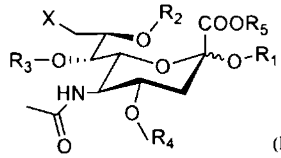
(Formula VIII)

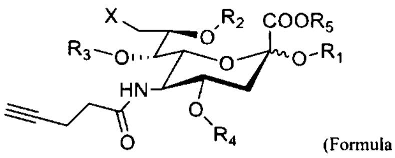
(Formula IX)

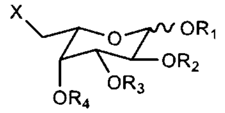
(Formula X)

wherein, X is the first functional group, $R_1$, $R_2$, $R_3$, $R_4$, $R_5$, and $R_6$ are each independently selected from protecting groups that can be hydrolyzed or enzymatically cleaved within cells, with the purpose of facilitating entry of the non-natural sugar into the cell and exposing the hydroxyl groups on the sugar after deprotection. Therefore, any group fulfilling this function falls within the scope of the present invention.

In some embodiments, $R_1$, $R_2$, $R_3$, and $R_4$ are each independently selected from the group consisting of hydrogen, halogen, substituted or unsubstituted $C_{1-6}$ alkyl, $C_{1-6}$ alkylthio, $C_{1-6}$ alkylcarbonyl, $C_{2-6}$ alkenyl, $C_{2-6}$ alkynyl, $C_{2-6}$ alkoxy, $C_{2-6}$ alkoxycarbonyl, and acyl, wherein said group is optionally substituted with a substituent selected from halogen, hydroxyl, amino, nitro, mercapto, and $C_{1-6}$ alkyl;

$R_5$ is selected from the group consisting of hydrogen, halogen, and substituted or unsubstituted $C_{1-6}$ alkyl, wherein said alkyl is optionally substituted with a substituent selected from halogen, hydroxyl, amino, nitro, and mercapto;
$R_6$ and $R_7$ are each independently selected from the group consisting of halogen and substituted or unsubstituted $C_{1-10}$ alkyl, wherein said alkyl is optionally substituted with a substituent selected from halogen, hydroxyl, amino, nitro, and mercapto;
$R_8$ is selected from the group consisting of halogen, substituted or unsubstituted $C_{1-10}$ alkyl, phenyl, and substituted or unsubstituted $C_{1-10}$ alkylene-phenyl, wherein said alkyl is optionally substituted with a substituent selected from halogen, hydroxyl, amino, nitro, and mercapto;
$Z_1$ and $Z_2$ are each independently selected from O and S;
n and m are each independently selected from the integers 1, 2, 3, 4, 5, and 6.

In some embodiments, $R_1$ is selected from the group consisting of hydrogen and -C(=O)-$C_{1-6}$ alkyl. For example, $R_1$ is hydrogen or -C(=O)-$CH_3$.

In some embodiments, $R_2$ is selected from the group consisting of hydrogen and -C(=O)-$C_{1-6}$ alkyl. For example, $R_2$ is hydrogen or -C(=O)-$CH_3$.

In some embodiments, $R_3$ is selected from the group consisting of hydrogen and -C(=O)-$C_{1-6}$ alkyl. For example, $R_3$ is hydrogen or -C(=O)-$CH_3$.

In some embodiments, $R_4$ is selected from the group consisting of hydrogen and -C(=O)-$C_{1-6}$ alkyl. For example, $R_4$ is hydrogen or -C(=O)-$CH_3$.

In some embodiments, $R_5$ is selected from the group consisting of hydrogen and substituted or unsubstituted $C_{1-6}$ alkyl. In some embodiments, $R_5$ is hydrogen or substituted or unsubstituted $C_{1-3}$ alkyl. For example, $R_4$ is hydrogen or -$CH_3$.

In some embodiments, $R_6$ is selected from the group consisting of hydrogen and substituted or unsubstituted $C_{1-10}$ alkyl.

In some embodiments, $R_7$ is selected from the group consisting of hydrogen and substituted or unsubstituted $C_{1-10}$ alkyl.

In some embodiments, $R_8$ is selected from the group consisting of methyl, ethyl, and -$CH_2$-phenyl.

In some embodiments, m is 1, 2, or 3.

In some embodiments, n is 1, 2, or 3.

In some embodiments, in Formula I, $R_1$ is selected from hydrogen and -C(=O)-$C_{1-6}$ alkyl, $R_2$ is selected from hydrogen and -C(=O)-$C_{1-6}$ alkyl, $R_3$ is selected from hydrogen and -C(=O)-$C_{1-6}$ alkyl, $R_4$ is selected from hydrogen and -C(=O)-$C_{1-6}$ alkyl, and $R_1$, $R_2$, $R_3$, and $R_4$ are not all hydrogen simultaneously.

In some embodiments, in Formula II, $R_1$ is selected from hydrogen and -C(=O)-$C_{1-6}$ alkyl, $R_2$ is selected from hydrogen and -C(=O)-$C_{1-6}$ alkyl, $R_3$ is selected from hydrogen and -C(=O)-$C_{1-6}$ alkyl, $R_4$ is selected from hydrogen and -C(=O)-$C_{1-6}$ alkyl, and $R_1$, $R_2$, $R_3$, and $R_4$ are not all hydrogen simultaneously.

In some embodiments, in Formula III, $R_1$ is selected from hydrogen and -C(=O)-$C_{1-6}$ alkyl, $R_2$ is selected from hydrogen and -C(=O)-$C_{1-6}$ alkyl, $R_3$ is selected from hydrogen and -C(=O)-$C_{1-6}$ alkyl, $R_4$ is selected from hydrogen and -C(=O)-$C_{1-6}$ alkyl, and $R_1$, $R_2$, $R_3$, and $R_4$ are not all hydrogen simultaneously.

In some embodiments, in Formula IV, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_6$ is selected from $C_{1-10}$ alkyl, $R_7$ is selected from $C_{1-10}$ alkyl, $Z_1$ is selected from O and S, $Z_2$ is selected from O and S, n is 1, 2, or 3, m is 1, 2, or 3, and $R_1$, $R_2$, and $R_3$ are not all hydrogen simultaneously.

In some embodiments, in Formula V, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_8$ is selected from $C_{1-3}$ alkyl, phenyl, $C_{1-3}$ alkylene-phenyl, and $R_1$, $R_2$, and $R_3$ are not all hydrogen simultaneously.

In some embodiments, in Formula VI, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_4$ is selected from hydrogen and -C(=O)-$C_{1-6}$ alkyl (preferably -C(=O)-$CH_3$), $R_5$ is selected from hydrogen and $C_{1-6}$ alkyl (preferably -$CH_3$), and $R_1$, $R_2$, $R_3$, and $R_4$ are not all hydrogen simultaneously, or $R_1$, $R_2$, $R_3$, $R_4$, $R_5$ are not all hydrogen simultaneously. In a specific embodiment, at most four of $R_1$, $R_2$, $R_3$, $R_4$, $R_5$ are hydrogen.

In some embodiments, in Formula VII, $R_1$ is selected from hydrogen and -C(=O)-$C_{1-6}$ alkyl (preferably -C(=O)-$CH_3$), R2 is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_4$ is selected from hydrogen and -C(=O)-$C_{1-6}$ alkyl (preferably -C(=O)-$CH_3$), $R_5$ is selected from hydrogen and $C_{1-6}$ alkyl (preferably -$CH_3$), and $R_1$, $R_2$, $R_3$, and $R_4$ are not all hydrogen simultaneously, or $R_1$, $R_2$, $R_3$, $R_4$, $R_5$ are not all hydrogen simultaneously. In a specific embodiment, at most four of $R_1$, $R_2$, $R_3$, $R_4$, $R_5$ are hydrogen.

In some embodiments, in Formula VIII, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably - C(=O)-$CH_3$), $R_3$ is selected from hydrogen and -C(=O)-$C_{1-6}$ alkyl (preferably -C(=O)-$CH_3$), $R_4$ is selected from hydrogen and -C(=O)-$C_{1-6}$ alkyl (preferably -C(=O)-$CH_3$), $R_5$ is selected from hydrogen and $C_{1-6}$ alkyl (preferably -$CH_3$), and $R_1$, $R_2$, $R_3$, and $R_4$ are not all hydrogen simultaneously, or $R_1$, $R_2$, $R_3$, $R_4$, $R_5$ are not all hydrogen simultaneously. In a specific embodiment, at most four of $R_1$, $R_2$, $R_3$, $R_4$, $R_5$ are hydrogen.

In some embodiments, in Formula VI, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably-C(=O)-$CH_3$), $R_3$ is selected from hydrogen and -C(=O)-$C_{1-6}$ alkyl (preferably -C(=O)-$CH_3$), $R_4$ is selected from hydrogen and -C(=O)-$C_{1-6}$ alkyl (preferably -C(=O)-$CH^3$), $R_5$ is selected from hydrogen and $C_{1-6}$ alkyl (preferably -$CH_3$), and $R_1$, $R_2$, $R_3$, and $R_4$ are not all hydrogen

simultaneously, or $R_1$, $R_2$, $R_3$, $R_4$, $R_5$ are not all hydrogen simultaneously. In a specific embodiment, at most four of $R_1$, $R_2$, $R_3$, $R_4$, $R_5$ are hydrogen.

In some embodiments, in Formula V, $R_1$ is selected from hydrogen and -C(=O)-$C_{1-6}$ alkyl (preferably -C(=O)-$CH_3$), $R_2$ is selected from hydrogen and -C(=O)-$C_{1-6}$ alkyl (preferably-C(=O)-$CH_3$), $R_3$ is selected from hydrogen and -C(=O)-$C_{1-6}$ alkyl (preferably -C(=O)-$CH_3$), and $R_4$ is selected from hydrogen and -C(=O)-$C_{1-6}$ alkyl (preferably -C(=O)-$CH_3$). In a specific embodiment, $R_1$, $R_2$, $R_3$, and $R_4$ are all hydrogen. In a specific embodiment, $R_1$, $R_2$, $R_3$, and $R_4$ are all -C(=O)-C1-6 alkyl (preferably -C(=O)-$CH_3$).

In one embodiment, the monosaccharide derivative may comprise any one or more structures selected from the group consisting of

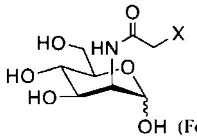
(Formula I-a-1)
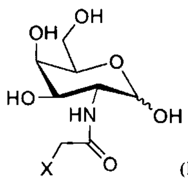
(Formula II-a-1)
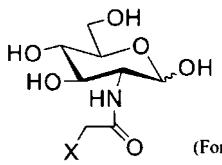
(Formula II-b-)

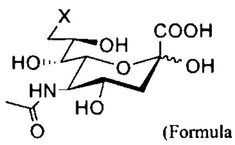
(Formula III-1)
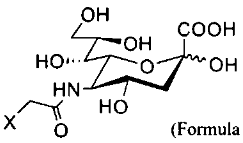
(Formula IV-1)
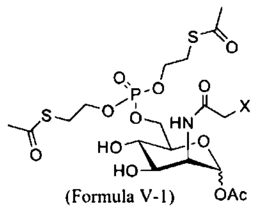
(Formula V-1)

wherein X is the first functional group.

In one embodiment, the monosaccharide derivative may be selected from any structure shown in Table A above.

The monosaccharide derivative may also comprise its tautomer, meso compound, racemate, enantiomer, diastereomer, or mixtures thereof, or pharmaceutically acceptable salts, prodrugs, or solvates thereof.

In the present application, the first functional group may be a group having bioorthogonal reactivity, and the bioorthogonal reaction may comprise at least one selected from the group consisting of click chemistry, photo-cross-linking, photosensitive, and glycosylation.

In the present application, the first functional group may be selected from the group consisting of azido, alkynyl, trans-cyclooctene, triazine, tetrazine, acyl hydrazone, cyclopropene, isonitrile, and cyclooctyne. In the present application, the first functional group may be selected from the group consisting of azido, dibenzocyclooctyne (DBCO), tetrazine, trans-cyclooctene, tetrazine, and norbornene, examples of which are shown below:

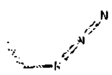
Azide
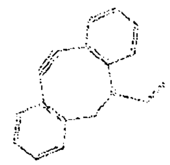
Dibenzocyclooctyne

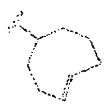
Transcyclooctene
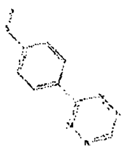
Tetrazine
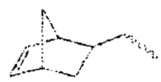
Norbornene

In the present application, the monosaccharide derivative containing the first functional group can be expressed on the cell membrane surface via the metabolic glycan pathway, embedding the first functional group into the cell membrane surface, thereby obtaining a cell modified with the first functional group. Besides sugars, the first functional group may also be conjugated to lipids (choline, cholesterol, or fatty acids) or amino acids (e.g., methionine, alanine, or phenylalanine) on the cell membrane surface.

In the present application, the contact time between the monosaccharide derivative containing the first functional group and the immune cell is about 2 h to 48 h, for example, about 16 h to 32 h, for example, about 24 h. In the present

application, the contact between the monosaccharide derivative containing the first functional group and the immune cell is carried out at room temperature or at 37°C.

In the present application, the immune cell may be selected from one or more of the group consisting of NK cells, DC cells, NKT cells, monocytes, macrophages, T cells, and/or B cells. In one embodiment, the immune cell is an NK cell. In another embodiment, the immune cell is a T cell. In a more specific embodiment, the immune cell is a γδ T cell.

The method may comprise contacting the cell with an effective amount of the monosaccharide derivative containing the first functional group. An effective amount may refer to the monosaccharide derivative containing the first functional group constituting at least 10% of the cell surface glycoproteins. For example, among cell surface glycoproteins, more than 10% of the glycoproteins are the monosaccharide derivative containing the first functional group, preferably more than 20%, more than 30%, more than 40%, more than 50%, more than 60%, more than 70%, more than 80%, more than 90% or higher. For each monosaccharide derivative containing the first functional group and each cell type, a person skilled in the art can readily determine the amount required for metabolic labeling of the cells.

In the present application, the purity of the immune cells may be assessed, then they are co-cultured with the monosaccharide derivative containing the first functional group for 24 h; thereby obtaining immune cells expressing the first functional group on their surface.

In the present application, the immune cells expressing the first functional group on their surface may be validated. For example, by co-incubating the immune cells with biotin or a fluorescent protein conjugated with a functional group capable of undergoing a bioorthogonal reaction with the first functional group, then detecting the fluorescent signal, thereby characterizing the labeling degree of the first functional group on the immune cell surface.

**Step S2**

In the present application, the method may comprise step S2: contacting an antigen-binding protein with a compound containing a second functional group to obtain the antigen-binding protein containing the second functional group.

In the present application, the antigen-binding protein may target a tumor antigen. The tumor antigen may be a tumor-specific antigen or a tumor-associated antigen. The tumor antigen may be an overexpressed or abnormally expressed cellular protein, a product of mutated oncogenes and tumor suppressor genes, products of other mutated genes, tumor antigens produced by oncogenic viruses, oncofetal antigens, cell surface glycolipids and glycoproteins, or cell type-specific differentiation antigens. In the present application, the antigen-binding protein may target a protein on the surface of B lymphocytes, for example, CD20. In the present application, the antigen-binding protein may target a claudin protein, for example claudin18.2. In the present application, the antigen-binding protein may be an anti-CD20 antibody. In the present application, the antigen-binding protein may be an anti-claudin18.2 antibody. For example, the antigen-binding protein of the present application may comprise a heavy chain variable region and/or a light chain variable region. For example, the antigen-binding protein of the present application may comprise a heavy chain and/or a light chain.

Antibodies of the present application can be prepared using techniques well known in the field, such as the hybridoma method, recombinant DNA technology, phage display technology, synthetic technology, or combinations thereof, or other techniques known in the field. Variants may refer to amino acid sequence mutants of the antibody, as well as covalent derivatives of the natural polypeptide, provided that biological activity comparable to the natural polypeptide is retained. Amino acid sequence mutants differ from the natural amino acid sequence generally by substitution of one or more amino acids in the natural amino acid sequence, and/or deletion and/or insertion of one or more amino acids in the polypeptide sequence. Deletion mutants include fragments of the natural polypeptide and N-terminal and/or C-terminal truncated mutants. Typically, amino acid sequence mutants have at least 70%, 75%, 80%, 85%, 90%, 95%, 98%, or 99% or more homology compared to the natural sequence.

In the present application, the second functional group may be a group capable of undergoing a bioorthogonal reaction with the first functional group. In the present application, the second functional group may be selected from the group consisting of azido, alkynyl, trans-cyclooctene, triazine, tetrazine, acyl hydrazone, cyclopropene, isonitrile, and cyclooctyne. In the present application, the second functional group may be selected from the group consisting of azido, dibenzocyclooctyne (DBCO), bicyclononyne (BCN), tetrazine, trans-cyclooctene (TCO), and norbornene, examples of which are shown below:

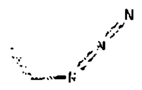

Azide

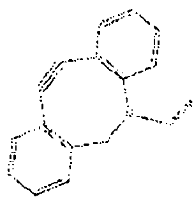

Dibenzocyclooctyne

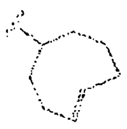

Transcyclooctene

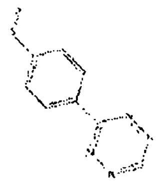

Tetrazine

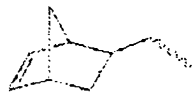

Norbornene

In one embodiment, one of the first and second functional groups may be azido, and the other may be dibenzocyclooctyne (DBCO). In one embodiment, one of the first and second functional groups may be tetrazine, and the other may be trans-cyclooctene (TCO). In one embodiment, one of the first and second functional groups may be tetrazine, and the other may be norbornene. In one embodiment, one of the first and second functional groups may be BCN, and the other may be azido.

The second functional group may be linked to the antigen-binding protein via a linker, such as a disulfide bond, hydrazone, bond, or enzyme-cleavable bond. In the present application, the compound containing the second functional group may comprise a group capable of reacting with an amino acid, and the second functional group is linked via said group to an amino acid of the antigen-binding protein. For example, the second functional group may be linked to the antigen-binding protein via maleimide or succinimide. In one embodiment, the structure of the compound containing the second functional group may be Formula L1, Formula L2, or Formula L3:

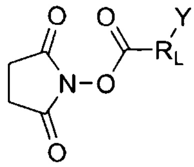

(Formula L1)

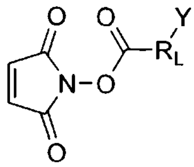

(Formula L2)

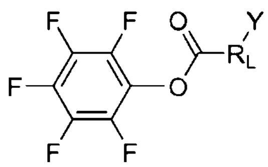

(Formula L3)

wherein, Y represents the second functional group, $R_L$ is selected from the group consisting of polyethylene glycol, amide group, -C(O)-, and/or alkyl, and combinations thereof. For example, $R_L$ may be selected from the group consisting of polyethylene glycol, amide group, and alkyl ether group. For another example, $R_L$ may be selected from $C_{1-20}$ alkylene or $C_{1-20}$ alkylene ether group.

In one embodiment, when the first functional group is azido, the second functional group may be DBCO, and optionally the compound containing the second functional group is

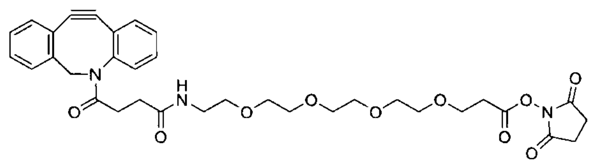

DBCO-PEG4-NHS

or

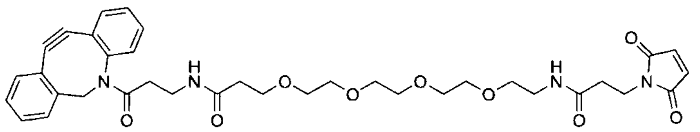

DBCO-PEG4-MAL .

The concentration ratio of the antigen-binding protein to the second functional group in the method of the present application may be from 1:1 to 1:10, for example, it may be 1:5. In the present application, the contact time between the antigen-binding protein and the compound containing the second functional group may be about 30 min to 6 h, for example, about 1 h to 2 h, for example, about 1 h. In the present application, the contact between the antigen-binding protein and the compound containing the second functional group may be carried out at room temperature.

**Step S**

The method of the present application comprises step S: contacting the immune cell expressing on its surface the monosaccharide derivative containing the first functional group with the antigen-binding protein containing the second functional group, such that the first functional group and the second functional group undergo a conjugation reaction, to obtain a cell modified on its surface with the antigen-binding protein. The pairing selection of the first functional group and the second functional group can be freely chosen by a person skilled in the art, including but not limited to those combinations listed above. In a specific example, the first functional group may be azido, and the second functional group may be DBCO.

The contact may be performed under conditions allowing the bioorthogonal reaction to occur, for example, under mild conditions, including neutral pH, aqueous solution and ambient temperature, and low reactant concentrations. In some embodiments, the contact results in the antigen-binding protein being modified on the cell surface while retaining the structural integrity, function, or activity of the antigen-binding protein. In other embodiments, the antigen-binding protein retains part of its structure or undergoes some structural changes but retains part of its function or activity.

A contact time sufficient for the first functional group and the second functional group to undergo the conjugation reaction, thereby modifying the antigen-binding protein on the cell surface, may be about 5 minutes, about 10 minutes, about 20 minutes, about 30 minutes, about 40 minutes, about 50 minutes, about 60 minutes, or more than about 60 minutes. In one example, a suitable contact time is about 30 minutes.

In the present application, the contact in step S may be carried out at room temperature. In the present application, the contact in step S may be carried out at 37°C.

Depending on the type of antigen-binding protein and cells, a person skilled in the art can readily determine the contact ratio between the immune cell containing the monosaccharide derivative with the first functional group and the antigen-binding protein containing the second functional group. In some embodiments, the concentration of the antigen-binding protein containing the second functional group may be about 10 $\mu$M to about 30 $\mu$M, and the density of the immune cell containing the monosaccharide derivative with the first functional group does not exceed $2\times10^7$ cells/mL. In some embodiments, the concentration of the antigen-binding protein containing the second functional group may be about 15 $\mu$M to about 25 $\mu$M, and the density of the immune cell containing the monosaccharide derivative with the first functional group does not exceed $1.5\times10^7$ cells/mL. In a specific example, the concentration of the antigen-binding protein containing the second functional group may be about 20 $\mu$M, and the density of the immune cell containing the monosaccharide derivative with the first functional group does not exceed $1\times10^7$ cells/mL.

In the present application, the cell modified on its surface with the antigen-binding protein may be validated. For example, by co-incubating the modified immune cells with a corresponding antigen bearing a detectable label, then detecting the signal intensity, thereby characterizing the degree of antigen-binding protein modification on the immune cell surface. The detectable label may be biotin, detected via AF488-conjugated streptavidin, characterizing the labeling situation of the antigen-binding protein on the cell surface, for example, by laser confocal imaging. In the present application, labeling of the antigen-binding protein may be detected on at least 10% (e.g., at least 20%, at least 30%, at least 40%, at least 50%, at least 60%, at least 70%, at least 80%, at least 90% or higher) of the cell surfaces.

**Modified Cells**

In another aspect, the present application provides a modified cell, prepared by the method described in the present application. In a specific example, the modified cell is an antibody-cell conjugate. The cell is conjugated on its surface with an antibody via a monosaccharide derivative through the method of the present application.

In another aspect, the present application provides a modified cell, which is modified on its cell surface with an antigen-binding protein, for example, modified via a bioorthogonal reaction onto non-natural sugar analogs expressed on

the cell surface, where the non-natural sugar analogs can be expressed on the cell surface via the metabolic glycan pathway. The cell may be an epithelial cell, fibroblast, neuronal cell, endothelial cell, or immune cell. In some embodiments, the cell is an immune cell. Examples of immune cells include, but are not limited to, peripheral blood leukocytes, spleen leukocytes, lymph node leukocytes, hybridoma cells, T cells (cytotoxic/suppressor cells, helper cells, memory cells, naive cells, and sensitized cells), B cells (memory cells and naive cells), monocytes, macrophages, granulocytes (basophils, eosinophils, and neutrophils), natural killer cells, natural suppressor cells, thymocytes, and dendritic cells.

The cell surface has azido groups expressed on the surface via the metabolic glycan pathway by uptake of non-natural sugar derivatives containing an azido group. The azido groups are connected to an antibody via a linking compound, one end of which has a first reactive group capable of reacting with a thiol or amino group, and the other end has a second reactive group capable of undergoing a bioorthogonal reaction with the azido group, wherein the first reactive group reacts and connects with a thiol or amino group on said antibody, and the second group reacts and connects with said azido group.

In one example, the cell is a T cell. Examples of T cells include, but are not limited to, CD4+ T cells, CD8+ T cells, Treg cells, memory T cells, natural killer T cells, mucosal-associated invariant T cells (MAIT), $\alpha\beta$-T cells, $\gamma\delta$ T cells, as well as engineered versions of the above cells, such as CAR T cells, TCR T cells.

In one example, the cell is a $\gamma\delta$ T cell. For example, the cell is a V$\gamma$9V$\delta$2 T cell. For another example, the cell is a V$\delta$1 T cell.

In one example, the cell is a CAR T cell.

In one example, the cell is an NK cell.

Immune cells modified by the method of the present application have enhanced targeting functionality. For example, when being modified with an antibody targeting a specific tumor antigen, the modified cells can efficiently target cells (e.g., tumor cells) expressing that tumor antigen. Compared to cells not modified with the antibody, the cells modified on their surface with the antibody may have enhanced ability to target the tumor antigen by at least 10%, or at least 20%, at least 30%, at least 40%, at least 50%, at least 60%, at least 70%, at least 80%, at least 90%, at least 100% or higher. The targeting ability can be detected by flow cytometry, laser confocal imaging, or other methods known in the art. The targeting ability can be detected by in vivo enrichment experiments; cells modified on their surface with the antibody are more likely to enrich within tumors expressing that antigen. Immune cells modified by the method described in the present application can reduce off-target effects and exhibit safety.

Immune cells modified by the method of the present application have enhanced cell-killing functionality. For example, when modified with an antibody targeting a specific tumor antigen, the modified cells can efficiently kill cells (e.g., tumor cells) expressing that tumor antigen. Compared to cells not modified with the antibody, the cells modified on their surface with the antibody may have enhanced ability to kill tumor cells expressing the corresponding antigen by at least 10%, or at least 20%, at least 30%, at least 40%, at least 50%, at least 60%, at least 70%, at least 80%, at least 90%, at least 100% or higher. The killing ability can be detected by in vitro or in vivo methods. For example, it can be detected using an LDH detection kit. For example, it can be detected in vivo using animal models.

## Pharmaceutical Compositions and Treatment Methods

In another aspect, the present application provides a method for treating and/or alleviating tumors, comprising administering to a subject in need thereof a modified cell prepared according to the method of the present application. A suitable antigen-binding protein can be selected according to the type of tumor to be treated and/or alleviated, and then modified cells targeting a specific tumor antigen are prepared according to the method of the present application.

For example, the tumor may be a B lymphocyte tumor. For example, the tumor may be a tumor with high CD20 expression. For example, the tumor may be a CD20-positive tumor. For example, CD20-positive tumors may include B-cell lymphoma or leukemia, including but not limited to: diffuse large B-cell lymphoma (DLBCL), follicular lymphoma (FL), marginal zone B-cell lymphoma (MZL), mantle cell lymphoma (MCL), chronic lymphocytic leukemia/small lymphocytic lymphoma (CLL/SLL), lymphoplasmacytic lymphoma (LPL), plasmablastic lymphoma (PCL), primary central nervous system lymphoma (PCNSL).

For another example, the tumor may be a tumor with high claudin18.2 expression. For example, the tumor may be a claudin18.2-positive tumor. For example, claudin18.2-positive tumors may include, but are not limited to, gastric cancer, pancreatic cancer, esophageal cancer, ovarian cancer, cholangiocarcinoma, liver cancer, breast cancer, and/or lung cancer.

The tumor may be a primary tumor or a metastatic tumor.

The modified cells of the present application may be administered alone or as a pharmaceutical composition together with a pharmaceutically acceptable adjuvant, for example, administered in combination with one or more pharmaceutically or physiologically acceptable carriers, diluents, or excipients. Such compositions may include buffers such as neutral buffered saline, phosphate buffered saline, etc.; carbohydrates such as glucose, mannose, sucrose or dextran, mannitol; proteins; polypeptides or amino acids such as glycine; antioxidants; chelating agents such as EDTA or

glutathione; adjuvants (e.g., aluminum hydroxide); and preservatives.

The cells or pharmaceutical compositions of the present application may be administered subcutaneously, intradermally, intratumorally, intranodally, intramedullarly, intramuscularly, intravenously (i.v.) injection, or intraperitoneally to a patient. Preferably, the pharmaceutical composition of the present application may be formulated for intravenous administration.

The modified cells of the present application may be co-administered with one or more other anti-tumor therapies (e.g., surgery, chemotherapy, radiotherapy, targeted therapy, or immune checkpoint inhibitors).

The dosage and frequency of administration of the cells of the present application will be determined by practical factors, such as the patient's condition, the type and severity of the patient's disease, and taking into account the patient's age, weight, tumor size, extent of infection or metastasis, and individual differences in the condition.

Without intending to be bound by any theory, the following examples are merely to illustrate the fusion proteins, preparation methods, and uses, etc., of the present application, and are not intended to limit the scope of the invention of the present application.

## EXAMPLES

### Example 1: Preparation of Compounds with Orthogonal Reactive Groups

Compounds with orthogonal reactive groups were prepared according to reference 1 (Cheng, B., Wang, C. T., Hao, Y., et al. (2023) Facile Synthesis of Clickable Non-natural sugars in the Unprotected and 1,6-Di-O-Acylated Forms for Metabolic Glycan Labeling. Chem-Eur J 29 (11), e202203054), reference 2 (Han, S. F., Collins, B. E., Bengtson, P., Paulson, J. C. (2005) Homomultimeric complexes of CD22 in B cells revealed by protein-glycan cross-linking. Nat. Chem. Biol. 1 (2), 93-7), reference 3 (Luchansky, S. J., Goon, S., Bertozzi, C. R. (2004) Expanding the diversity of unnatural cell-surface sialic acids. ChemBioChem 5 (3), 371-4.), and reference 4 (Cheng, B., Dong, L., Zhu, Y. T., et al. (2019) 9-Azido Analogues of Three Sialic Acid Forms for Metabolic Remodeling of Cell-Surface Sialoglycans. ACS Chem. Biol. 14 (10), 2141-2147.), yielding the monosaccharide derivatives of the present application, taking compound a

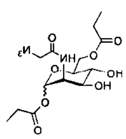

and compound b

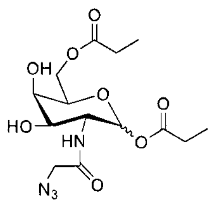

as an example. The monosaccharide derivatives of the present application can also be obtained commercially, taking $Ac_4$ManNAz (Sigma-Aldrich, Cat. No. 900917) and $Ac_4$GalNAz (Sigma-Aldrich, Cat. No. 900915) as examples. A total of 42 monosaccharide derivatives with orthogonal reactive groups were obtained, with structures as shown in Figures 2-7, where Figure 2 shows 5 GalNAz derivatives, Figure 3 shows 5 GlcNAz derivatives, Figure 4 shows 5 ManNAz derivatives, Figure 5 shows 13 ManNAz-6-P derivatives, Figure 6 shows 12 azido sialic acid derivatives, and Figure 7 shows 2 azido fucose derivatives.

To observe the labeling efficiency of the 42 non-natural sugars, γδ T cells, T cells, and NK cells were labeled with the corresponding concentration for each non-natural sugar. After 48 h of labeling, the labeling efficiency of the non-natural sugar on each group of cells was detected by flow cytometry, with results expressed as Mean Fluorescence Intensity (MFI). Then, the cells were labeled with rituximab and Hu7v3 antibodies that had been pre-labeled with DBCO. After 1 h of antibody labeling, cells were taken out, and the antibody labeling efficiency was detected by flow cytometry. Then, killing assays were performed against Raji and SNU-620 cell lines, respectively, to observe differences between cells labeled with different non-natural sugars. Specific examples are as follows.

### Example 2: DBCO Labeling of Antibodies

(1) Antibody desalting: An appropriate amount of the two antibodies to be labeled with DBCO (rituximab targeting CD20 and Hu7v3 antibody targeting Claudin18.2) were taken out for desalting. Four desalting columns were taken per group, washed 5 times with 1× PBS. Then, 2.5 mL of antibody was added to each column. After the antibody flowed through, 0.5 mL of 1× PBS was added. After the PBS flowed through, the desalting columns were removed from the rack, wiped dry with paper towel, placed above a 15 mL centrifuge tube, and 3 mL of 1× PBS was added to each column to collect the antibody. After antibody desalting, the desalting columns were washed 5 times with 1× PBS, washed 5 times with pure water, and stored at 4°C.

(2) DBCO labeling of antibodies: The concentration of the collected antibodies was determined using a UV spectrophotometer at A280, divided by the extinction coefficient to calculate the molar concentration of the two antibodies, and 5 equivalents of DBCO were added accordingly. Labeling was performed at 30°C for 1 h.

(3) Desalting after DBCO labeling: The antibodies after DBCO labeling were desalted again following the same steps as antibody desalting, changing the storage medium to PBS. The concentration of the DBCO-labeled antibodies was determined after desalting.

### Example 3: ACC Preparation

The non-natural sugars with azido groups prepared in Example 1 were added to the culture medium of γδ T cells, T cells, or NK cells, and culture was continued for 48 h.

Specifically, cells cultured to day 11 were labeled with the corresponding concentrations of 42 non-natural sugars, a total of 42 experimental groups. The control group received no non-natural sugar.

Table 1 Labeling Concentrations of Non-natural sugars

| No. | Formula | Labeling Concentration |
|---|---|---|
| 1 | ManNAz | 1 mM |
| 2 | 1,6-$Ac_2$ManNAz | 200 μM |
| 3 | 1,6-$Pr_2$ManNAz | 50 μM |
| 4 | 1,6-$Bu_2$ManNAz | 50 μM |
| 5 | $Ac_4$ManNAz | 50 μM |
| 6 | GlcNAz | 2 mM |
| 7 | 1,6-$Ac_2$GalNAz | 500 μM |
| 8 | 1,6-$Pr_2$GalNAz | 100 μM |
| 9 | 1,6-$Bu_2$GalNAz | 50 μM |
| 10 | GalNAz | 2 mM |
| 11 | 1,6-$Ac_2$GlcNAz | 500 μM |
| 12 | 1,6-$Pr_2$GlcNAz | 100 μM |
| 13 | 1,6-$Bu_2$GlcNAz | 50 μM |
| 14 | $Ac_4$GlcNAz | 50 μM |
| 15 | $Ac_4$GalNAz | 50 μM |
| 16 | 1-O-Ac-ManNAz-6-bis(Et-SATE)-P | 100 μM |
| 17 | 1-O-Ac-ManNAz-6-bis(Bu-SATE)-P | 50 μM |
| 18 | 1-O-Ac-ManNAz-6-bis(tBu-SATE)-P | 20 μM |
| 19 | 1-O-Ac-ManNAz-6-bis(Hep-SATE)-P | 50 μM |
| 20 | 1-O-Ac-6-bis(Me-SATE)-P-ManNAz | 200 μM |

(continued)

| No. | Formula | Labeling Concentration |
|---|---|---|
| 21 | 1-O-Ac-6-bis-POM-P-ManNAz | 25 μM |
| 22 | 1-O-Pr-6-bis-POM-P-ManNAz | 20 μM |
| 23 | $Ac_3$-6-bis(Me-SATE)-P-ManNAz | 25 μM |
| 24 | 1-O-Pr-6-(Gly-OMe-ProTide)-P-ManNAz | 250 μM |
| 25 | 1-O-Pr-6-(Leu-OEt-ProTide)-P-ManNAz | 200 μM |
| 26 | 1-O-Pr-6-(Ala-OMe-ProTide)-P-ManNAz | 200 μM |
| 27 | 1-O-Pr-6-(Ala-OEt-ProTide)-P-ManNAz | 300 μM |
| 28 | 1-O-Pr-6-(Ala-OBn-ProTide)-P-ManNAz | 300 μM |
| 29 | SiaNAz | 2 mM |
| 30 | 9AzSiaNAz | 2 mM |
| 31 | 9AzSia | 2 mM |
| 32 | 9AzSiaNAl | 2 mM |
| 33 | SiaNAz1Met | 2 mM |
| 34 | 9AzSiaNAz1Met | 2 mM |
| 35 | 9AzSia1Met | 2 mM |
| 36 | 9AzSiaNAl1Met | 2 mM |
| 37 | $Ac_5$SiaNAz1Met | 100 μM |
| 38 | $Ac_4$9AzSiaNAz1Met | 100 μM |
| 39 | $Ac_4$9AzSia1Met | 100 μM |
| 40 | $Ac_4$9AzSiaNAl1Met | 100 μM |
| 41 | 6AzFucose | 2 mM |
| 42 | $Ac_3$6AzFucose | 100 μM |

(2) Using DBCO-PEG-biotin and SA-488 as primary and secondary antibodies, respectively, flow cytometry (Wellgrow) was used to detect whether orthogonal groups were labeled on the cell surface.

Specifically, $1\times10^5$ cells were taken from each group into EP tubes, centrifuged, washed, and resuspended: after centrifugation at 300 g for 3 min, the supernatant was discarded and the cells were resuspended in 1 mL of flow cytometry loading buffer (PBS containing 1% serum), centrifuged at 300 g for 3 min, and then resuspended in 200 μL of flow cytometry loading buffer. To all groups, 5 μL of 10 mM DBCO-PEG4-Biotin antibody was added, and incubated at room temperature protected from light for 30 min. After incubation, centrifugation, washing, and resuspension were performed. To all groups, 2 μL of SA-488 antibody was added, and incubated at room temperature protected from light for 30 min. After incubation, centrifugation, washing, and resuspension were performed, and the cells were transferred to corresponding labeled flow cytometry tubes for flow cytometry analysis. The results are shown in Figure 8 (γδ T cells), Figure 13 (T cells), and Figure 18 (NK cells). Compared with the control group, it was concluded that although the labeling efficiency of various non-natural sugars varied for each cell type, all were labeled.

### 3.2 Labeling of Cells with Antibodies

(1) Experimental group cells labeled with non-natural sugars for 48 h and control group cells were taken out, centrifuged at 300 g for 3 min, washed with 10 mL of medium to remove residual sugars.

(2) The cells were labeled with rituximab at a density of $1\times10^7$ cells/mL and a concentration of 20 μM, and with Hu7v3 at a density of $1\times10^7$ cells/mL and a concentration of 20 μM. Incubation was carried out at 37°C for 1 h. After incubation, the supernatant was discarded by centrifugation, the cells were washed once with medium, counted, and

resuspended in an appropriate volume of medium.

### 3.3 Flow Cytometry Measurement

For flow cytometry measurement of both antibodies, FITC Anti-Human IgG Fc antibody was used.

(1) $1\times10^5$ cells from all groups were taken into EP tubes, centrifuged at 300 g for 3 min, and the supernatant was discarded. 1 mL of flow cytometry loading buffer was added to each tube to resuspend the cells, followed by washing by centrifugation at 300 g for 3 min.
(2) After washing, 200 μL of flow cytometry loading buffer was added to each tube to resuspend the cells.
(3) 1 μL of FITC Anti-Human IgG Fc antibody was added to all groups, the tubes were flicked gently to distribute the antibody evenly, and incubated at room temperature protected from light for 30 min.
(4) After incubation, centrifugation at 300 g for 3 min, the supernatant was discarded. The cells were resuspended in 1 mL of flow cytometry loading buffer, centrifuged at 300g for 3min, and the supernatant was discarded to wash away residual antibodies.
(5) The cells were resuspended in 500 μL of flow cytometry loading buffer, transferred to corresponding labeled flow cytometry tubes, and analyzed by flow cytometry.

Flow cytometry was performed to detect the labeling of rituximab and Hu7v3 antibodies on the cells.
The results are shown in Figures 9, 10, 14, 15, 19, and 20. Compared with the control group, it was concluded that γδ T cells, T cells, and NK cells can all be labeled with these two antibodies.

### Example 4: Determination of Killing Efficiency of Target Cells

(1) Calcein-AM labeling of target cells:

① Cell counting: In a biosafety cabinet, the cultured Raji cells and SNU-620 cells were gently mixed and transferred entirely to 15-mL centrifuge tubes, centrifuged at 300 g for 3 min.
② After centrifugation, the tube walls and caps were sprayed with alcohol and placed in the biosafety cabinet. The lids of the 15-mL tubes were opened in the cabinet, the supernatant was discarded, and 1 mL of corresponding complete medium was added for resuspension. After mixing by pipetting, 10 μL was taken and mixed at a ratio of 1:1 with 10 μL of AOPI staining solution. 12 μL was taken for counting using a cell counter. Live cell count and viability were recorded.
③ Based on the counting results, an appropriate number of cells were taken into labeled 15-mL centrifuge tubes.
④ 10 mL of killing buffer was added to each centrifuge tube, centrifuged at room temperature, 300 g for 5 min, and the supernatant was discarded. Then, the bottom of the tube was gently tapped with a fingertip to disperse the cell pellet.
⑤ Killing buffer was added to resuspend the cells, adjusting the cell density to $1\times10^6$ cells/mL. Then, Calcein-AM solution (Calcein-AM should be mixed well before use) was added to the cell suspension in the tube at a volume ratio of CAM:cell suspension = 1:100, and mixed gently by pipetting. The cell suspension was placed in a 37°C incubator for 30 min.
⑥ After incubation, 10 mL of killing buffer was added to the tube to wash the cells, centrifuged at room temperature, 300 g for 5 min, and the supernatant was discarded. The washing step was repeated twice. After each supernatant removal, the tube bottom was gently tapped with a finger to disperse the cells. Finally, an appropriate volume of killing buffer was added to the tube to resuspend the cells, resulting in a cell suspension density of $1\times10^5$ cells/mL.
⑦ Cells labeled with rituximab were used to kill Raji cells, and cells labeled with Hu7v3 were used to kill SNU-620 cells, to determine the killing efficiency of the two antibodies, respectively.

(2) Preparation of γδ T cells, T cells, NK cells

① Cell counting: In a biosafety cabinet, cells labeled with the two antibodies were gently mixed, 10 μL was taken and mixed at a ratio of 1:1 with 10 μL of AOPI staining solution. 12 μL was taken for counting using a cell counter, and live cell count and viability were recorded.
② Based on the above counting results, for an effector-to-target ratio of 10:1, i.e., $1\times10^5$ cells/well, 4 replicate wells were made, i.e., $4\times10^5$ γδ T cells, T cells, or NK cells were taken from each group into labeled 15-mL centrifuge tubes.
③ 10 mL of killing buffer was added to each centrifuge tube, centrifuged at room temperature, 300 g for 5 min, and the

supernatant was discarded. The tube bottom was gently tapped with a fingertip to disperse the cell pellet. The operation was repeated once.
④ Killing buffer was added to the centrifuge tube to adjust the viable cell density to 1×10$_6$ cells/mL.

(3) Co-culture of γδ T cells, T cells, NK cells with target cells

① 100 μL of γδ T cells, T cells, or NK cells were added to the corresponding marked wells of a U-bottom 96-well plate, with 4 replicate wells for each effector-to-target ratio; 100 μL of lysis buffer and 100 μL of killing buffer were added to the corresponding marked wells, respectively.
② 100 μL of Calcein-AM labeled target cells were added to the corresponding marked wells.
③ The U-bottom 96-well plate was placed in a 37°C incubator and incubated for 2.5 h.

(4) Result reading

① The 96-well plate was taken out from the incubator, centrifuged at 400 g for 10 min. 150 μL of supernatant was taken from each well (taking care to take from the middle-upper layer of the liquid surface, avoiding the bottom pellet) and transferred to a black-bottom 96-well plate (sample positions should correspond to those marked in the table).
② The plate was read using a microplate reader with fluorescence colorimetry (488 nm excitation, 520 nm emission) to measure the absorbance of each well.

(5) Data processing and statistical analysis of results

Calculation formula:

Specific lysis percentage = (Experimental group fluorescence value - Spontaneous release group fluorescence value) / (Maximum release group fluorescence value - Spontaneous release group fluorescence value) × 100%.

Experimental group fluorescence value = Average fluorescence value of target cells and specific concentration of effector cells.
Spontaneous release group fluorescence value = Average fluorescence value of target cells and killing buffer.
Maximum release group fluorescence value = Average fluorescence value of target cells and lysis buffer.

The results are shown in Tables 2 to 7, and Figures 11, 12, 16, 17, 21, and 22. The results show that after labeling with the two antibodies, γδ T cells, NK cells, and T cells all have higher killing efficiency compared to unlabeled cells (control group), exhibiting targeted killing effects.

**Table 2: Killing results of Raji cells by γδ T cells labeled with 42 sugars followed by labeling with rituximab.**

| Non-natural Sugar No. | Killing Rate (%) | Non-natural Sugar No. | Killing Rate (%) |
|---|---|---|---|
| 1 | 67.79 | 22 | 78.67 |
| 2 | 76.51 | 23 | 47.62 |
| 3 | 82.05 | 24 | 87.94 |
| 4 | 82.49 | 25 | 45.86 |
| 5 | 72.89 | 26 | 67.62 |
| 6 | 32.72 | 27 | 59.87 |
| 7 | 31.94 | 28 | 37.43 |
| 8 | 29.51 | 29 | 88.99 |

(continued)

| Non-natural Sugar No. | Killing Rate (%) | Non-natural Sugar No. | Killing Rate (%) |
|---|---|---|---|
| 9 | 33 | 30 | 87.62 |
| 10 | 80.62 | 31 | 87.07 |
| 11 | 77.95 | 32 | 86.78 |
| 12 | 76.69 | 33 | 87.54 |
| 13 | 80.01 | 34 | 89.75 |
| 14 | 25.41 | 35 | 86.74 |
| 15 | 71.63 | 36 | 88.82 |
| 16 | 63.85 | 37 | 88.77 |
| 17 | 47.92 | 38 | 85.78 |
| 18 | 51.21 | 39 | 92.17 |
| 19 | 47.08 | 40 | 88.93 |
| 20 | 55.33 | 41 | 28.24 |
| 21 | 82.91 | 42 | 27.8 |
| | | Control | 11.44 |

**Table 3: Killing results of SNU-620 cells by γδ T cells labeled with 42 sugars followed by labeling with Hu7v3 antibody.**

| Non-natural Sugar No. | Killing Rate (%) | Non-natural Sugar No. | Killing Rate (%) | Non-natural Sugar No. | Killing Rate (%) |
|---|---|---|---|---|---|
| 1 | 38.49 | 15 | 35.61 | 29 | 16.25 |
| 2 | 37.3 | 16 | 46.52 | 30 | 21.27 |
| 3 | 52.49 | 17 | 56.83 | 31 | 24.87 |
| 4 | 36.35 | 18 | 46.29 | 32 | 16.35 |
| 5 | 37.76 | 19 | 39.27 | 33 | 19.02 |
| 6 | 14.99 | 20 | 40.72 | 34 | 38.08 |
| 7 | 16.66 | 21 | 32.23 | 35 | 31.81 |
| 8 | 11.56 | 22 | 35.12 | 36 | 35.15 |
| 9 | 19.63 | 23 | 45.68 | 37 | 30.98 |
| 10 | 29.3 | 24 | 51.14 | 38 | 29.04 |
| 11 | 27.44 | 25 | 45.23 | 39 | 26.81 |
| 12 | 28.36 | 26 | 39.15 | 40 | 13.13 |
| 13 | 48.21 | 27 | 33.29 | 41 | 14.21 |
| 14 | 20.16 | 28 | 43.19 | 42 | 13.89 |
| | | | | Control | 2.6 |

**Table 4: Killing results of Raji cells by NK cells labeled with 42 sugars followed by labeling with rituximab.**

| Non-natural Sugar No. | Killing Rate (%) | Non-natural Sugar No. | Killing Rate (%) |
|---|---|---|---|
| 1 | 93.98 | 22 | 88.74 |
| 2 | 93.22 | 23 | 83.87 |
| 3 | 92.04 | 24 | 35.78 |

(continued)

| Non-natural Sugar No. | Killing Rate (%) | Non-natural Sugar No. | Killing Rate (%) |
|---|---|---|---|
| 4 | 84.69 | 25 | 36.56 |
| 5 | 66.91 | 26 | 35.69 |
| 6 | 41.93 | 27 | 35.62 |
| 7 | 62.58 | 28 | 39.04 |
| 8 | 68.61 | 29 | 63.83 |
| 9 | 68.62 | 30 | 69.09 |
| 10 | 63.86 | 31 | 66.31 |
| 11 | 65.13 | 32 | 60.34 |
| 12 | 58.34 | 33 | 64.17 |
| 13 | 62.69 | 34 | 58.16 |
| 14 | 67.83 | 35 | 56 |
| 15 | 50.83 | 36 | 58.59 |
| 16 | 42.01 | 37 | 53.48 |
| 17 | 45.21 | 38 | 45.47 |
| 18 | 45.95 | 39 | 55.6 |
| 19 | 53.78 | 40 | 55.22 |
| 20 | 67.33 | 41 | 66.26 |
| 21 | 73.01 | 42 | 64.07 |
| | | Control | 20.88 |

**Table 5: Killing results of SNU-620 cells by NK cells labeled with 42 sugars followed by labeling with Hu7v3 antibody.**

| Non-natural Sugar No. | Killing Rate (%) | Non-natural Sugar No. | Killing Rate (%) | Non-natural Sugar No. | Killing Rate (%) |
|---|---|---|---|---|---|
| 1 | 73.69 | 15 | 47.43 | 29 | 77.83 |
| 2 | 72.38 | 16 | 38.18 | 30 | 90.76 |
| 3 | 68.62 | 17 | 36.92 | 31 | 88.86 |
| 4 | 68.44 | 18 | 38.15 | 32 | 77.66 |
| 5 | 52.75 | 19 | 49.12 | 33 | 72.92 |
| 6 | 37.13 | 20 | 56.93 | 34 | 76.61 |
| 7 | 47.08 | 21 | 72.82 | 35 | 64.57 |
| 8 | 58.86 | 22 | 64.98 | 36 | 58.02 |
| 9 | 57.74 | 23 | 49.21 | 37 | 52.18 |
| 10 | 47.59 | 24 | 36.66 | 38 | 53.58 |
| 11 | 43.48 | 25 | 32.28 | 39 | 51.13 |
| 12 | 71.09 | 26 | 31.79 | 40 | 55.58 |
| 13 | 54.22 | 27 | 38.88 | 41 | 78.96 |
| 14 | 53.4 | 28 | 35.24 | 42 | 87.69 |
| | | | | Control | 19.45 |

**Table 6: Killing results of Raji cells by T cells labeled with 42 sugars followed by labeling with rituximab.**

| Non-natural Sugar No. | Killing Rate (%) | Non-natural Sugar No. | Killing Rate (%) | Non-natural Sugar No. | Killing Rate (%) |
|---|---|---|---|---|---|
| 1 | 70.87 | 15 | 70.82 | 29 | 7 |
| 2 | 65.93 | 16 | 67.17 | 30 | 73.73 |
| 3 | 76.33 | 17 | 72.43 | 31 | 52.07 |
| 4 | 79.6 | 18 | 79.43 | 32 | 55.87 |
| 5 | 67.23 | 19 | 74.43 | 33 | 65.73 |
| 6 | 34.93 | 20 | 71.53 | 34 | 52.4 |
| 7 | 34.33 | 21 | 79.63 | 35 | 56.03 |
| 8 | 32.83 | 22 | 73.13 | 36 | 56.67 |
| 9 | 36.92 | 23 | 74.83 | 37 | 61.2 |
| 10 | 80.52 | 24 | 82.4 | 38 | 72.31 |
| 11 | 74.83 | 25 | 83.57 | 39 | 71.51 |
| 12 | 73.67 | 26 | 81.24 | 40 | 80.77 |
| 13 | 75.47 | 27 | 82.33 | 41 | 30.87 |
| 14 | 29.32 | 28 | 83.44 | 42 | 31.8 |
| Control | 15.17 | | | | |

**Table 7: Killing results of SNU-620 cells by T cells labeled with 42 sugars followed by labeling with Hu7v3 antibody**.

| Non-natural Sugar No. | Killing Rate (%) | Non-natural Sugar No. | Killing Rate (%) | Non-natural Sugar No. | Killing Rate (%) |
|---|---|---|---|---|---|
| 1 | 48.43 | 15 | 45.63 | 29 | 27.32 |
| 2 | 47.37 | 16 | 56.57 | 30 | 33.07 |
| 3 | 62.83 | 17 | 66.53 | 31 | 35.94 |
| 4 | 47.8 | 18 | 56.73 | 32 | 27.35 |
| 5 | 48.43 | 19 | 50.03 | 33 | 30.36 |
| 6 | 25.53 | 20 | 50.77 | 34 | 49.27 |
| 7 | 17.73 | 21 | 45.43 | 35 | 41.67 |
| 8 | 22.23 | 22 | 46.37 | 36 | 46.23 |
| 9 | 29.43 | 23 | 55.43 | 37 | 41.57 |
| 10 | 40.63 | 24 | 61.93 | 38 | 40.03 |
| 11 | 38.93 | 25 | 55.43 | 39 | 36.73 |
| 12 | 39.32 | 26 | 50.33 | 40 | 19.63 |
| 13 | 58.73 | 27 | 44.34 | 41 | 14.77 |
| 14 | 31.27 | 28 | 55.47 | 42 | 13.07 |
| | | | | Control | 2.27 |

**Example 5: In Vivo Tumor Inhibition Experiment in Mouse Model**

γδ T cells labeled with rituximab targeting CD20 (CD20-γδ T) prepared in Example 3 were used as effector cells for in vivo tumor inhibition in a mouse model. The tumor cells in the mice were Raji-Luc cells.

Raji-Luc cells were expanded. Two days in advance, NPG mice were injected intravenously with Raji-Luc cells,

6×10⁵ cells per mouse; administration was performed on days 0, 2, 4, and 6. The PBS group received 100 μL PBS, while other groups received 5×10⁶ cells dissolved in 100 μL PBS. Bioluminescence imaging was performed on days 8, 16, and 24. Mouse death was recorded.

From the bioluminescence imaging (Figures 23, 25, and 27), compared with the control group, it can be concluded that CD20-γδ T products prepared with the 13 sugars can significantly inhibit tumor growth in mice. From the mouse survival curves (Figures 24, 26, and 28) compared with the PBS group, it can be concluded that CD20-γδ T products prepared with the 13 sugars can significantly prolong mouse survival.

γδ T cells labeled with Hu7v3 targeting Claudin18.2 (Claudin18.2-γδ T) prepared in Example 3 were used as effector cells for in vivo tumor inhibition in a mouse model. The tumor cells in the mice were SNU-620-Luc cells.

SNU-620-Luc cells were expanded. Two days in advance, NSG mice were injected intravenously with SNU-620-Luc cells, 5×10⁶ cells per mouse; administration was performed on days 0, 2, 4, 6, and 8. The PBS group received 100 μL PBS, while other groups received 5×10⁶ cells dissolved in 100 μL PBS. Tumor volume was measured and calculated on days 0, 8, 16, 24, 32, 40, and 48. Mouse death was recorded.

From the tumor volume data (Figures 29 to 31), compared with the control group, it can be concluded that Claudin18.2-γδ T products prepared with the 13 sugars can all significantly inhibit tumor growth in mice.

Figure legend: "20-CD20-γδT" indicates γδ T cells linked with CD20 antibody via non-natural sugar No. 20, and so forth. PBS is the solvent control group, γδT represents the cell control group without antibody linking.

## Claims

1. A method for preparing modified cells, comprising step S: contacting an immune cell expressing on its surface a monosaccharide derivative comprising a first functional group with an antigen-binding protein comprising a second functional group, such that the first functional group and the second functional group are linked and reacted to obtain the modified cell; wherein the monosaccharide derivative comprises any one or more structures selected from Formula I to Formula X:

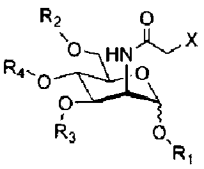
(Formula I)

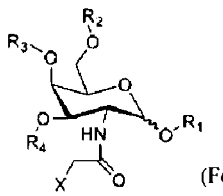
(Formula II)

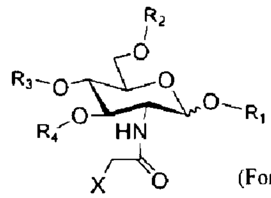
(Formula III)

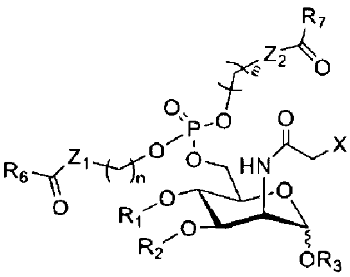
(Formula IV)

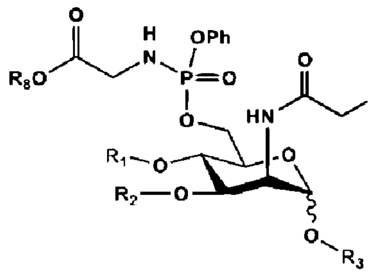
(Formula V)

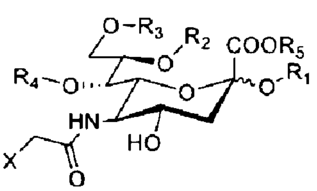
(Formua VI)

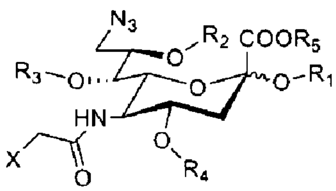
(Formula VII)

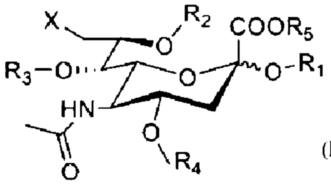 (Formula VIII) 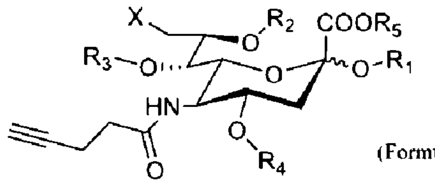 (Formula IX)

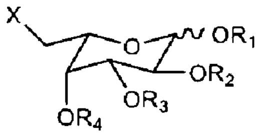 (Formula X)

wherein X is the first functional group;

$R_1$, $R_2$, $R_3$, and $R_4$ are each independently selected from the group consisting of hydrogen, halogen, substituted or unsubstituted $C_{1-6}$ alkyl, $C_{1-6}$ alkylthio, $C_{1-6}$ alkylcarbonyl, $C_{2-6}$ alkenyl, $C_{2-6}$ alkynyl, $C_{2-6}$ alkoxy, $C_{2-6}$ alkoxycarbonyl, and acyl, wherein said group is optionally substituted with a substituent selected from the group consisting of halogen, hydroxyl, amino, nitro, mercapto, and $C_{1-6}$ alkyl;

$R_5$ is selected from the group consisting of hydrogen, halogen, and substituted or unsubstituted $C_{1-6}$ alkyl, wherein said alkyl is optionally substituted with a substituent selected from the group consisting of halogen, hydroxyl, amino, nitro, and mercapto;

$R_6$ and $R_7$ are each independently selected from the group consisting of halogen and substituted or unsubstituted $C_{1-10}$ alkyl, wherein said alkyl is optionally substituted with a substituent selected from the group consisting of halogen, hydroxyl, amino, nitro, and mercapto;

$R_8$ is selected from the group consisting of halogen, substituted or unsubstituted $C_{1-10}$ alkyl, phenyl, and substituted or unsubstituted $C_{1-10}$ alkylene-phenyl, wherein said alkyl is optionally substituted with a substituent selected from the group consisting of halogen, hydroxyl, amino, nitro, and mercapto;

$Z_1$ and $Z_2$ are each independently selected from O and S;

n and m are each independently selected from the integers 1, 2, 3, 4, 5, and 6.

**2.** The method according to claim 1, further comprising step S1: contacting a monosaccharide derivative comprising a first functional group with an immune cell to obtain the immune cell expressing on its surface the monosaccharide derivative comprising the first functional group.

**3.** The method according to claim 1 or 2, further comprising step S2: contacting an antigen-binding protein with a compound comprising a second functional group to obtain the antigen-binding protein comprising the second functional group.

**4.** The method according to any one of claims 1-3, wherein $R_1$, $R_2$, $R_3$, and $R_4$ are each independently selected from the group consisting of hydrogen and -C(=O)-$C_{1-6}$ alkyl, preferably hydrogen and $-C(=O)-CH_3$.

**5.** The method according to any one of claims 1-4, wherein $R_6$ and $R_7$ are each independently selected from substituted or unsubstituted $C_{1-10}$ alkyl.

**6.** The method according to any one of claims 1-5, wherein $R_8$ is selected from: methyl, ethyl, and $-CH_2$-phenyl.

**7.** The method according to any one of claims 1-6, wherein n and m are each independently selected from the integers 1, 2, and 3.

**8.** The method according to any one of claims 1-7, wherein the first functional group and/or the second functional group is a bioorthogonal reactive group, preferably, the bioorthogonal reaction comprises at least one selected from the group consisting of: click chemistry, photo-crosslinking, photosensitive reaction, and glycosylation.

**9.** The method according to claim 8, wherein the first functional group comprises one or more selected from the group consisting of azido, alkynyl, trans-cyclooctene, triazine, tetrazine, acylhydrazone, cyclopropene, isonitrile, and cyclooctyne.

**10.** The method according to any one of claims 8-9, wherein the second functional group comprises one or more selected

from the group consisting of azido, alkynyl, trans-cyclooctene, triazine, tetrazine, acylhydrazone, cyclopropene, isonitrile, and cyclooctyne.

**11.** The method according to any one of claims 1-10, wherein X is an azido group.

**12.** The method according to any one of claims 1-11, wherein the first functional group is an azido group, and the second functional group is

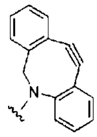

.

**13.** The method according to any one of claims 1-12, wherein the monosaccharide derivative comprising a first functional group is selected from any one of the following:

| No. | Formula | Structure |
|---|---|---|
| 1 | ManNAz | 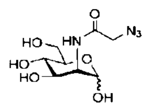 |
| 2 | 1,6-$Ac_2$ManNAz | 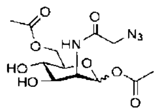 |
| 3 | 1,6-$Pr_2$ManNAz | 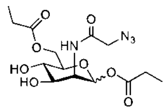 |
| 4 | 1,6-$Bu_2$ManNAz | 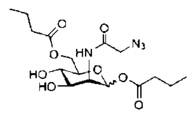 |
| 5 | Ac4ManNAz | 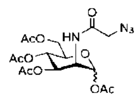 |
| 6 | GlcNAz | 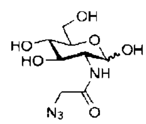 |
| 7 | 1,6-$Ac_2$GalNAz | 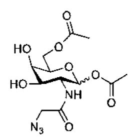 |
| 8 | 1,6-$Pr_2$GalNAz | 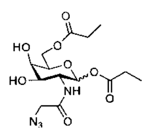 |
| 9 | 1,6-$Bu_2$GalNAz | 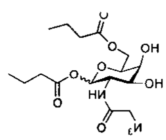 |

(continued)

| No. | Formula | Structure |
|---|---|---|
| 10 | GalNAz | 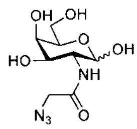 |
| 11 | 1,6-$Ac_2$GlcNAz | 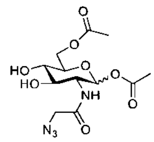 |
| 12 | 1,6-$Pr_2$GlcNAz | 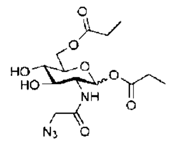 |
| 13 | 1,6-$Bu_2$GlcNAz | 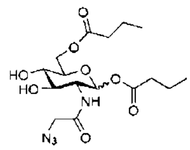 |
| 14 | Ac4GlcNAz | 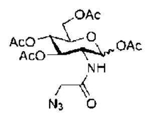 |
| 15 | Ac4GalNAz | 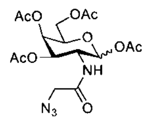 |
| 16 | 1-O-Ac-ManNAz-6-bis(Et-SATE)-P | 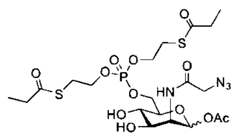 |
| 17 | 1-O-Ac-ManNAz-6-bis(Bu-SATE)-P | 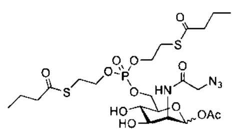 |
| 18 | 1-O-Ac-ManNAz-6-bis(tBu-SATE)-P | 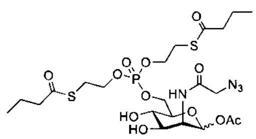 |
| 19 | 1-O-Ac-ManNAz-6-bis(Hep-SATE)-P | 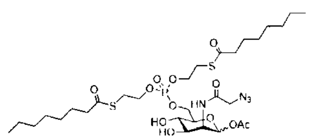 |
| 20 | 1-O-Ac-6-bis(Me-SATE)-P-ManNAz | 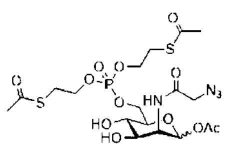 |

(continued)

| No. | Formula | Structure |
|---|---|---|
| 21 | 1-O-Ac-6-bis-POM-P-ManNAz | 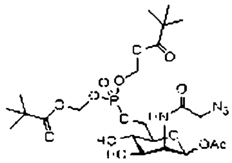 |
| 22 | 1-O-Pr-6-bis-POM-P-ManNAz | 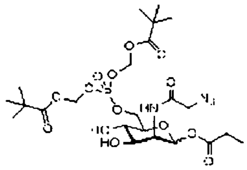 |
| 23 | $Ac_3$-6-bis(Me-SATE)-P-ManNAz | 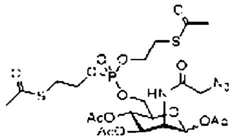 |
| 24 | 1-O-Pr-6-(Gly-OMe-ProTide)-P-ManNAz | 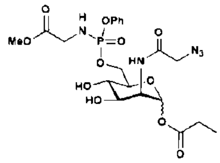 |
| 25 | 1-O-Pr-6-(Leu-OEt-ProTide)-P-ManNAz | 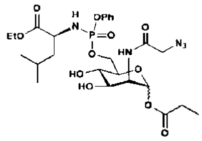 |
| 26 | 1-O-Pr-6-(Ala-OMe-ProTide)-P-ManNAz | 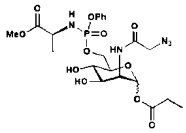 |
| 27 | 1-O-Pr-6-(Ala-OEt-ProTide)-P-ManNAz | 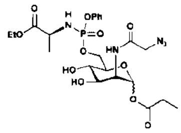 |
| 28 | 1-O-Pr-6-(Ala-OBn-ProTide)-P-ManNAz | 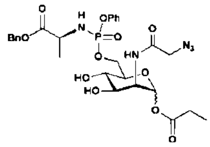 |
| 29 | SiaNAz | 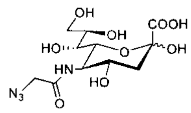 |
| 30 | 9AzSiaNAz | 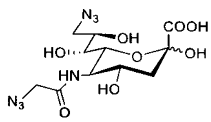 |

(continued)

| No. | Formula | Structure |
|---|---|---|
| 31 | 9AzSia | 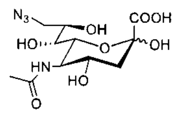 |
| 32 | 9AzSiaNAl | 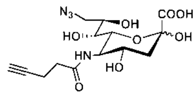 |
| 33 | SiaNAz1Met | 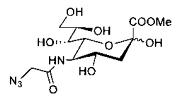 |
| 34 | 9AzSiaNAz1Met | 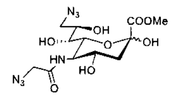 |
| 35 | 9AzSia1Met | 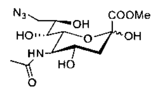 |
| 36 | 9AzSiaNAl1Met | 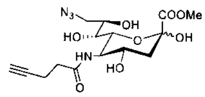 |
| 37 | Ac$_5$SiaNAz1Met | 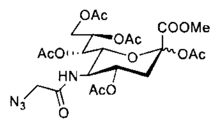 |
| 38 | Ac$_4$9AzSiaNAz1Met | 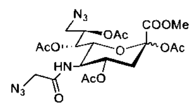 |
| 39 | Ac$_4$9AzSia1Met | 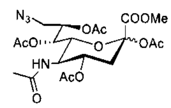 |
| 40 | Ac$_4$9AzSiaNAl1Met | 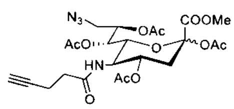 |
| 41 | 6AzFucose | 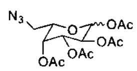 |
| 42 | Ac$_3$6AzFucose | 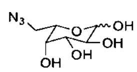 |

**14.** The method according to any one of claims 3-13, wherein the compound comprising a second functional group further comprises a group capable of covalently linking to a thiol group; preferably, the group capable of covalently linking to a thiol group is a succinimidyl ester group, a maleimide group, or a maleic anhydride group.

**15.** The method according to any one of claims 3-14, wherein the structure of the compound comprising a second functional group is as shown in Formula L1, Formula L2, or Formula L3:

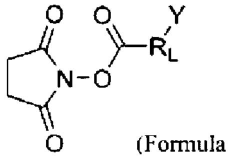
(Formula L1)

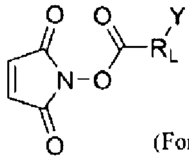
(Formula L2)

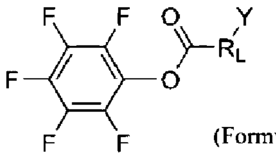
(Formula L3)

wherein Y is the second functional group; $R_L$ is selected from the group consisting of polyethylene glycol, amide group, and alkyl ether group; preferably, $R_L$ is selected from $C_{1-20}$ alkylene or $C_{1-20}$ alkylene ether group.

**16.** The method according to claim 15, wherein the compound of Formula L1 in step S2 is DBCO-PEG4-NHS:

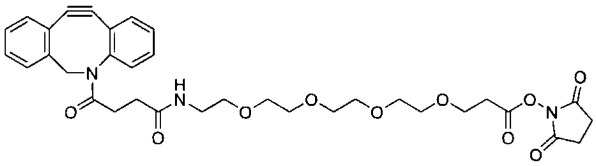

NHS: DBCO-PEG4-NHS

**17.** The method according to claim 16, wherein the compound of Formula L2 in step S2 is DBCO-PEG4-MAL:

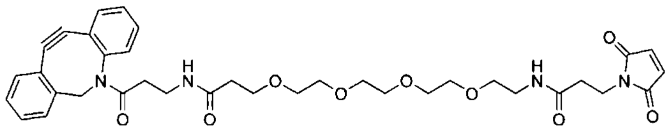

MAL: DBCO-PEG4-MAL

**18.** The method according to any one of claims 1-17, wherein the immune cell comprises NK cells, DC cells, NKT cells, monocytes, macrophages, T cells, and/or B cells.

**19.** The method according to any one of claims 1-18, wherein the immune cell is a T cell.

**20.** The method according to any one of claims 1-19, wherein the immune cell is a γδ T cell.

**21.** The method according to claim 20, wherein the γδ T cell comprises Vγ9Vδ2 T cells and/or Vδ1 T cells.

**22.** The method according to any one of claims 1-21, wherein the antigen-binding protein comprises an antibody or an antigen-binding fragment thereof.

**23.** The method according to claim 22, wherein the antigen-binding protein comprises a Fab, Fab', F(ab)2, Fv fragment, F(ab')2, scFv, di-scFv, VHH, and/or dAb.

**24.** The method according to any one of claims 1-23, wherein the antigen-binding protein is capable of specifically binding to a tumor antigen.

**25.** The method according to claim 24, wherein the tumor antigen comprises a tumor-associated antigen and/or a tumor-specific antigen.

**26.** The method according to claim 24 or 25, wherein the tumor antigen is selected from the group consisting of: CD20 and claudin18.2.

**27.** The method according to any one of claims 1-26, wherein the immune cell is derived from human peripheral blood.

**28.** The method according to any one of claims 1-27, wherein the contact time in step S is about 10 min to 60 min.

**29.** The method according to any one of claims 2-28, wherein the contact time in step S1 is about 2 h to 48 h.

**30.** The method according to any one of claims 3-29, wherein the contact time in step S2 is about 30 min to 6 h.

**31.** A modified cell prepared by the method according to any one of claims 1-30.

**32.** A modified cell, having an antigen-binding protein conjugated to its surface via an azido group-containing monosaccharide derivative, wherein the structure of the azido group-containing monosaccharide derivative is selected from one or more of the following:

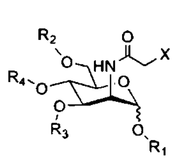 (Formula I) 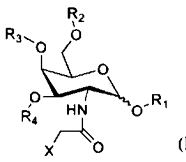 (Formula II) 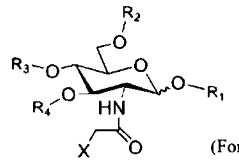 (Formula III)

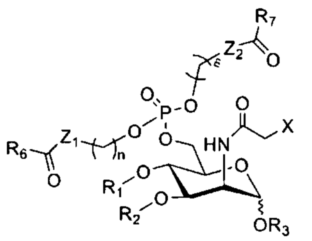 (Formula IV) 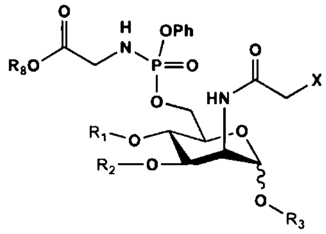 (Forumula V)

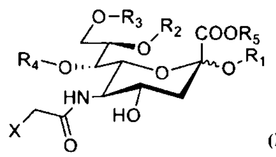 (Formula VI) 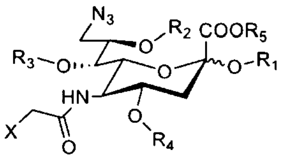 (Formula VII)

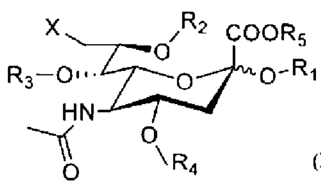 (Formula VIII) 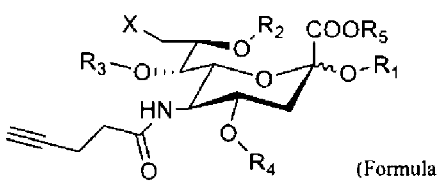 (Formula IX)

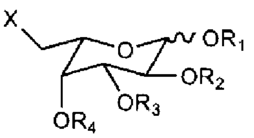 (Formula X)

wherein X is an azido group;
$R_1$, $R_2$, $R_3$, and $R_4$ are each independently selected from the group consisting of hydrogen, halogen, substituted or unsubstituted $C_{1-6}$ alkyl, $C_{1-6}$ alkylthio, $C_{1-6}$ alkylcarbonyl, $C_{2-6}$ alkenyl, $C_{2-6}$ alkynyl, $C_{2-6}$ alkoxy, $C_{2-6}$ alkoxycarbonyl, and acyl, wherein said group is optionally substituted with a substituent selected from the group consisting of: halogen, hydroxyl, amino, nitro, mercapto, and $C_{1-6}$ alkyl;
$R_5$ is selected from the group consisting of hydrogen, halogen, and substituted or unsubstituted $C_{1-6}$ alkyl, wherein said alkyl is optionally substituted with a substituent selected from the group consisting of halogen, hydroxyl, amino, nitro, and mercapto;
$R_6$ and $R_7$ are each independently selected from the group consisting of halogen and substituted or unsubstituted $C_{1-10}$ alkyl, wherein said alkyl is optionally substituted with a substituent selected from the group consisting of halogen, hydroxyl, amino, nitro, and mercapto;
$R_8$ is selected from the group consisting of halogen, substituted or unsubstituted $C_{1-10}$ alkyl, phenyl, and substituted or unsubstituted $C_{1-10}$ alkylene-phenyl, wherein said alkyl is optionally substituted with a substituent selected from the group consisting of halogen, hydroxyl, amino, nitro, and mercapto;
$Z_1$ and $Z_2$ are each independently selected from O and S;

n and m are each independently selected from the integers 1, 2, 3, 4, 5, and 6.

**33.** The modified cell according to claim 32, wherein $R_1$, $R_2$, $R_3$, and $R_4$ are each independently selected from the group consisting of hydrogen and -C(=O)-$C_{1-6}$ alkyl, preferably hydrogen and -C(=O)-$CH_3$.

**34.** The modified cell according to claim 32-33, wherein $R_6$ and $R_7$ are each independently selected from substituted or unsubstituted $C_{1-10}$ alkyl.

**35.** The modified cell according to any one of claims 32-34, wherein $R_8$ is selected from the group consisting of methyl, ethyl, and -$CH_2$-phenyl.

**36.** The modified cell according to any one of claims 32-35, wherein n and m are each independently selected from the integers 1, 2, and 3.

**37.** The modified cell according to any one of claims 32-36, wherein $R_1$, $R_2$, $R_3$, and $R_4$ are each independently selected from the group consisting of hydrogen and -C(=O)-$C_{1-6}$ alkyl, preferably hydrogen and -C(=O)-$CH_3$.

**38.** The modified cell according to any one of claims 32-37, wherein $R_6$ and $R_7$ are each independently selected from substituted or unsubstituted $C_{1-10}$ alkyl.

**39.** The modified cell according to any one of claims 32-38, wherein $R_8$ is selected from methyl, ethyl, and -$CH_2$-phenyl.

**40.** The modified cell according to any one of claims 32-39, wherein n and m are each independently selected from the integers 1, 2, and 3.

**41.** The modified cell according to any one of claims 32-40, wherein the monosaccharide derivative comprising a first functional group is selected from any one of the following:

| No. | Formula | Structure |
|---|---|---|
| 1 | ManNAz | 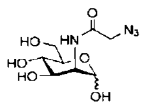 |
| 2 | 1,6-$Ac_2$ManNAz | 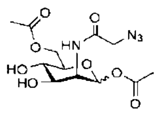 |
| 3 | 1,6-$Pr_2$ManNAz | 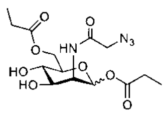 |
| 4 | 1,6-$Bu_2$ManNAz | 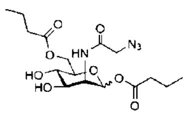 |
| 5 | Ac4ManNAz | 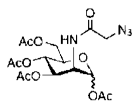 |
| 6 | GlcNAz | 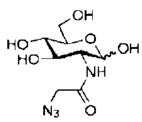 |

(continued)

| No. | Formula | Structure |
|---|---|---|
| 7 | 1,6-$Ac_2$GalNAz | 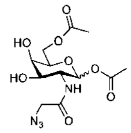 |
| 8 | 1,6-$Pr_2$GalNAz | 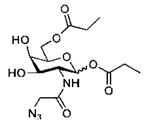 |
| 9 | 1,6-$Bu_2$GalNAz | 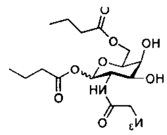 |
| 10 | GalNAz | 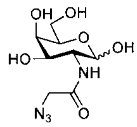 |
| 11 | 1,6-$Ac_2$GlcNAz | 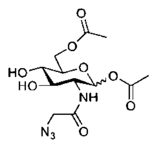 |
| 12 | 1,6-$Pr_2$GlcNAz | 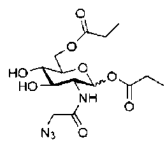 |
| 13 | 1,6-$Bu_2$GlcNAz | 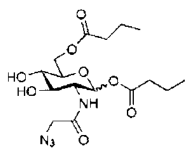 |
| 14 | Ac4GlcNAz | 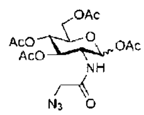 |
| 15 | Ac4GalNAz | 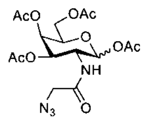 |
| 16 | 1-O-Ac-ManNAz-6-bis(Et-SATE)-P | 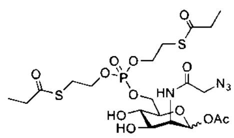 |
| 17 | 1-O-Ac-ManNAz-6-bis(Bu-SATE)-P | 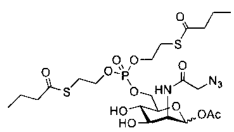 |

(continued)

| No. | Formula | Structure |
|---|---|---|
| 18 | 1-O-Ac-ManNAz-6-bis(tBu-SATE)-P | 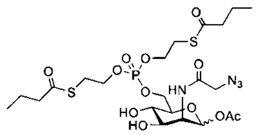 |
| 19 | 1-O-Ac-ManNAz-6-bis(Hep-SATE)-P | 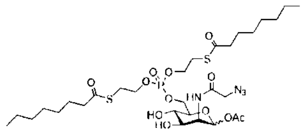 |
| 20 | 1-O-Ac-6-bis(Me-SATE)-P-ManNAz | 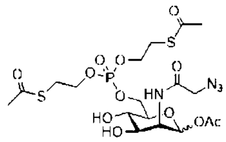 |
| 21 | 1-O-Ac-6-bis-POM-P-ManNAz | 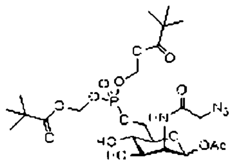 |
| 22 | 1-O-Pr-6-bis-POM-P-ManNAz | 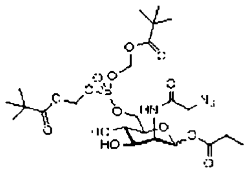 |
| 23 | $Ac_3$-6-bis(Me-SATE)-P-ManNAz | 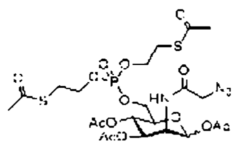 |
| 24 | 1-O-Pr-6-(Gly-OMe-ProTide)-P-ManNAz | 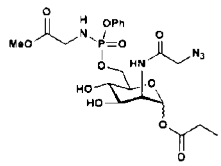 |
| 25 | 1-O-Pr-6-(Leu-OEt-ProTide)-P-ManNAz | 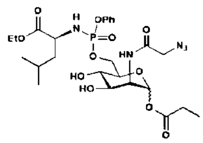 |
| 26 | 1-O-Pr-6-(Ala-OMe-ProTide)-P-ManNAz | 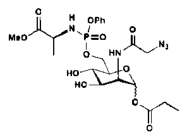 |
| 27 | 1-O-Pr-6-(Ala-OEt-ProTide)-P-ManNAz | 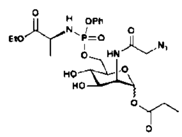 |

(continued)

| No. | Formula | Structure |
|---|---|---|
| 28 | 1-O-Pr-6-(Ala-OBn-ProTide)-P-ManNAz | 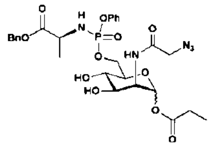 |
| 29 | SiaNAz | 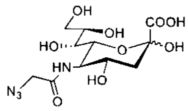 |
| 30 | 9AzSiaNAz | 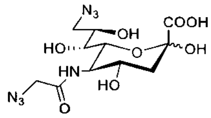 |
| 31 | 9AzSia | 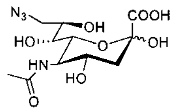 |
| 32 | 9AzSiaNAl | 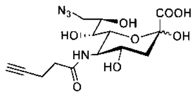 |
| 33 | SiaNAz1Met | 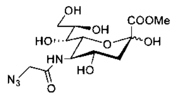 |
| 34 | 9AzSiaNAz1Met | 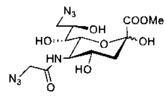 |
| 35 | 9AzSia1Met | 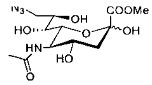 |
| 36 | 9AzSiaNAl1Met | 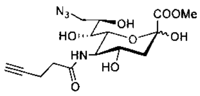 |
| 37 | $Ac_5$SiaNAz1Met | 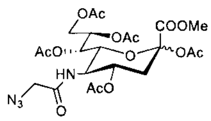 |
| 38 | $Ac_4$9AzSiaNAz1Met | 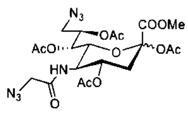 |
| 39 | $Ac_4$9AzSia1Met | 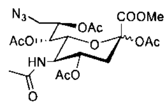 |
| 40 | $Ac_4$9AzSiaNAl1Met | 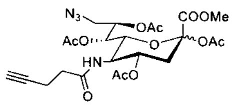 |

(continued)

| No. | Formula | Structure |
|---|---|---|
| 41 | 6AzFucose | 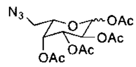 |
| 42 | $Ac_3$6AzFucose | 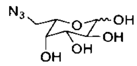 |

**42.** The modified cell according to any one of claims 32-41, wherein the cell is an immune cell, preferably an immune cell capable of inducing a cell-mediated immune response.

**43.** The modified cell according to any one of claims 32-42, wherein the cell is an NK cell (natural killer cell), a T cell, and/or a γδ T cell.

**44.** The modified cell according to any one of claims 32-43, wherein the cell has azido groups expressed on its surface by uptake of the monosaccharide derivative via the metabolic glycan pathway.

**45.** The modified cell according to any one of claims 32-44, wherein the cell is linked to the antigen-binding protein bearing the second functional group via the azido group of the monosaccharide derivative, preferably through a bond formed by a conjugation reaction between the azido group and the second functional group.

**46.** The modified cell according to any one of claims 32-45, wherein the second functional group comprises

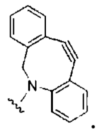

**47.** The modified cell according to any one of claims 32-46, wherein the structure of the second functional group is as shown in Formula L1, Formula L2, or Formula L3:

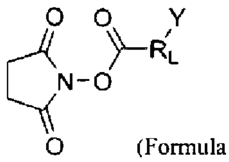

(Formula L1)

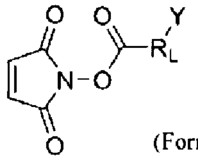

(Formula L2)

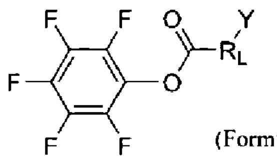

(Formula L3)

wherein, Y is the second functional group; $R_L$ is selected from the group consisting of polyethylene glycol, amide group, and alkyl ether group; preferably, $R_L$ is selected from $C_{1-20}$ alkylene or $C_{1-20}$ alkylene ether group.

**48.** The modified cell according to any one of claims 32-47, wherein the structure of the second functional group is selected from the group consisting of

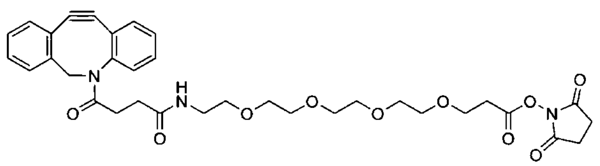

DBCO-PEG4-NHS

and

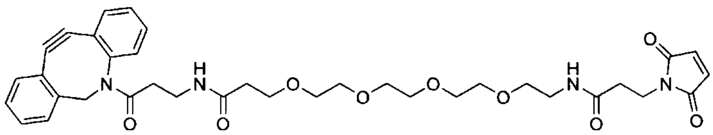

DBCO-PEG4-MAL

.

**49.** The modified cell according to any one of claims 32-38, wherein the antigen-binding protein comprises an antibody or an antigen-binding fragment thereof.

**50.** The modified cell according to any one of claims 32-49, wherein the antigen-binding protein comprises a Fab, Fab', F(ab)2, Fv fragment, F(ab')2, scFv, di-scFv, VHH, and/or dAb.

**51.** The modified cell according to any one of claims 32-50, wherein the antigen-binding protein is capable of specifically binding to a tumor antigen.

**52.** The modified cell according to any one of claims 32-51, wherein the tumor antigen comprises a tumor-associated antigen and/or a tumor-specific antigen.

**53.** The modified cell according to any one of claims 32-52, wherein the antigen-binding protein targets CD20 or claudin18.2.

**54.** The modified cell according to any one of claims 32-53, wherein the antigen-binding protein comprises an antibody heavy chain variable region HCDR3, and the HCDR3 comprises the amino acid sequence set forth in SEQ ID NO: 3.

**55.** The modified cell according to any one of claims 32-54, wherein the antigen-binding protein comprises an antibody heavy chain variable region HCDR2, and the HCDR2 comprises the amino acid sequence set forth in SEQ ID NO: 2.

**56.** The modified cell according to any one of claims 32-55, wherein the antigen-binding protein comprises an antibody heavy chain variable region HCDR1, and the HCDR1 comprises the amino acid sequence set forth in SEQ ID NO: 1.

**57.** The modified cell according to any one of claims 32-56, wherein the antigen-binding protein comprises an antibody heavy chain variable region, and the antibody heavy chain variable region comprises the amino acid sequence set forth in SEQ ID NO: 4.

**58.** The modified cell according to any one of claims 32-57, wherein the antigen-binding protein comprises an antibody light chain variable region LCDR3, and the LCDR3 comprises the amino acid sequence set forth in SEQ ID NO: 7.

**59.** The modified cell according to any one of claims 32-58, wherein the antigen-binding protein comprises an antibody light chain variable region LCDR2, and the LCDR2 comprises the amino acid sequence set forth in SEQ ID NO: 6.

**60.** The modified cell according to any one of claims 32-59, wherein the antigen-binding protein comprises an antibody light chain variable region LCDR1, and the LCDR1 comprises the amino acid sequence set forth in SEQ ID NO: 5.

**61.** The modified cell according to any one of claims 32-60, wherein the antigen-binding protein comprises an antibody light chain variable region, and the antibody light chain variable region comprises the amino acid sequence set forth in SEQ ID NO: 8.

**62.** The modified cell according to any one of claims 32-61, wherein the antigen-binding protein comprises an antibody heavy chain variable region HCDR3, and the HCDR3 comprises the amino acid sequence set forth in SEQ ID NO: 11.

**63.** The modified cell according to any one of claims 31-62, wherein the antigen-binding protein comprises an antibody heavy chain variable region HCDR2, and the HCDR2 comprises the amino acid sequence set forth in SEQ ID NO: 10.

**64.** The modified cell according to any one of claims 32-63, wherein the antigen-binding protein comprises an antibody heavy chain variable region HCDR1, and the HCDR1 comprises the amino acid sequence set forth in SEQ ID NO: 9.

**65.** The modified cell according to any one of claims 32-64, wherein the antigen-binding protein comprises an antibody

heavy chain variable region, and the antibody heavy chain variable region comprises the amino acid sequence set forth in SEQ ID NO: 12.

**66.** A pharmaceutical composition comprising the modified cell according to any one of claims 31-65.

**67.** Use of the cell according to claim 66 in the manufacture of a medicament for treating and/or alleviating a tumor.

**68.** The use according to claim 67, wherein the tumor comprises a tumor with high CD20 expression.

**69.** The use according to claim 67, wherein the tumor comprises a tumor with high claudin18.2 expression.

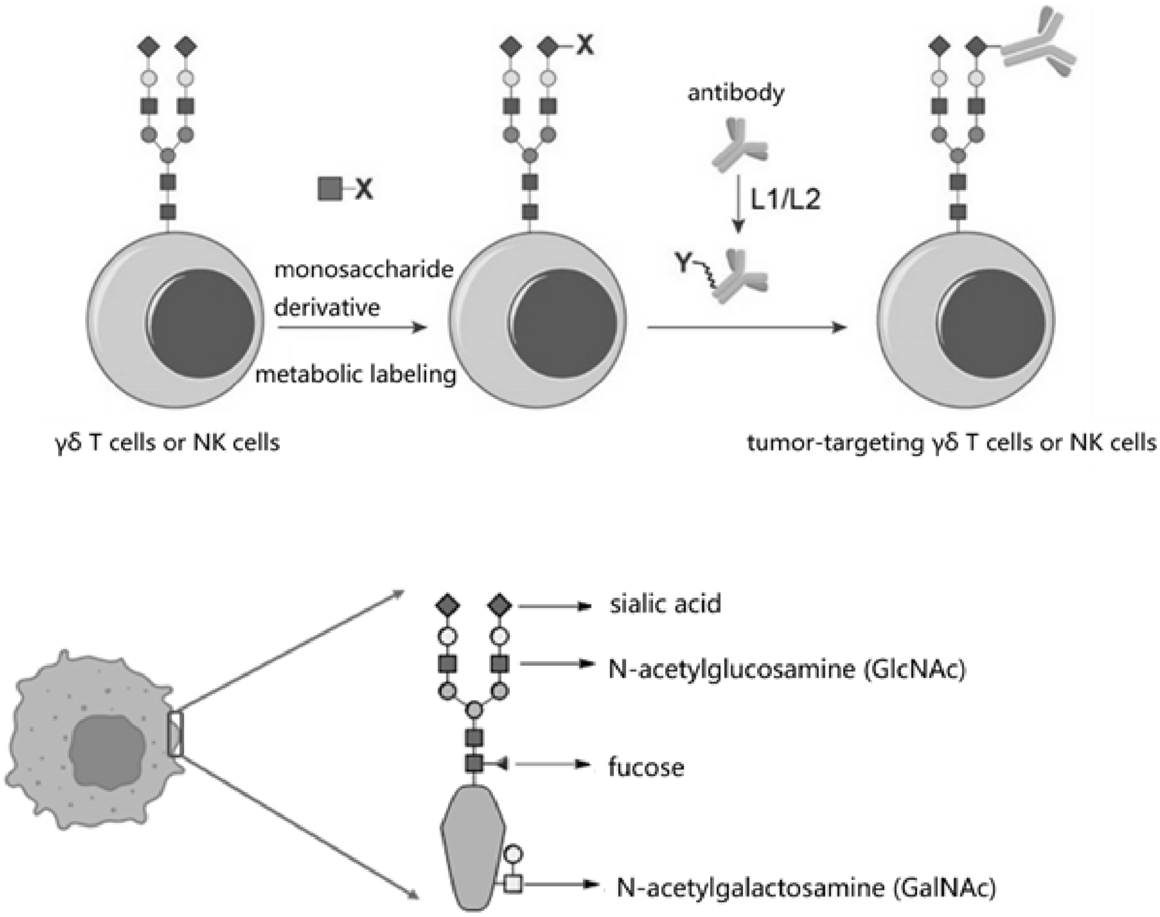
X
antibody
L1/L2
Y
monosaccharide
derivative
metabolic labeling
γδ T cells or NK cells
tumor-targeting γδ T cells or NK cells
sialic acid
N-acetylglucosamine (GlcNAc)
fucose
N-acetylgalactosamine (GalNAc)

**Figure 1**

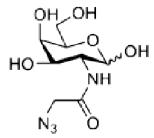

GalNAz

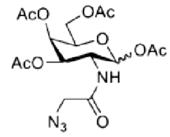

$Ac_4GalNAz$

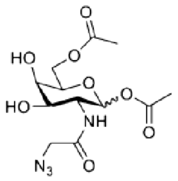

1,6-$Ac_2GalNAz$

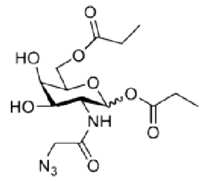

1,6-
$Pr_2GalNAz$

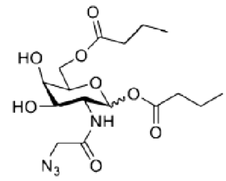

1,6-$Bu_2GalNAz$

**Figure 2**

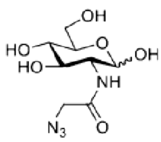

GlcNAz

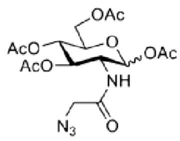

$Ac_4GlcNAz$

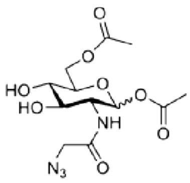

1,6-$Ac_2GlcNAz$

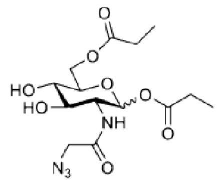

1,6-$Pr_2GlcNAz$

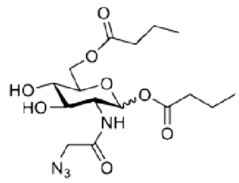

1,6-$Bu_2GlcNAz$

**Figure 3**

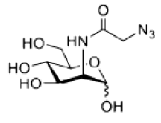

ManNAz

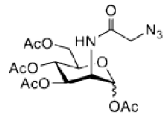

$Ac_4ManNAz$

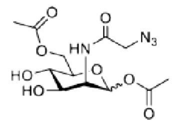

1,6-$Ac_2ManNAz$

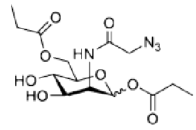

1,6-$Pr_2ManNAz$

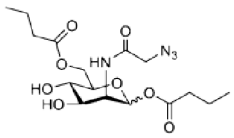

1,6-$Bu_2ManNAz$

**Figure 4**

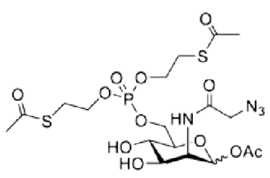

1-O-Ac-6-bis(Me-SATE)-P-ManNAz

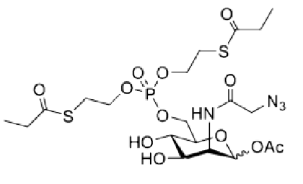

1-O-Ac-ManNAz-6-bis(Et-SATE)-P

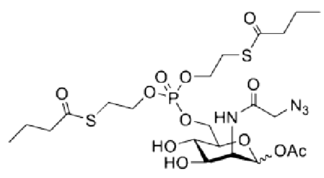

1-O-Ac-ManNAz-6-bis(Bu-SATE)-P

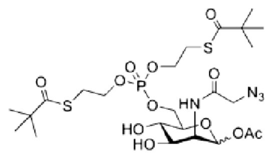

1-O-Ac-ManNAz-6-bis(tBu-SATE)-P

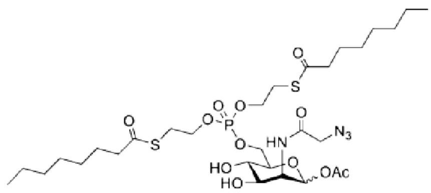

1-O-Ac-ManNAz-6-bis(Hep-SATE)-P

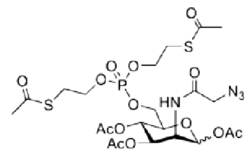

$Ac_3$-6-bis(Me-SATE)-P-ManNAz

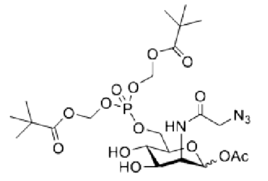

1-O-Ac-6-bis-POM-P-ManNAz

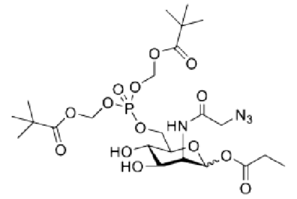

1-O-Pr-6-bis-POM-P-ManNAz

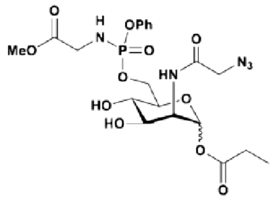

1-O-Pr-6-(Gly-OMe-ProTide)-P-ManNAz

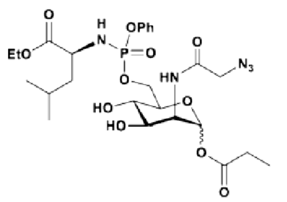

1-O-Pr-6-(Leu-OEt-ProTide)-P-ManNAz

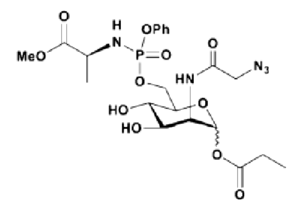

1-O-Pr-6-(Ala-OMe-ProTide)-P-ManNAz

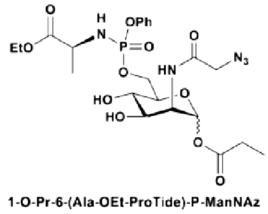

1-O-Pr-6-(Ala-OEt-ProTide)-P-ManNAz

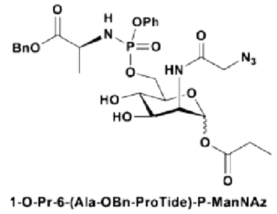

1-O-Pr-6-(Ala-OBn-ProTide)-P-ManNAz

Figure 5

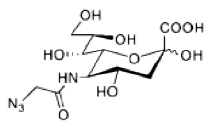
**SiaNAz**

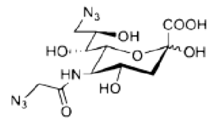
**9AzSiaNAz**

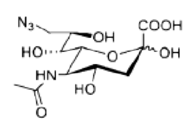
**9AzSia**

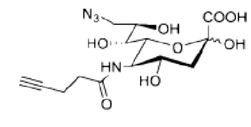
**9AzSiaNAAl**

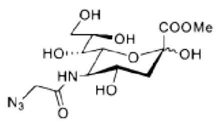
**SiaNAz1Met**

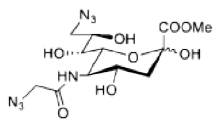
**9AzSiaNAz1Met**

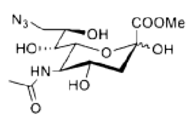
**9AzSiaMet**

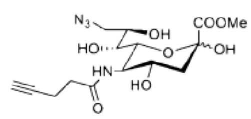
**9AzSiaNAl1Met**

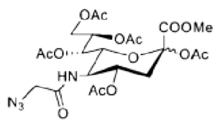
**$Ac_5$SiaNAz1Met**

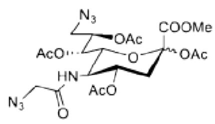
**$Ac_4$9AzSiaNAz1Met**

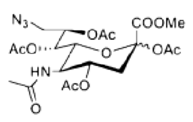
**$Ac_4$9AzSia1Met**

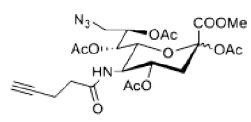
**$Ac_4$9AzSiaNAl1Met**

**Figure 6**

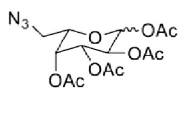
$Ac_4$6AzFucose

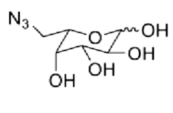
6AzFucose

**Figure 7**

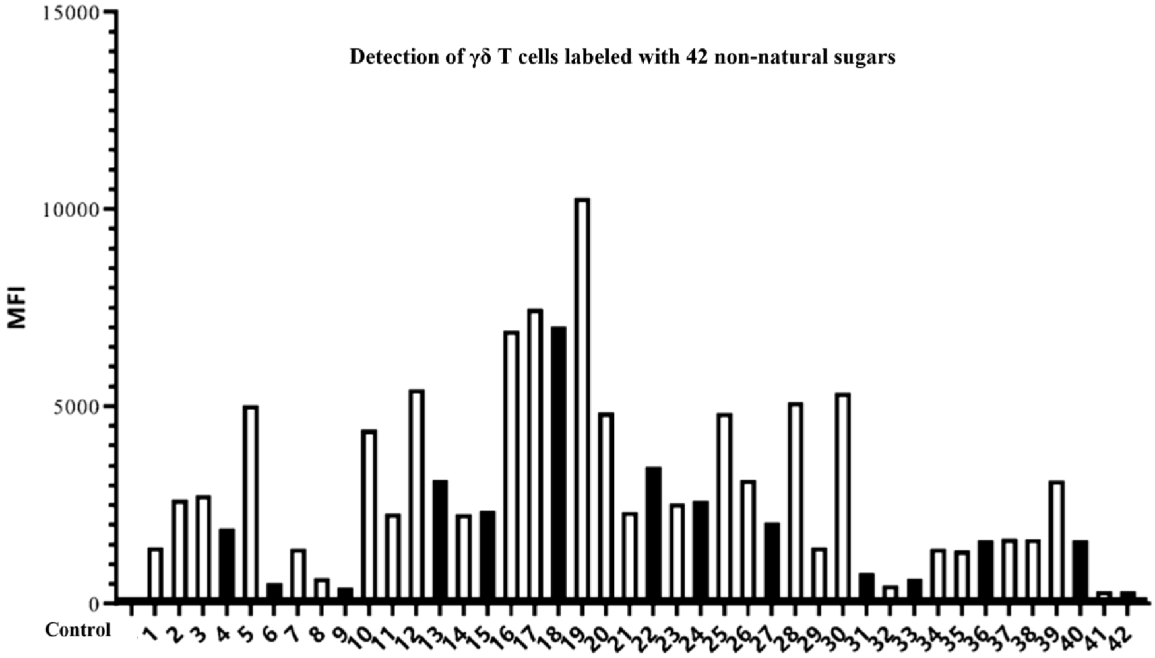
Detection of γδ T cells labeled with 42 non-natural sugars
MFI
15000
10000
5000
0
Control
1
2
3
4
5
6
7
8
9
10
11
12
13
14
15
16
17
18
19
20
21
22
23
24
25
26
27
28
29
30
31
32
33
34
35
36
37
38
39
40
41
42

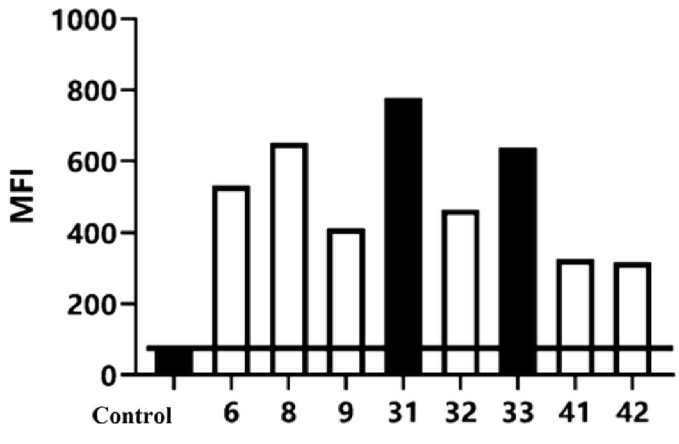
MFI
1000
800
600
400
200
0
Control
6
8
9
31
32
33
41
42

Figure 8

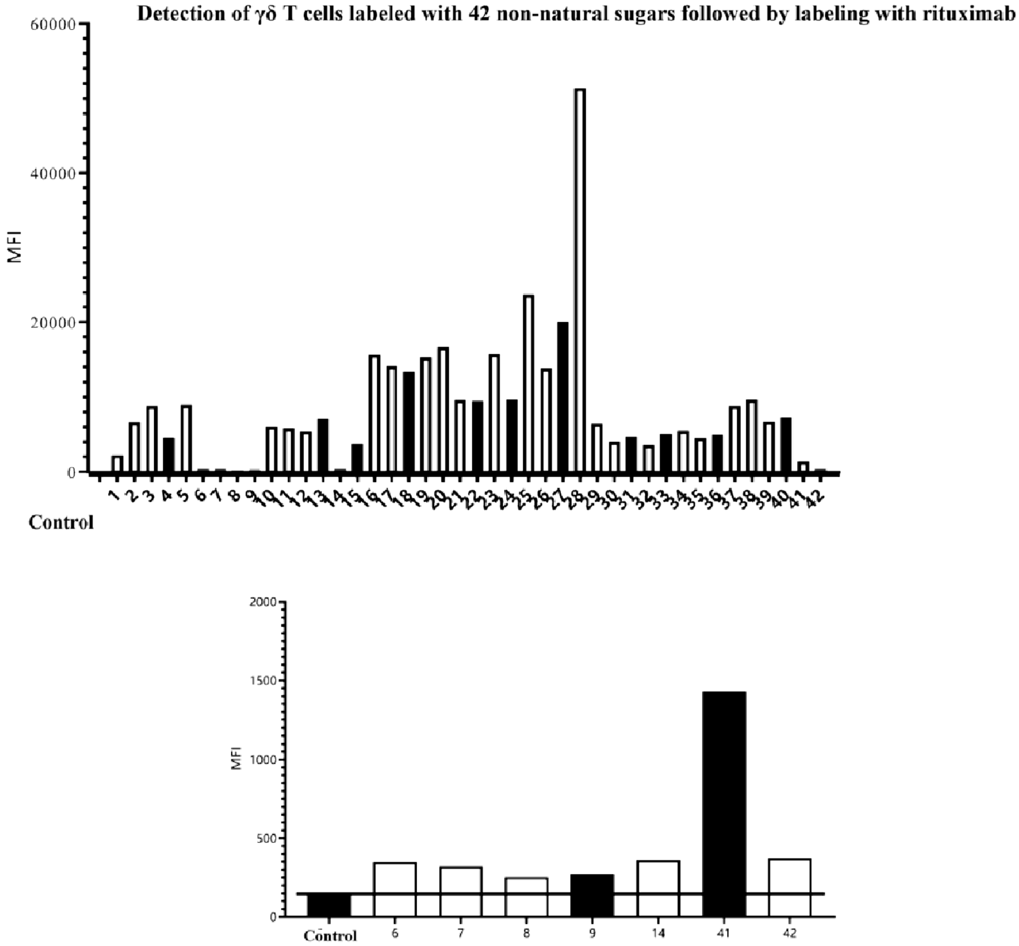
Detection of γδ T cells labeled with 42 non-natural sugars followed by labeling with rituximab
60000
40000
20000
0
MFI
1 2 3 4 5 6 7 8 9 10 11 12 13 14 15 16 17 18 19 20 21 22 23 24 25 26 27 28 29 30 31 32 33 34 35 36 37 38 39 40 41 42
Control
2000
1500
1000
500
0
MFI
Control 6 7 8 9 14 41 42

Figure 9

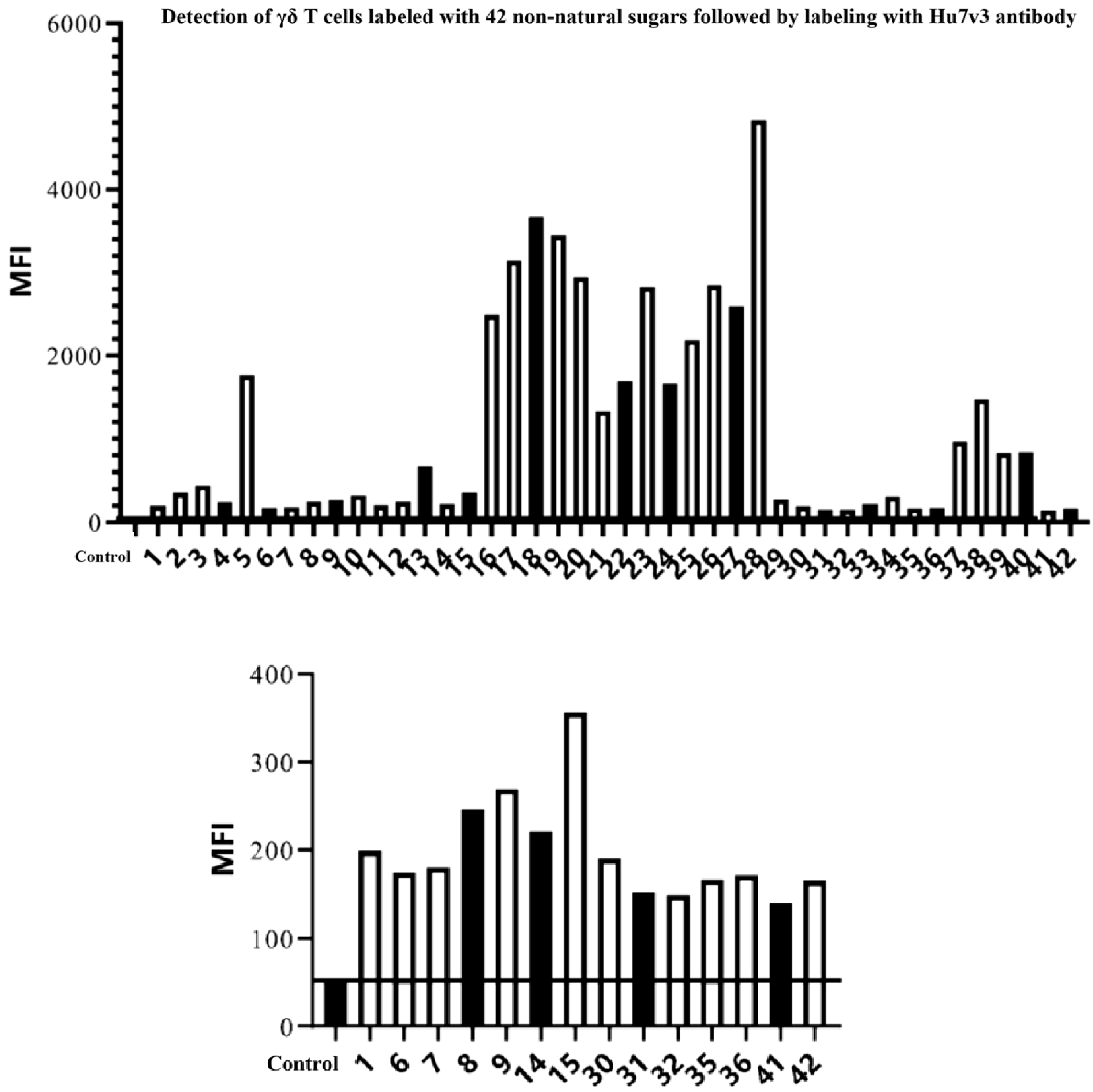
Detection of γδ T cells labeled with 42 non-natural sugars followed by labeling with Hu7v3 antibody
MFI
6000
4000
2000
0
Control
400
300
200
100
0
Control
1
6
7
8
9
14
15
30
31
32
35
36
41
42

Figure 10

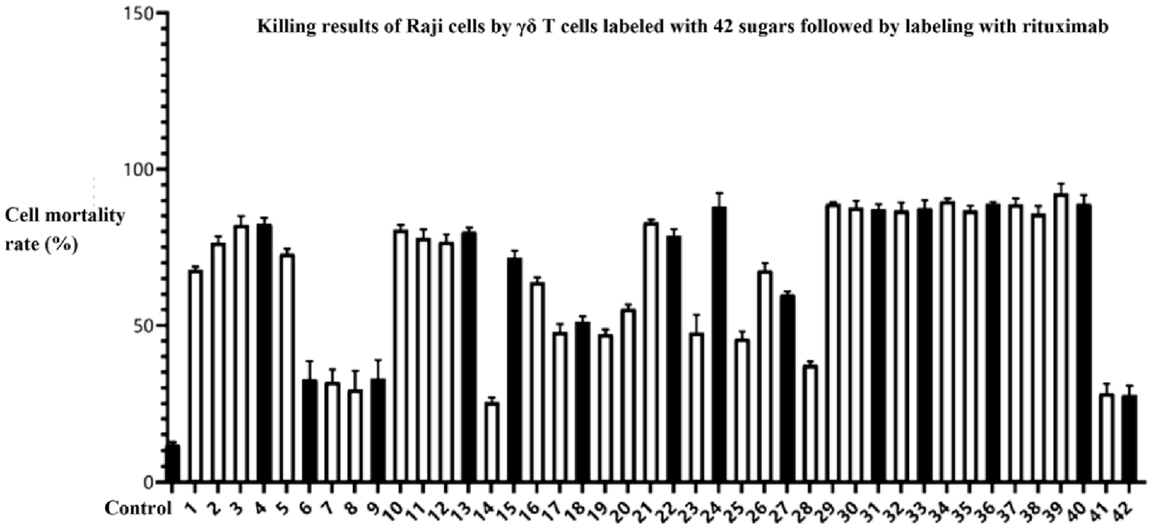


Figure 11

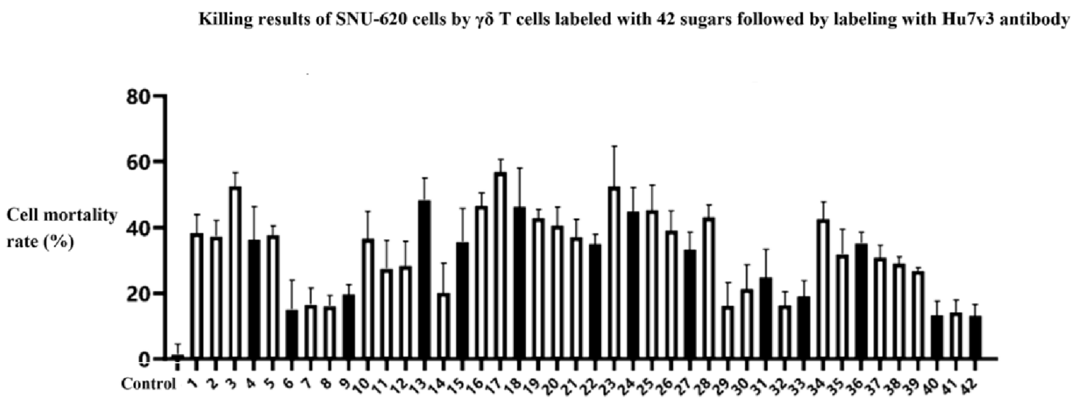


Figure 12

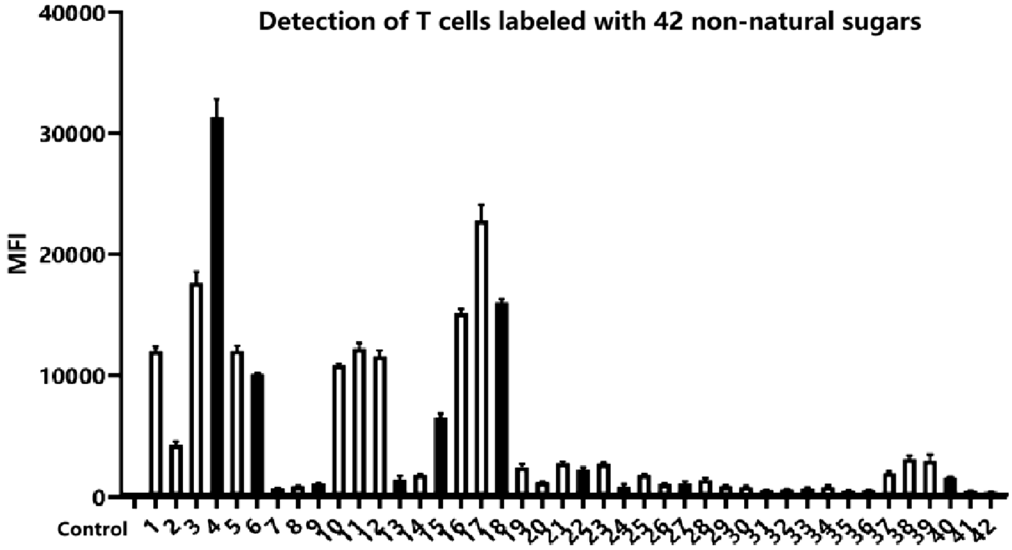
Detection of T cells labeled with 42 non-natural sugars
MFI
40000
30000
20000
10000
0
Control
1
2
3
4
5
6
7
8
9
10
11
12
13
14
15
16
17
18
19
20
21
22
23
24
25
26
27
28
29
30
31
32
33
34
35
36
37
38
39
40
41
42

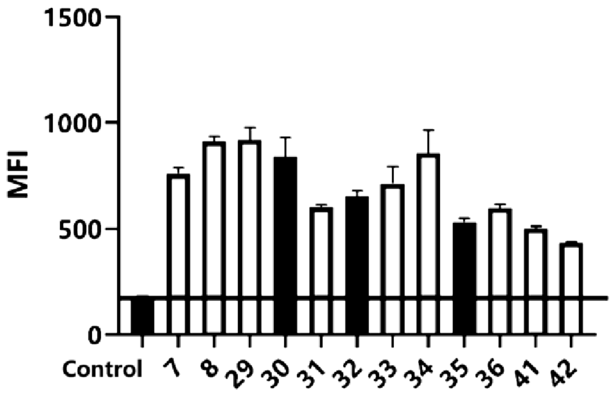
MFI
1500
1000
500
0
Control
7
8
29
30
31
32
33
34
35
36
41
42

Figure 13

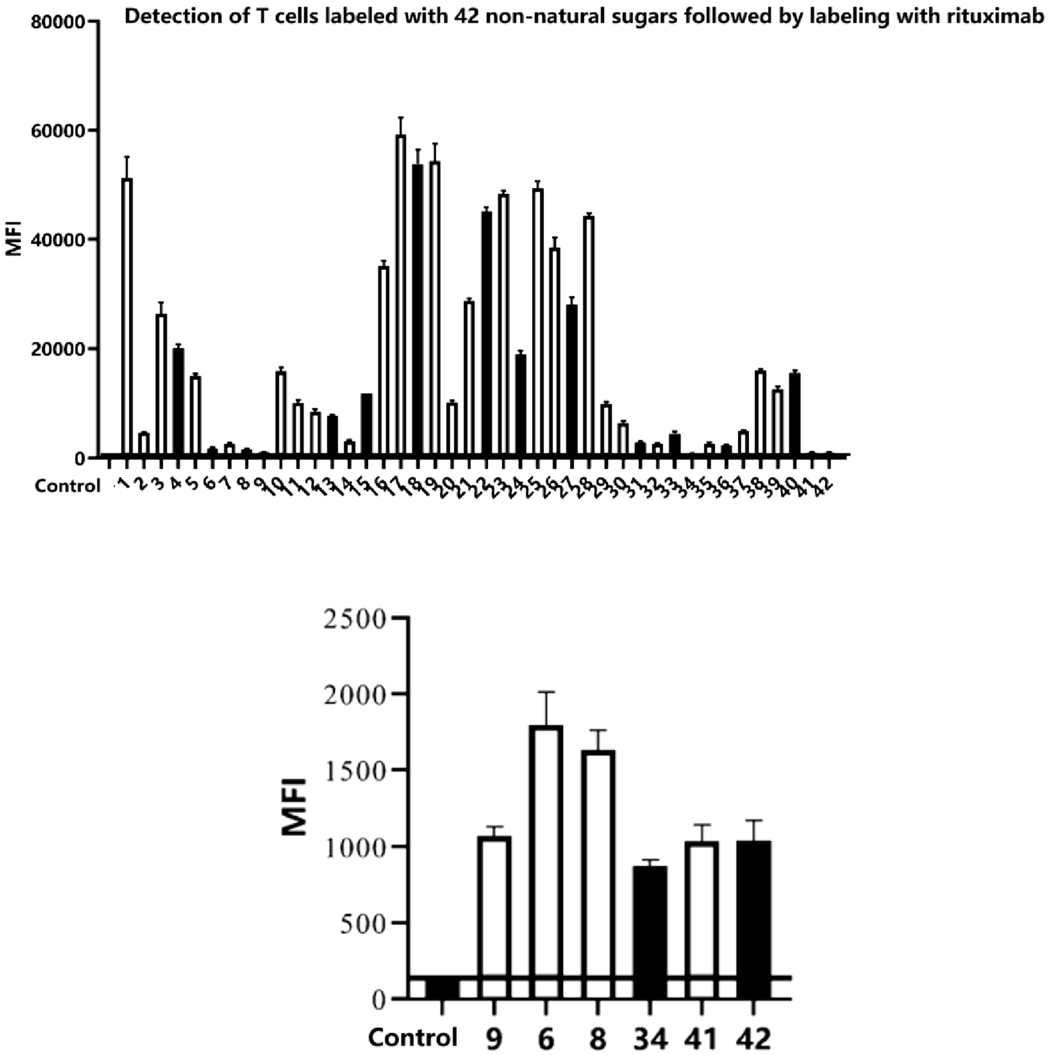
Detection of T cells labeled with 42 non-natural sugars followed by labeling with rituximab
MFI
80000
60000
40000
20000
0
Control
MFI
2500
2000
1500
1000
500
0
Control 9 6 8 34 41 42

Figure 14

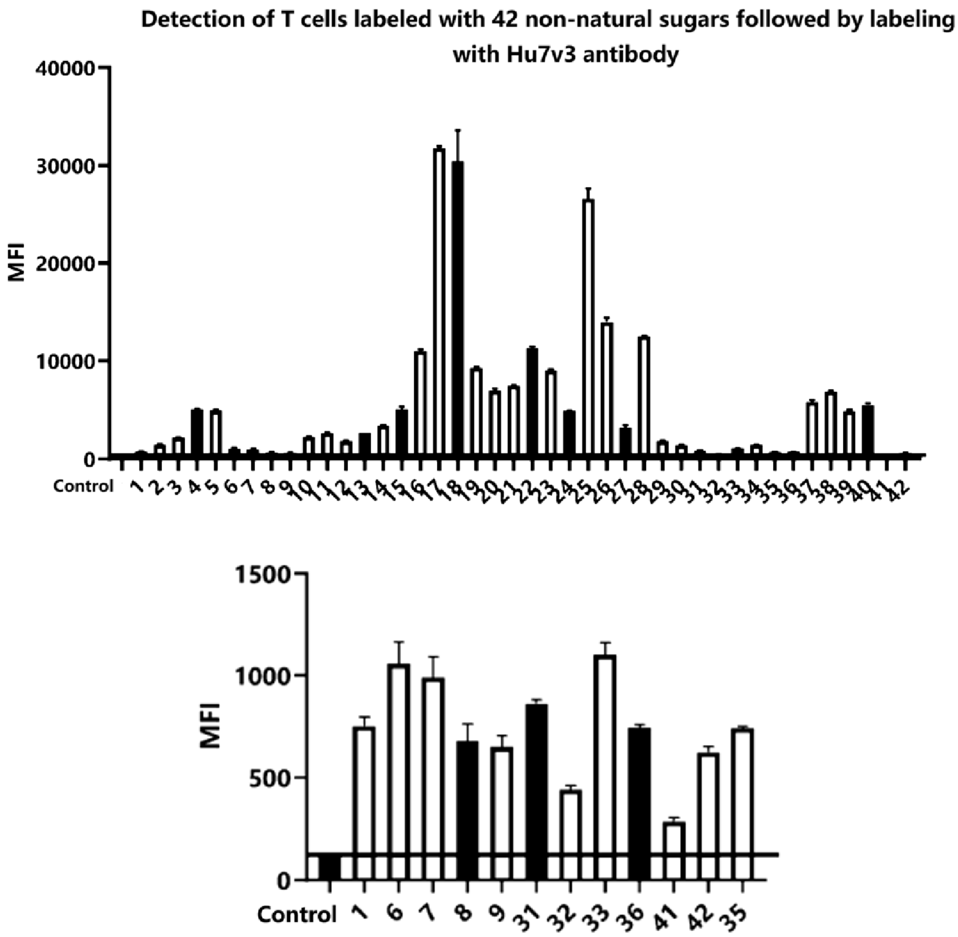
Detection of T cells labeled with 42 non-natural sugars followed by labeling with Hu7v3 antibody
MFI
40000
30000
20000
10000
0
Control
1 2 3 4 5 6 7 8 9 10 11 12 13 14 15 16 17 18 19 20 21 22 23 24 25 26 27 28 29 30 31 32 33 34 35 36 37 38 39 40 41 42
MFI
1500
1000
500
0
Control 1 6 7 8 9 31 32 33 36 41 42 35

Figure 15

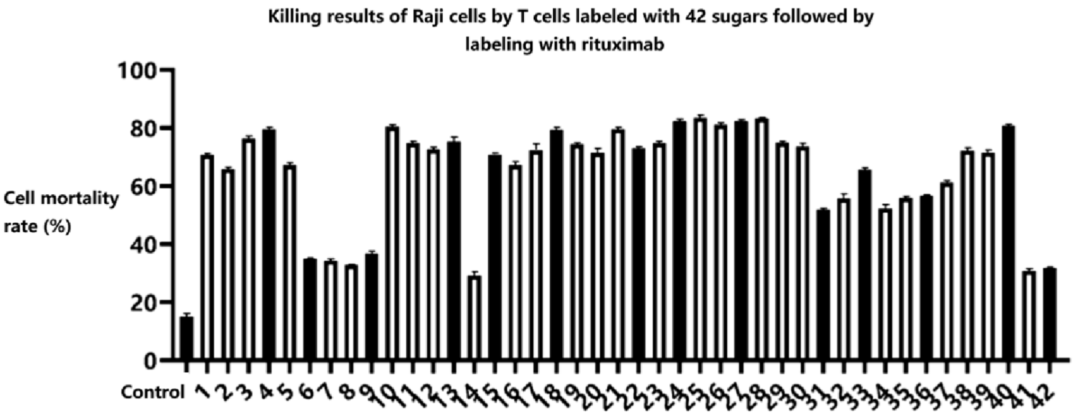


Figure 16

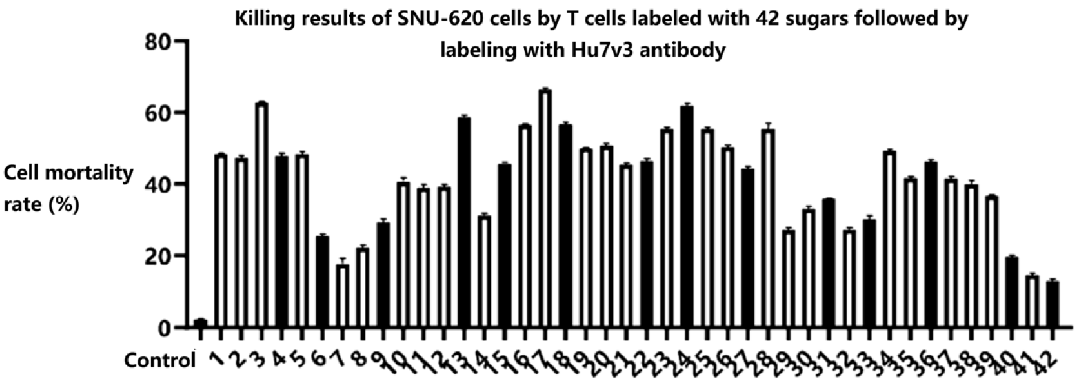


Figure 17

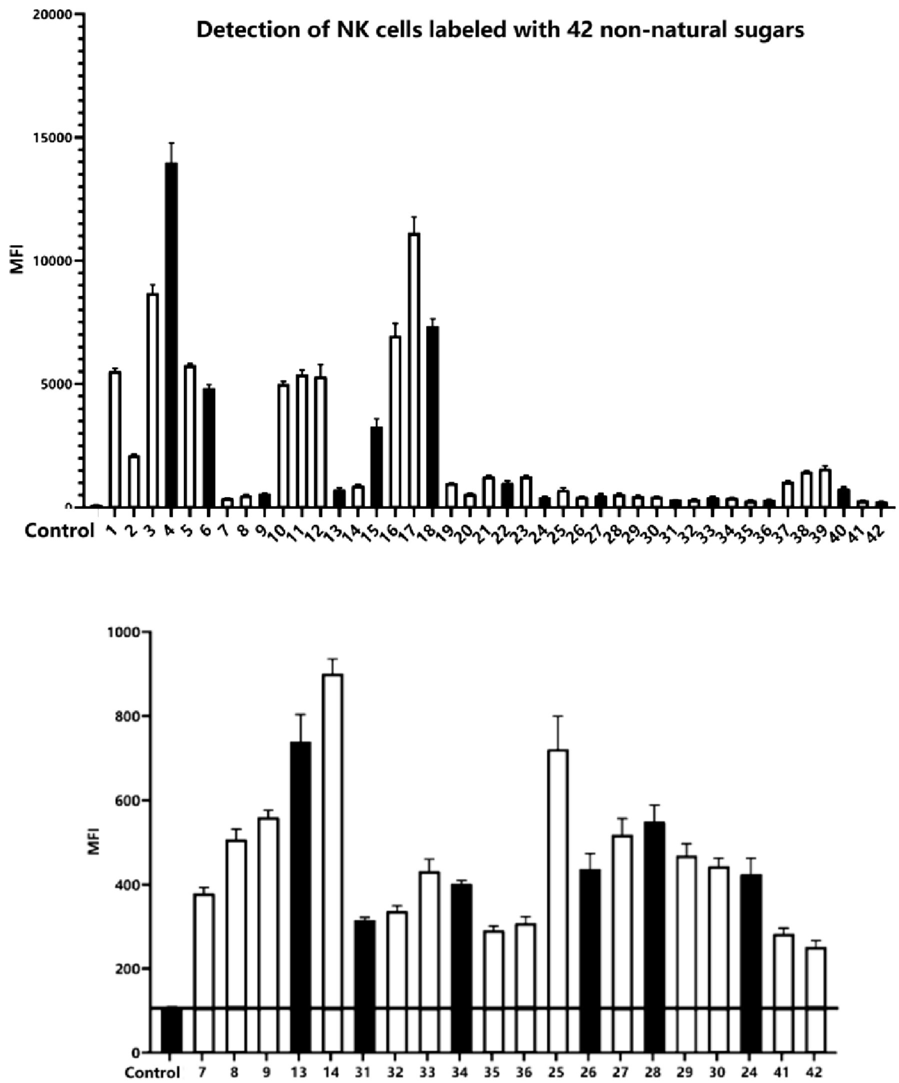
Detection of NK cells labeled with 42 non-natural sugars
20000
15000
10000
5000
0
MFI
Control 1 2 3 4 5 6 7 8 9 10 11 12 13 14 15 16 17 18 19 20 21 22 23 24 25 26 27 28 29 30 31 32 33 34 35 36 37 38 39 40 41 42
1000
800
600
400
200
0
MFI
Control 7 8 9 13 14 31 32 33 34 35 36 25 26 27 28 29 30 24 41 42

Figure 18

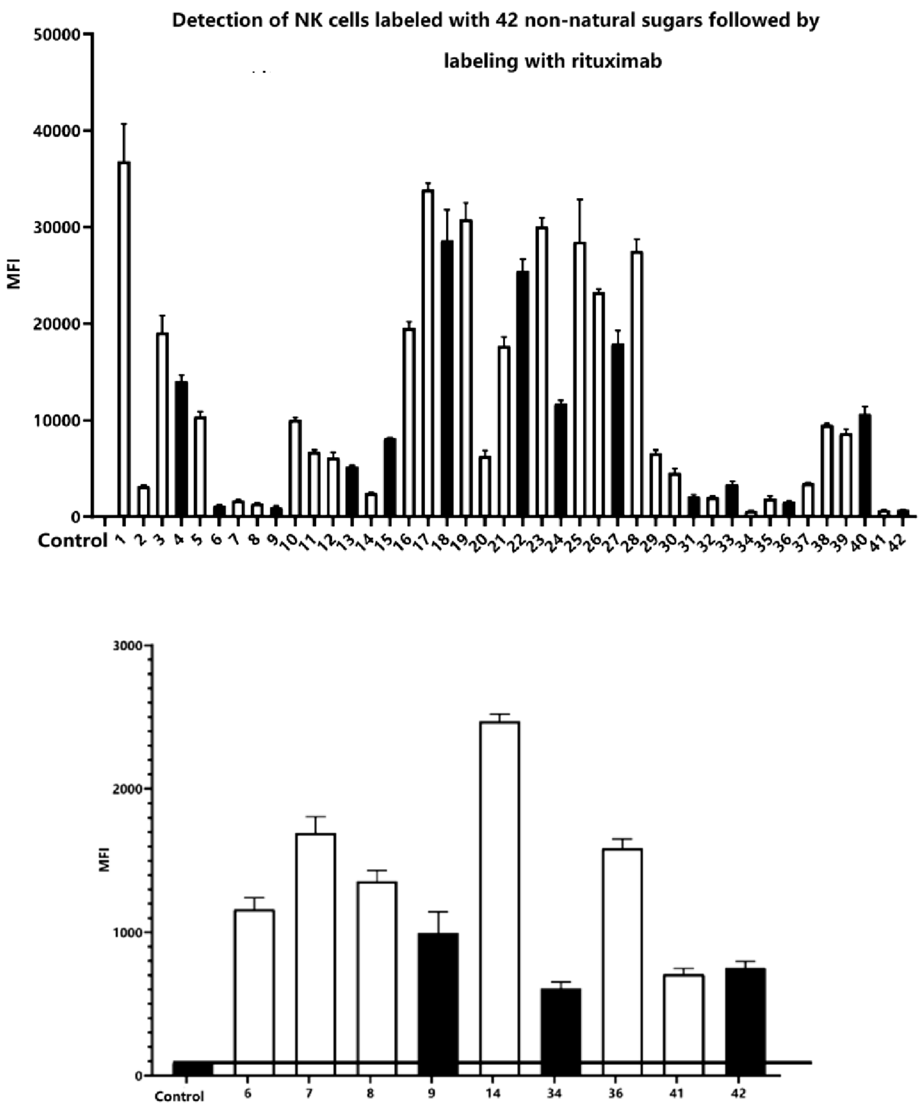
Detection of NK cells labeled with 42 non-natural sugars followed by labeling with rituximab
MFI
50000
40000
30000
20000
10000
0
Control 1 2 3 4 5 6 7 8 9 10 11 12 13 14 15 16 17 18 19 20 21 22 23 24 25 26 27 28 29 30 31 32 33 34 35 36 37 38 39 40 41 42
3000
2000
1000
0
MFI
Control 6 7 8 9 14 34 36 41 42

Figure 19

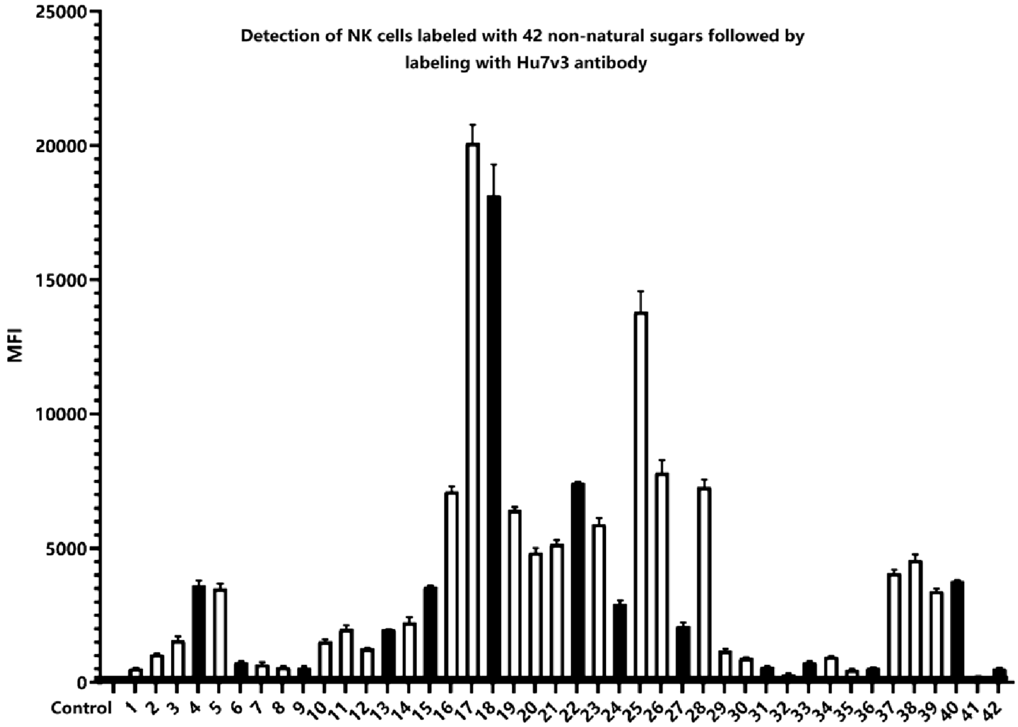
Detection of NK cells labeled with 42 non-natural sugars followed by labeling with Hu7v3 antibody
MFI
25000
20000
15000
10000
5000
0
Control
1
2
3
4
5
6
7
8
9
10
11
12
13
14
15
16
17
18
19
20
21
22
23
24
25
26
27
28
29
30
31
32
33
34
35
36
37
38
39
40
41
42

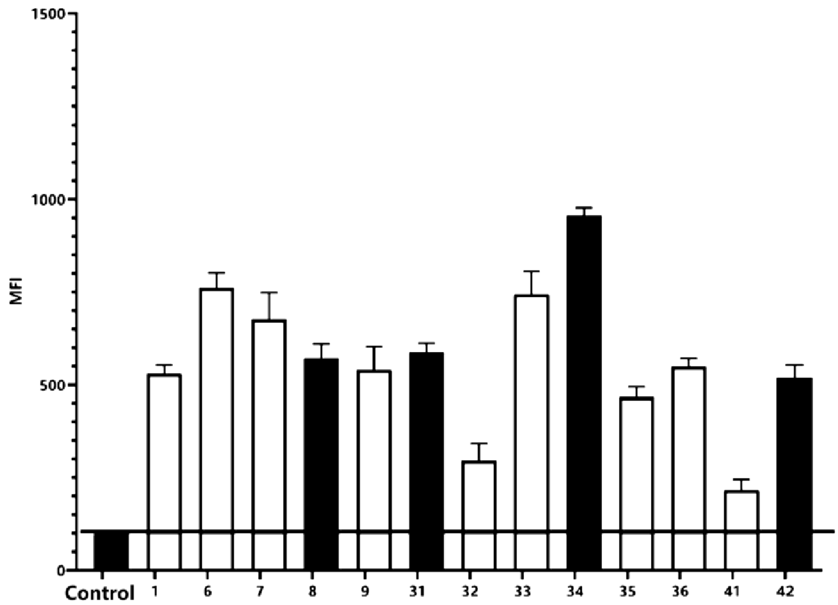
MFI
1500
1000
500
0
Control
1
6
7
8
9
31
32
33
34
35
36
41
42

Figure 20

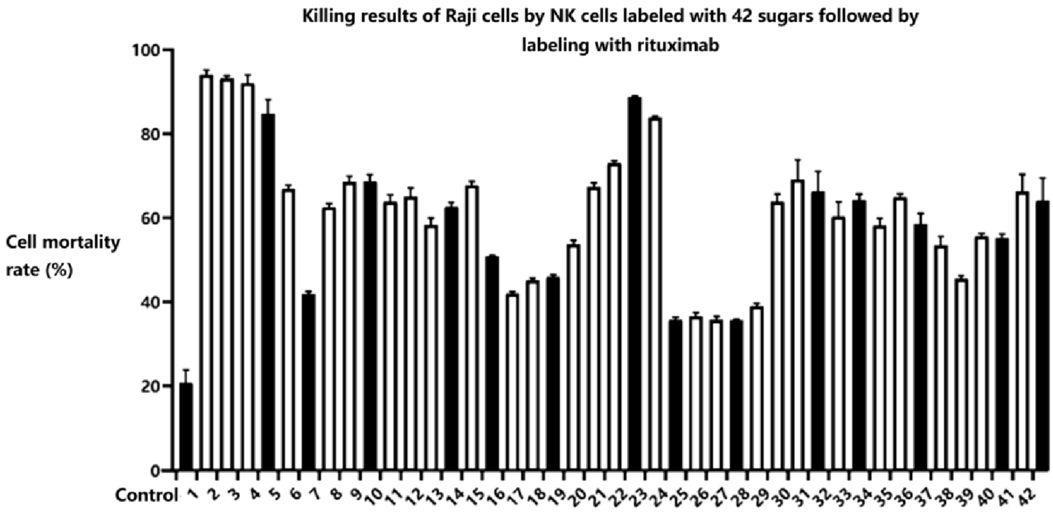


Figure 21

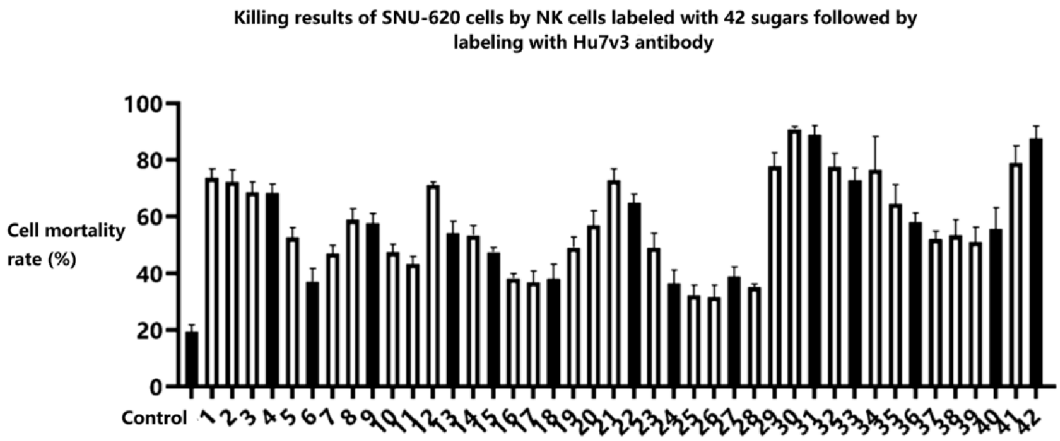


Figure 22

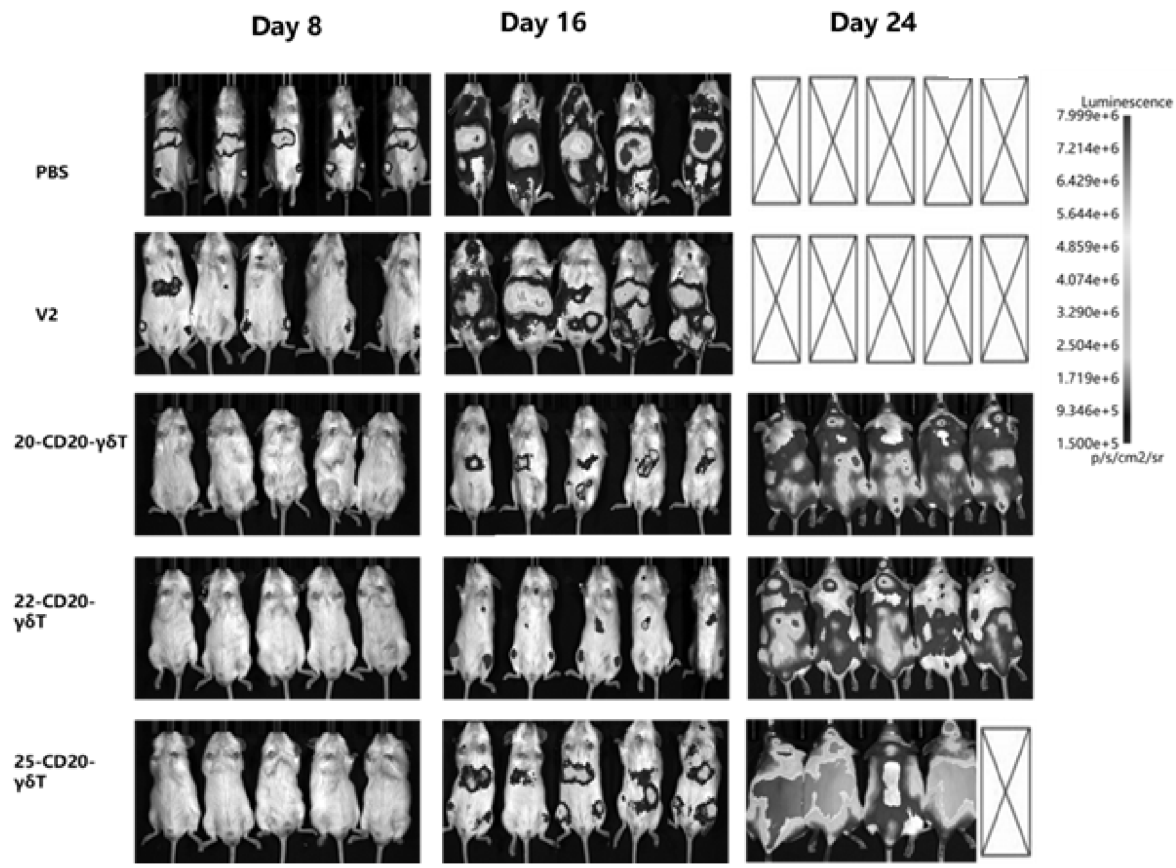
Day 8
Day 16
Day 24
PBS
V2
20-CD20-γδT
22-CD20-γδT
25-CD20-γδT
Luminescence
7.999e+6
7.214e+6
6.429e+6
5.644e+6
4.859e+6
4.074e+6
3.290e+6
2.504e+6
1.719e+6
9.346e+5
1.500e+5
p/s/cm2/sr

Figure 23

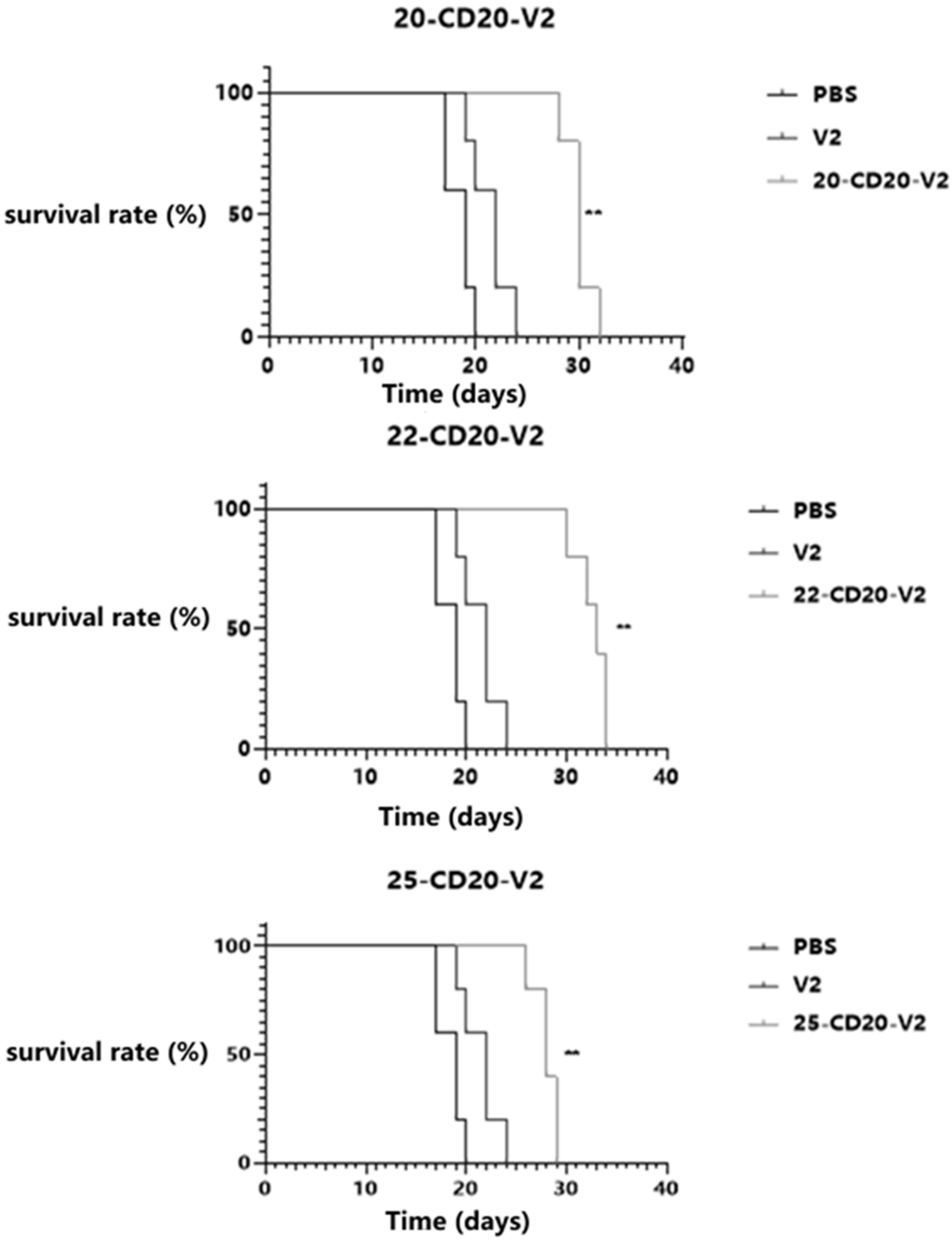
20-CD20-V2
survival rate (%)
100
50
0
0
10
20
30
40
Time (days)
PBS
V2
20-CD20-V2
**
22-CD20-V2
survival rate (%)
Time (days)
PBS
V2
22-CD20-V2
**
25-CD20-V2
survival rate (%)
Time (days)
PBS
V2
25-CD20-V2
**

Figure 24

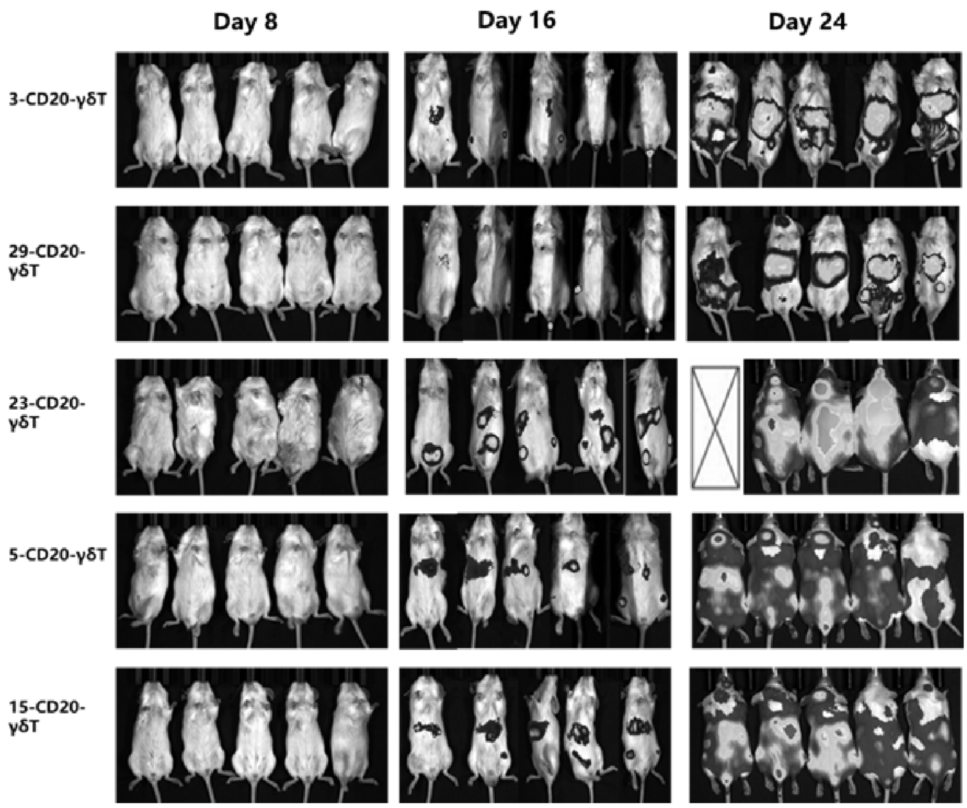
Day 8
Day 16
Day 24
3-CD20-γδT
29-CD20-γδT
23-CD20-γδT
5-CD20-γδT
15-CD20-γδT

Figure 25

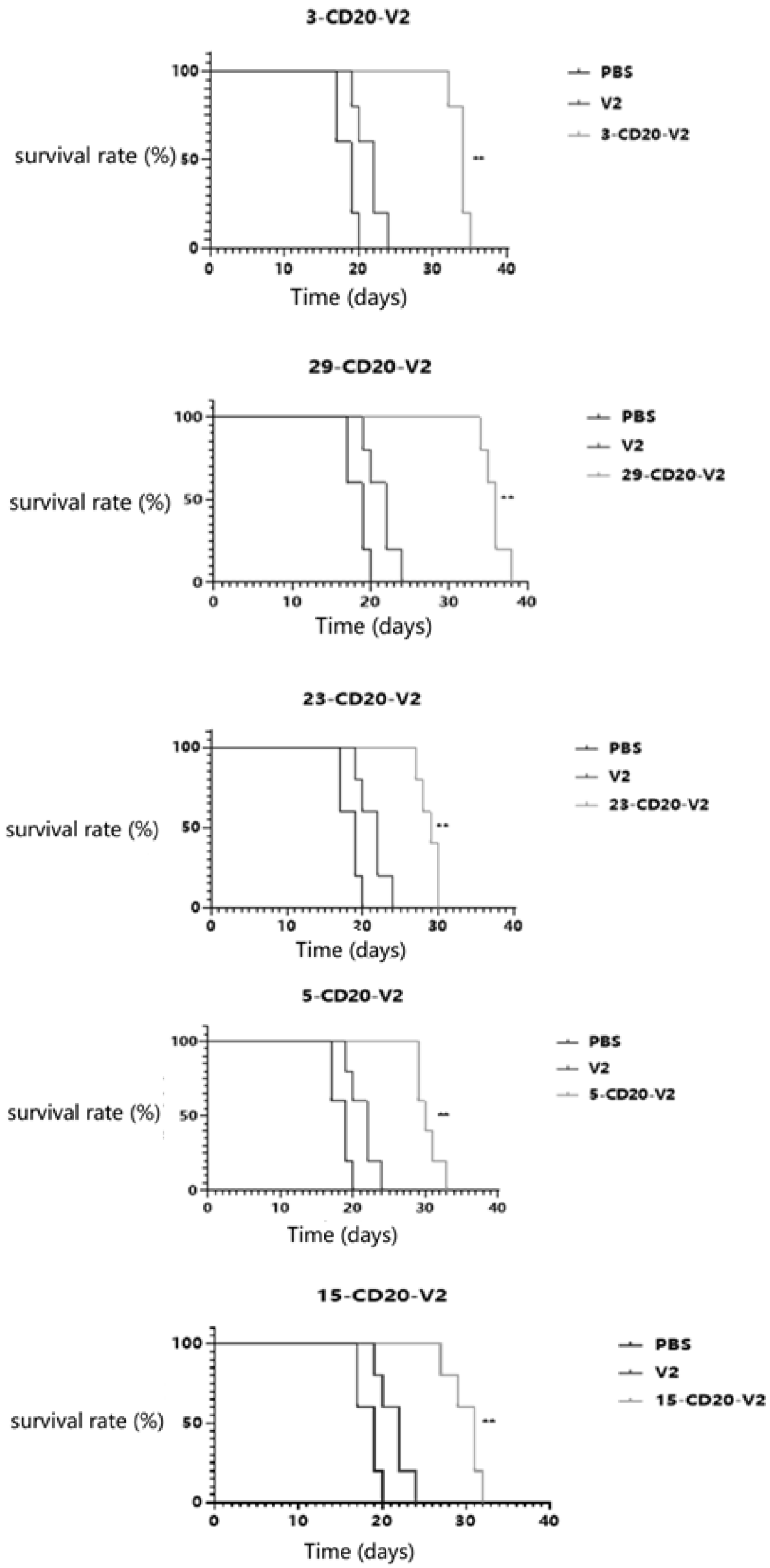
3-CD20-V2
100
survival rate (%) 50
0
0
10
20
30
40
Time (days)
PBS
V2
3-CD20-V2
**
29-CD20-V2
100
survival rate (%) 50
0
0
10
20
30
40
Time (days)
PBS
V2
29-CD20-V2
**
23-CD20-V2
100
survival rate (%) 50
0
0
10
20
30
40
Time (days)
PBS
V2
23-CD20-V2
**
5-CD20-V2
100
survival rate (%) 50
0
0
10
20
30
40
Time (days)
PBS
V2
5-CD20-V2
**
15-CD20-V2
100
survival rate (%) 50
0
0
10
20
30
40
Time (days)
PBS
V2
15-CD20-V2
**

Figure 26

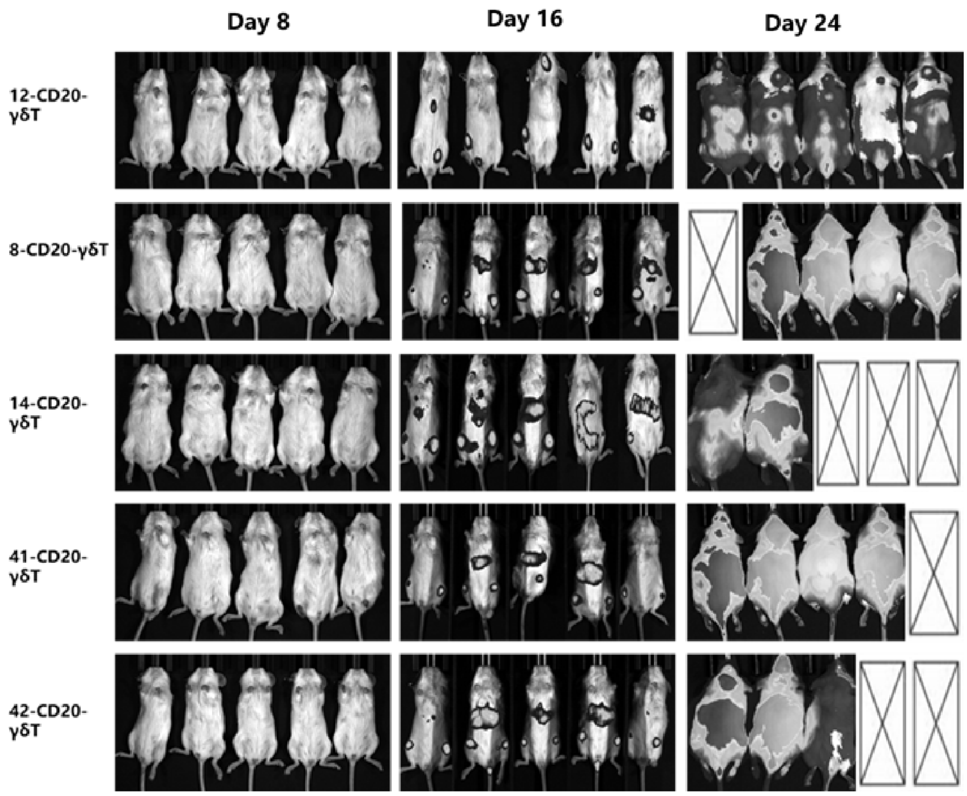
Day 8
Day 16
Day 24
12-CD20-γδT
8-CD20-γδT
14-CD20-γδT
41-CD20-γδT
42-CD20-γδT

Figure 27

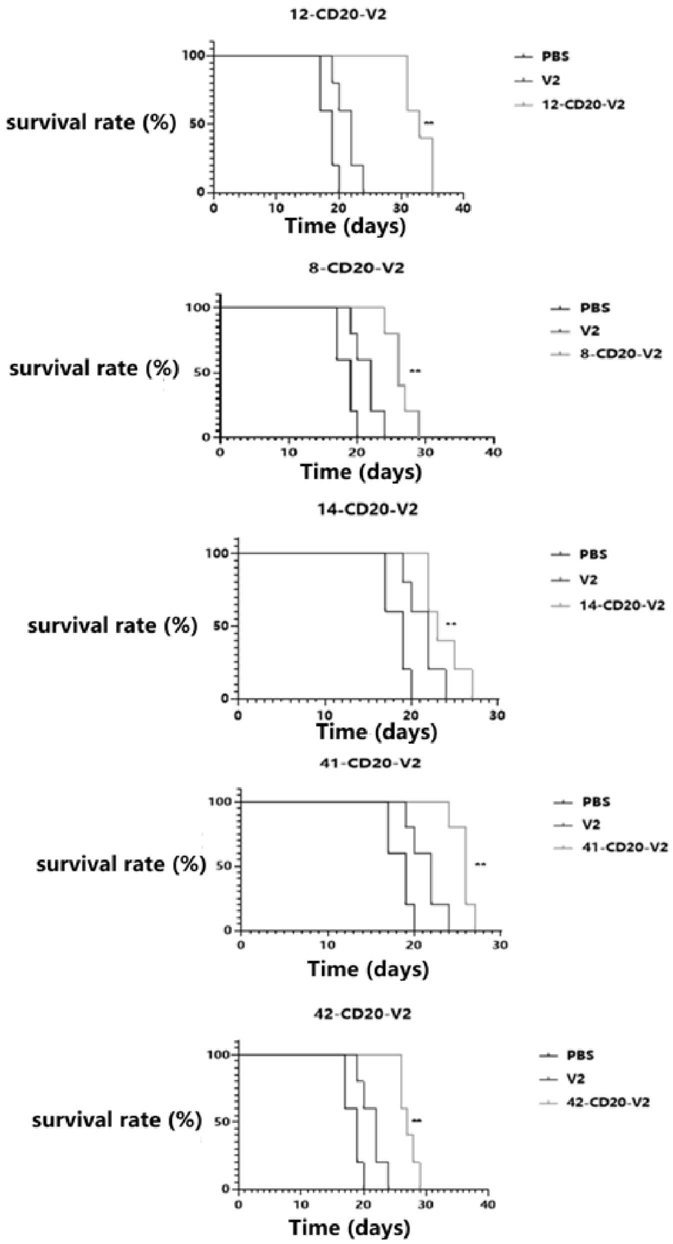
12-CD20-V2
survival rate (%)
Time (days)
PBS
V2
12-CD20-V2
8-CD20-V2
survival rate (%)
Time (days)
PBS
V2
8-CD20-V2
14-CD20-V2
survival rate (%)
Time (days)
PBS
V2
14-CD20-V2
41-CD20-V2
survival rate (%)
Time (days)
PBS
V2
41-CD20-V2
42-CD20-V2
survival rate (%)
Time (days)
PBS
V2
42-CD20-V2

Figure 28

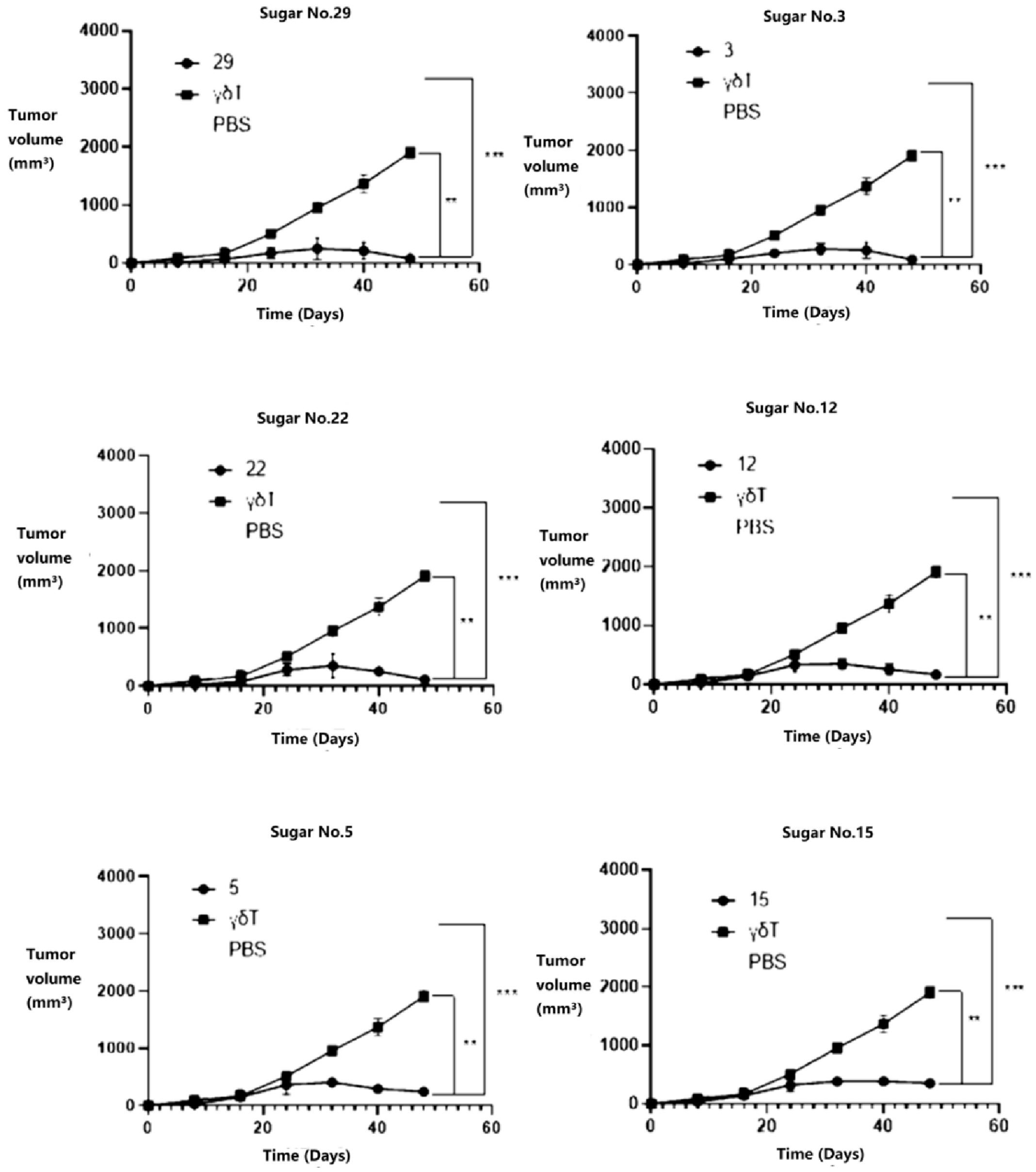
Sugar No.29
29
γδT
PBS
Tumor volume (mm³)
Time (Days)
Sugar No.3
3
γδT
PBS
Tumor volume (mm³)
Time (Days)
Sugar No.22
22
γδT
PBS
Tumor volume (mm³)
Time (Days)
Sugar No.12
12
γδT
PBS
Tumor volume (mm³)
Time (Days)
Sugar No.5
5
γδT
PBS
Tumor volume (mm³)
Time (Days)
Sugar No.15
15
γδT
PBS
Tumor volume (mm³)
Time (Days)

Figure 29

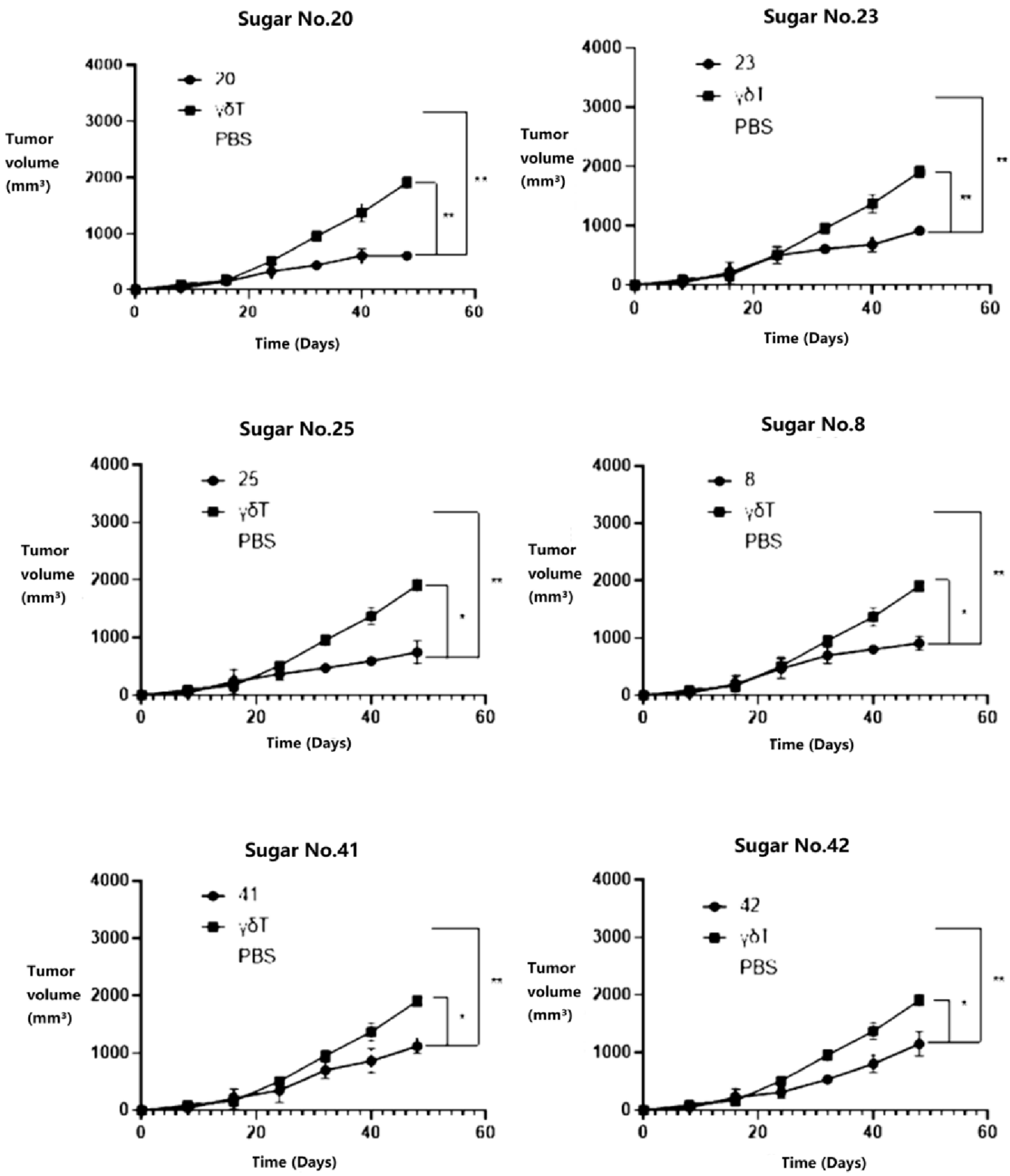
Sugar No.20
20
γδT
PBS
Sugar No.23
23
γδT
PBS
Sugar No.25
25
γδT
PBS
Sugar No.8
8
γδT
PBS
Sugar No.41
41
γδT
PBS
Sugar No.42
42
γδT
PBS
Tumor volume ($mm^3$)
Time (Days)
0
20
40
60
1000
2000
3000
4000
*
**

**Figure 30**

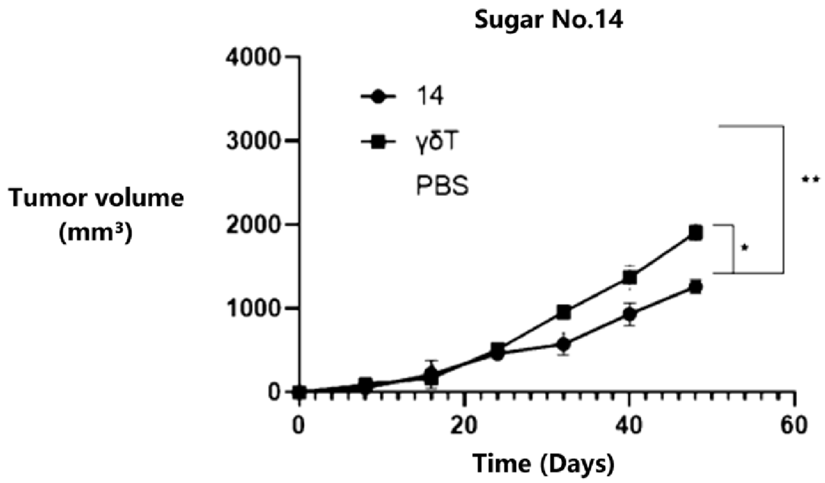
Sugar No.14
Tumor volume
(mm³)
4000
3000
2000
1000
0
14
γδT
PBS
*
**
0
20
40
60
Time (Days)

Figure 31

**INTERNATIONAL SEARCH REPORT**

International application No.
**PCT/CN2024/119388**

**A. CLASSIFICATION OF SUBJECT MATTER**

C12N5/0783(2010.01)i; A61K35/17(2015.01)i; A61P35/00(2006.01)i

According to International Patent Classification (IPC) or to both national classification and IPC

**B. FIELDS SEARCHED**

Minimum documentation searched (classification system followed by classification symbols)

IPC: C12N; A61K; A61P

Documentation searched other than minimum documentation to the extent that such documents are included in the fields searched

Electronic data base consulted during the international search (name of data base and, where practicable, search terms used)

CNTXT, DWPI, ENTXT, ENTXTC, VEN, CNABS, CNKI, PUBMED, ISI WEB OF SCIENCE, GenBank, STNext, 中国专利生物序列检索系统, China Patents Biological Sequence Search System: NK细胞, T细胞, γδT细胞, 免疫细胞, Fv片段, 叠氮, 免疫细胞, 生物正交反应, 点击化学, 北京领新博瑞生物科技有限公司, 周鹏, 6azfucose, Ac2ManNaz, Ac36azfucose, azide, bioorthogonal, BU2ManNaz, CD20, dAb, DBCO, Fab, Pr2GalNaz, Pr2GlcNaz, Pr2ManNaz, scFv, spaac, VHH, di-scFv, Ac3-6-bis(Me-SATE)-P-ManNaz, Immune cell, T cell, 1-O-ac-6-bis(Me-SATE)-P-ManNaz, F(ab')2, 1-O-Pr-6-bis-POM-P-ManNaz, 1-O-Pr-6-(Leu-OET-ProTIDE)-P-ManNaz, peng zhou, NK cell, F(ab)2, claudin18.2, linxcell, SEQ ID NOs: 1-12

**C. DOCUMENTS CONSIDERED TO BE RELEVANT**

| Category* | Citation of document, with indication, where appropriate, of the relevant passages | Relevant to claim No. |
|---|---|---|
| X | CN 112675202 A (JIANGNAN UNIVERSITY) 20 April 2021 (2021-04-20)<br>claims 1-10, and description ,paragraphs 15, 50, 53, and 55 | 1-69 |
| X | CN 111534475 A (XIAMEN NKD BIOTECH CO., LTD.) 14 August 2020 (2020-08-14)<br>claims 1-10, and description, paragraphs 56-60, 63-66, and 71-75 | 1-69 |
| X | US 2023101939 A1 (THE CHARLOTTE MECKLENBURG HOSPITAL AUTHORITY D/B/A ATRIUM HEALTH) 30 March 2023 (2023-03-30)<br>description, embodiments 2-3 | 1-69 |
| PX | CN 117264904 A (PEKING UNIVERSITY THIRD HOSPITAL (PEKING UNIVERSITY THIRD CLINICAL MEDICAL COLLEGE)) 22 December 2023 (2023-12-22)<br>claims 1-10 | 1-69 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 December 2024** | **23 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/119388**

**C. DOCUMENTS CONSIDERED TO BE RELEVANT**

| Category* | Citation of document, with indication, where appropriate, of the relevant passages | Relevant to claim No. |
|---|---|---|
| PX | CN 117264905 A (PEKING UNIVERSITY THIRD HOSPITAL (PEKING UNIVERSITY THIRD CLINICAL MEDICAL COLLEGE)) 22 December 2023 (2023-12-22)<br>claims 1-10 | 1-69 |
| PX | CN 117264906 A (PEKING UNIVERSITY THIRD HOSPITAL (PEKING UNIVERSITY THIRD CLINICAL MEDICAL COLLEGE)) 22 December 2023 (2023-12-22)<br>claims 1-10 | 1-69 |
| A | CN 110157682 A (SHENZHEN INSTITUTES OF ADVANCED TECHNOLOGY) 23 August 2019 (2019-08-23)<br>description, paragraphs 5-18 | 1-69 |
| A | NISHIMOTO, K. P. et al. "Allogeneic CD20-targeted γδ T Cells Exhibit Innate and Adaptive Antitumor Activities in Preclinical B-cell lymphoma Models"<br>*Clinical & Translational Immunology,* Vol. 11, 31 December 2022 (2022-12-31),<br>article number e1373: page 1, abstract | 1-69 |

Form PCT/ISA/210 (second sheet) (July 2022)

**INTERNATIONAL SEARCH REPORT**

International application No.
**PCT/CN2024/119388**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/119388**

| Patent document cited in search report | | | Publication date (day/month/year) | Patent family member(s) | | | Publication date (day/month/year) |
|---|---|---|---|---|---|---|---|
| CN | 112675202 | A | 20 April 2021 | US | 2022202949 | A1 | 30 June 2022 |
| CN | 111534475 | A | 14 August 2020 | CN | 111534475 | B | 15 March 2022 |
| | | | | WO | 2021104414 | A1 | 03 June 2021 |
| | | | | US | 2022380721 | A1 | 01 December 2022 |
| US | 2023101939 | A1 | 30 March 2023 | US | 11879010 | B2 | 23 January 2024 |
| | | | | US | 2024083992 | A1 | 14 March 2024 |
| CN | 117264904 | A | 22 December 2023 | | None | | |
| CN | 117264905 | A | 22 December 2023 | | None | | |
| CN | 117264906 | A | 22 December 2023 | | None | | |
| CN | 110157682 | A | 23 August 2019 | CN | 110157682 | B | 12 November 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **CHENG, B.** ; **WANG, C. T.** ; **HAO, Y. et al.** Facile Synthesis of Clickable Non-natural sugars in the Unprotected and 1,6-Di-O-Acylated Forms for Metabolic Glycan Labeling. *Chem-Eur J*, 2023, vol. 29 (11), e202203054
- **HAN, S. F.** ; **COLLINS, B. E.** ; **BENGTSON, P.** ; **PAULSON, J. C.** Homomultimeric complexes of CD22 in B cells revealed by protein-glycan cross-linking. *Nat. Chem. Biol.*, 2005, vol. 1 (2), 93-7
- **LUCHANSKY, S. J.** ; **GOON, S.** ; **BERTOZZI, C. R.** Expanding the diversity of unnatural cell-surface sialic acids. *ChemBioChem*, 2004, vol. 5 (3), 371-4
- **CHENG, B.** ; **DONG, L.** ; **ZHU, Y. T. et al.** 9-Azido Analogues of Three Sialic Acid Forms for Metabolic Remodeling of Cell-Surface Sialoglycans. *ACS Chem. Biol.*, 2019, vol. 14 (10), 2141-2147